# EUROPEAN PATENT SPECIFICATION

(11) **EP 1 180 114 B1**
(45) Date of publication and mention of the grant of the patent: **21.09.2011**
(21) Application number: 00930170.6
(22) Date of filing: 27.04.2000
(51) Int. Cl.: C07K 14/47

(54) **Use of resisitin specific antibodies in the treatment of diabetes**
Verwendung von Resistin spezifischen Antikörpern in der Behandlung von Diabetes
Utilisation d'anticorps spécifiques contre la resistine dans le traitement du diabète

(30) Priority: 27.04.1999 US 131263 P
(43) Date of publication of application: 20.02.2002
(73) Proprietor: The Trustees of The University of Pennsylvania, Philadelphia, PA 19104 (US)
(72) Inventor: LAZAR, Mitchell, A., Gladwyne, PA 19035 (US)
(74) Representative: Grünecker, Kinkeldey, Stockmair & Schwanhäusser Anwaltssozietät
(86) International application number: PCT/US2000/011272
(87) International publication number: WO 2000/064920

(56) References cited:
- WO-A-00/05259
- WO-A-00/20447
- WO-A-98/58061
- WO-A-99/11293
- WO-A-99/55868
- REGINATO MAURICIO J ET AL: "Mechanisms by which thiazolidinediones enhance insulin action" TRENDS IN ENDOCRINOLOGY AND METABOLISM, vol. 10, no. 1, January 1999 (1999-01), pages 9-13, XP002283032 ISSN: 1043-2760
- STEPPAN CLAIRE M ET AL: "Resistin and obesity-associated insulin resistance." TRENDS IN ENDOCRINOLOGY AND METABOLISM, vol. 13, no. 1, pages 18-23, XP002283033 ISSN: 1043-2760
- STEPPAN C M ET AL: "The hormone resistin links obesity to diabetes" NATURE, MACMILLAN JOURNALS LTD. LONDON, GB, vol. 409, 18 January 2001 (2001-01-18), pages 307-312, XP002970710 ISSN: 0028-0836
- FORMAN ET AL.: 'Unique response pathways are established by allosteric interactions among nuclear hormone receptors' CELL, vol. 81, 19 May 1995, pages 541 - 550, XP002930686
- KLIEWER ET AL.: 'Fatty acids and eicosanoids regulate gene expression through direct interactions with peroxisome proliferator-activated receptors alpha and gamma' PROC. NATL. ACAD. SCI. USA, vol. 94, April 1997, pages 4318 - 4323, XP002930687
- MUKHERJEE ET AL.: 'Sensitization of diabetic and obese mice to insulin by retinoid X receptor agonists' NATURE, vol. 386, 27 March 1997, pages 407 - 410, XP002930688
- TONTONOZ ET AL.: 'Stimulatin of adipogenesis in fibroblasts by PPARgamma2, a lipid-activated transcription factor' CELL, vol. 79, 30 December 1994, pages 1147 - 1156, XP002930689

## Description

### BACKGROUND OF THE INVENTION

Non-insulin-dependent diabetes mellitus (NIDDM) is a common, chronic disease that is a major cause of morbidity and mortality in industrialized societies. NIDDM affects 95% of diabetics and afflicts more than 5% of the world's population. NIDDM has a strong genetic component and is tightly linked to obesity. The disorder is characterized by severe tissue resistance to the effects of insulin. Although impaired insulin secretion contributes to NIDDM, insulin levels are often increased in the early course of the disease. This peripheral insulin resistance is a major difference between NIDDM and insulin-dependent diabetes (Olefsky, 1985, Am. J. Med. 79:1-7). The resistance occurs despite qualitatively and quantitatively normal insulin receptors, thus implicating one or more defective steps in the insulin signaling pathway downstream from insulin binding to its receptor.

Nevertheless, although NIDDM is characterized by insulin resistance, the only available pharmacological treatments for NIDDM until only recently were insulin or agents that increase insulin secretion. New pharmacological approaches to treating NIDDM have been developed recently that target other metabolic abnormalities (Larkins, 1997, TEM 8:187-191). For instance, the thiazolidinediones (TZDs) are a new class of orally active drugs that are particularly exciting because they decrease insulin resistance by enhancing the actions of insulin at a level distal to the insulin receptor (Henry, 1997, Endo. Metab. Clin. North Amer. 26:553-573).

TZDs, which include troglitazone, pioglitazone, and rosaglitazone are thought to sensitize target tissues to the action of insulin. These compounds are ineffective at lowering serum glucose levels in the absence of insulin. In animal models of NIDDM, TZDs lower plasma glucose levels, and decrease insulin and triglycerides to near normal levels (Fujita, 1983, Diabetes 32:804-810; Fujiwara, 1988, Diabetes 37:1549-1558).

In human studies, approximately 75% of patients with NIDDM responded to troglitazone treatment (Suter et al., 1992, Diabetes Care 16:193-203). In addition to reduced plasma glucose levels, TZDs also cause insulin levels and/or dose requirements to decrease (Kumar et al., 1996, Diabetologia 39:701-709). TZDs also significantly lower serum concentrations of triglycerides and free fatty acids (FFA), with a small rise in HDL cholesterol (Spencer and Markham, 1997, Drugs 54:89-102; Schwartz et al., 1998, N. Engl. J. Med. 338:861-866). Recent hyperinsulinemic-euglycemic clamp studies have suggested that troglitazone works primarily by increasing the rate of peripheral glucose disposal in skeletal muscle (Inzucchi et al., 1998, N. Engl. J. Med. 338:867-869).

TZDs are generally well tolerated by patients although troglitazone therapy has been associated with hepatic dysfunction which has been fatal in a few cases (Watkins and Whitcomb, 1998, N. Engl. J. Med. 338:916-917). Thus, liver function tests should be monitored frequently although the causal relationship and mechanism of TZD-mediated liver toxicity have not been established. Chronic TZD therapy also leads to modest weight gain in rodents and humans.

TZDs were originally developed by screening analogs of clofibric acid for anti-pipidemic and anti-hyperglycemic effects (Kawamatsu et al., 1980, Arznein.-Forsch./Drug Res. 30:454-459). The anti-diabetic effects of these compounds were not understood but the discovery that TZDs enhanced adipocyte differentiation (Hiragun et al., 1988, J. Cell. Physiol. 134:124-130; Kletzien et al., 1992, Mol. Pharmacol. 41:393-398) was an important clue to identifying its molecular target. Activators of peroxisome proliferator activated receptor γ (PPARγ), a member of the nuclear hormone receptor superfamily, were also found to induce adipogenesis. PPARγ was shown to be predominantly expressed in adipose tissue and to function as a key transcription factor in adipocyte differentiation (Tontonoz et al., 1994, Cell 79:1147-1156). Shortly after these studies, TZDs were demonstrated to be direct ligands for PPARγ.

PPARγ is a member of the nuclear hormone receptor superfamily of transcription factors that are activated by small, lipophilic ligands. There are two PPARγ isoforms, γ1 and γ2, derived from alternate promoter usage. PPARy2 contains an additional 31 amino acids at its N-terminus, but the functional significance of this addition is unclear. Interestingly, PPARγ2 is found exclusively in adipocytes, whereas PPARγ1 is predominantly expressed in adipocytes but also expressed in other tissues.

PPARγ belongs to a subset of nuclear receptors that forms heterodimers with the retinoid X receptor (RXR), which greatly enhances the ability of the receptor to bind specific DNA sequences in target genes. The DNA sequences recognized by the PPAR/RXR heterodimer are referred to as PPAR-response elements (PPREs). PPAR/RXR heterodimers bind to PPREs in the absence of ligand, but binding of the ligand leads to a conformational change which results in activation of transcription of the target gene. The active conformation recruits a multiprotein coactivator complex that acetylates histones (leading to an open, more active conformation of the nucleosome) as well as interacting directly with the basal transcription machinery. PPREs have been found in the regulatory regions of a number of genes involved in lipid metabolism and energy balance (Lemberger et al., 1996, Annu. Rev. Cell Dev. Biol. 12:335-363).

Ectopic expression of PPARγ in preadipocytes, fibroblasts, and myoblasts induces adipocyte differentiation in the presence of TZD ligand (Tontonoz et al., 1994, Cell 79:1147-1156). The ability of TZDs acting via PPARγ to induce adipocyte differentiation may explain the modest weight increases observed in vivo in mammals. It is not clear how to reconcile the fact that excess fat cell mass is a major risk factor for insulin resistance and NIDDM with the antihyperglycemic effects of TZDs.

There is strong evidence that TZDs function via PPARγ. PPARγ has been shown to bind to a number of different ligands including a number of fatty acids as well as prostaglandin J derivatives, such as 15-deoxy-Δ12,14-prostaglandin J2 and others (Forman et al., 1995, Cell 81:541-550; Kliewer et al., 1997, Proc. Natl. Acad. Sci. USA 94:4318-4323). However, none of these compounds binds to PPARγ with affinities in the nanomolar range. In contrast, TZDs have been shown to bind to PPARγ with an affinity in the range of 40-200 nM. Not only are TZDs activating ligands for PPARγ at nanomolar concentrations, there is also a remarkable correlation between TZD potencies for in vivo plasma glucose lowering with their order of potency for both PPARγ activation and direct binding to PPARγ (Wilson, 1996, J. Med. Chem. 39:665-668; Berger et al., 1996, Endocrinology 137:4189-4195). RXR ligands can also activate the PPARγ/RXR heterodimer, and synthetic RXR agonists increase insulin sensitivity in obese mice and work in combination with TZDs to further enhance antidiabetic activity (Mukherjee, et al., 1997, Nature 386:407-410). This further suggests that the PPARγ/RXR heterodimer complex is the molecular target for treatment of insulin resistance *in vivo.*

There is general agreement that TZDs are effective antidiabetic agents because they enhance insulin-responsive glucose disposal in vivo. It is also clear that TZDs are high affinity, activating ligands for PPARγ. However, the mechanism by which PPARγ mediates the antidiabetic actions of TZDs is not clear since the main site of TZD-enhanced glucose disposal primarily occurs in skeletal muscle whereas the main site of PPARγ expression is in adipose tissue. However, although PPARγ expression predominantly occurs in adipocytes, PPARγ expression has been demonstrated in a variety of extra-adipose tissues, including liver, colon, breast, type II pneumocytes of the lung, and macrophages. More importantly, PPARγ expression in skeletal muscle has also been reported albeit at much lower levels than in adipose tissue: the level of PPARγ mRNA is more than 50-fold higher in adipose tissue than in skeletal muscle. Therefore, the exact tissue site at which TZDs function to promote insulin action in muscle remains to be elucidated.

It is possible that mechanisms other than PPARγ activation explain the effects of TZDs on glucose disposal in muscle. However, given the nanomolar binding affinity of TZDs for PPARγ and the remarkable correlation between PPARγ activation and enhancement of insulin action, it is likely that PPARγ binding and activation are related to the *in vivo* actions of TZDs. A number of potential mechanisms have been proposed to explain how the activation of PPARγ may be causally connected to insulin action.

There is a need to elucidate the mechanisms of action of TZDs and to identify genes which are modulated by these compounds in mammalian cells. Also, there is an acute need to develop screening methods to identify potential anti-diabetic compounds and for the development of treatments for diabetes and other related diseases such as Syndrome X. The present invention satisfies these needs.

### BRIEF SUMMARY OF THE INVENTION

X → 5a illustrates The invention illustrates an isolated nucleic acid encoding a mammalian resistin, or a fragment thereof. The nucleic acid may share at least about 30% sequence identity with an nucleic acid encoding at least one of mouse *resistin* (SEO ID NO:1) and human *resistin* (SEQ ID NO:3). The nucleic acid may also share at least about 30% sequence identity with a nucleic acid having the sequence of SEQ ID NO:1 and shares at least about 30% sequence identity with a nucleic acid having the sequence of SEQ ID NO:3.

Further illustrated is an isolated nucleic acid encoding a mammalian resistin, wherein the amino acid sequence of the resistin shares at least about 30% sequence identity with an amino acid sequence of at least one of (SEQ ID NO:2) and (SEQ ID NO:4). The amino acid sequence of the resistin may share at least about 30% sequence identity with an amino acid sequence of (SEQ ID NO:2), The amino acid sequence of the resistin may also share at least about 30% sequence identity with an amino acid sequence of (SEQ ID NO:4).

The invention additionally illustrates an isolated polypeptide comprising a mammalian resistin. The mammalian resistin may share at least about 30% sequence identity with an amino acid sequence of at least one of SEQ ID NO:2 and SEQ ID NO:4. The mammalian resistin may share at least about 30% sequence identity with an amino acid sequence of SEQ ID NO:2. The mammalian resistin may share at least about 30% sequence identity with an amino acid sequence of SEQ ID NO:4.

Embodiments of the present invention are:
1. Use of an antibody or an antibody fragment that specifically binds to a mammalian resistin polypeptide for the preparation of a medicament for the treatment of a diabetic or prediabetic condition wherein said mammalian resistin polypeptide shares at least about 95% sequence identity with SEQ ID NO: 2 or SEQ ID NO: 4.
2. Use according to embodiment 1, wherein said condition is Syndrome X.
3. Use according to embodiment 1 or 2, wherein said condition is type 2 diabetes.
4. Use according to embodiment 1, wherein the composition is for administration in a resistin-inhibiting amount and has the ability to antagonize insulin action on glucose uptake.
5. Use or method according to any of embodiments 1 to 3, wherein said antibody is selected from the group consisting of a polyclonal antibody, a monoclonal antibody, a humanized antibody, and a synthetic antibody.
6. Antibody or an antibody fragment that specifically binds to a mammalian resistin polypeptide for the treatment of a diabetic or prediabetic condition wherein said mammalian resistin polypeptide shares at least about 95% sequence identity with SEQ ID NO: 2 or SEQ ID NO: 4.
7. Use of a composition comprising an antibody or an antibody fragment that specifically binds to a mammalian resistin polypeptide for the preparation of a medicament for the treatment of a diabetic or prediabetic condition wherein said mammalian resistin polypeptide shares at least about 95% sequence identity with SEQ ID NO: 2 or SEQ ID NO: 4.
8. Composition comprising an antibody or an antibody fragment that specifically binds to a mammalian resistin polypeptide for the treatment of a diabetic or prediabetic condition wherein said mammalian resistin polypeptide shares at least about 95% sequence identity with SEQ ID NO: 2 or SEQ ID NO: 4.

With respect to the isolated nucleic acid comprising mammalian resistin, the nucleic acid further comprise a nucleic acid encoding a tag polypeptide covalently linked thereto. The tag polypeptide may be selected from the group consisting of a myc tag polypeptide, a glutathione-S-transferase tag polypeptide, a green fluorescent protein tag polypeptide, a myc-pyruvate kinase tag polypeptide, a His6 tag polypeptide, an influenza virus hemagglutinin tag polypeptide, a flag tag polypeptide, and a maltose binding protein tag polypeptide.

In addition, the nucleic acid further comprises a nucleic acid encoding a promoter/regulatory sequence operably linked thereto.

Also illustrated in the invention is a vector comprising an isolated nucleic acid encoding a mammalian resistin, or a fragment thereof. The vector may further comprise a nucleic acid encoding a promoter/regulatory sequence operably linked thereto.

Further illustrated is a recombinant cell comprising an isolated nucleic acid encoding mammalian resistin or a fragment thereof, and a recombinant cell comprising a vector cell comprising an isolated nucleic acid encoding mammalian resistin or a fragment thereof.

The invention further illustrates an isolated nucleic acid complementary to a nucleic acid encoding mammalian resistin or a fragment thereof, the isolated nucleic acid being in an antisense orientation. The complementary nucleic acid may snare at least about 30% identity with a nucleic acid complementary to a nucleic acid having the sequence of at least one of mouse *resistin* (SEQ ID NO:1) and human *resistin* (SEQ ID NO:3).

The invention also illustrates a recombinant cell comprising an isolated nucleic acid complementary to a nucleic acid encoding a mammalian resistin, or a fragment thereof and a recombinant cell comprising a vector comprising an isolated nucleic acid complementary to a nucleic acid encoding a mammalian resistin, or a fragment thereof.

The invention includes an antibody that specifically binds with a mammalian resistin polypeptide, or a fragment thereof. The antibody may be selected from the group consisting of a polyclonal antibody, a monoclonal antibody, and a synthetic antibody, and is also provided as an antidiabetic composition comprising the antibody and a pharmaceutically-acceptable carrier.

Also illustrated is an antidiabetic composition comprising an isolated nucleic acid complementary to a nucleic acid encoding mammalian resistin or a fragment thereof, and a pharmaceutically acceptable carrier.

Further illustrated is a composition comprising an isolated nucleic acid encoding mammalian resistin and a pharmaceutically-acceptable carrier.

The invention additionally illustrates a knock-out targeting vector. The vector comprises a first nucleic acid portion encoding a nucleic acid comprising a sequence 5' of the open reading frame encoding *resistin* and a second nucleic acid portion comprising a nucleic acid sequence 3' of the open reading frame encoding a mammalian *resistin.* The vector may comprise a nucleic acid encoding a selectable marker covalently linked thereto. The first and second nucleic acid portions may flank the nucleic acid encoding the selectable marker.

There is also illustrated a recombinant cell comprising the knock-out targeting vector just described and a transgenic non-human mammal composing the knock-out targeting vector just described. The mammal may be a rodent.

Further illustrated is a transgenic non-human mammal comprising an isolated nucleic acid encoding mammalian resistin or a fragment thereof.

The invention also comprises alleviating type 2 diabetes: Embodiment 3 contemplates administering to a patient afflicted with type 2 diabetes a glucose uptake-enhancing amount of an antidiabetic composition comprising an antibody that specifically binds with mammalian resistin or a fragment thereof and a pharmaceutically acceptable carrier.

There is further included alleviating Syndrome X: Embodiment 2 contemplates, administering to a patient afflicted with Syndrome X a glucose uptake-enhancing amount of an antidiabetic composition comprising an antibody that specifically binds with mammalian resistin or a fragment thereof and a pharmaceutically acceptable carrier.

The invention further includes treating type 2 diabetes: Embodiment 3 contemplates administering to a patient afflicted with type 2 diabetes a glucose uptake-enhancing amount of a composition selected from the group consisting of an antidiabetic composition comprising an antibody that specifically binds with 5 mammalian resistin or a fragment thereof and a pharmaceutically acceptable carrier.

Further included is treating Syndrome X; Embodiment contemplates administering to a patient afflicted with Syndrome X a glucose uptake-enhancing amount of a composition selected from the group consisting of an antidiabetic composition comprising an antibody that specifically binds with mammalian resistin or a fragment thereof and a pharmaceutically acceptable carrier.

In addition, there is included alleviating type 2 diabetes: : Embodiment 3 contemplates administering to a patient afflicted with type 2 diabetes a resistin-inhibiting amount of a composition selected from the group consisting of an antidiabetic composition comprising an antibody that specifically binds with mammalian resistin or a fragment thereof and a pharmaceutically acceptable carrier.

Further included is alleviating Syndrome X: Embodiment 2 contemplates administering to a patient afflicted with Syndrome X a resistin-inhibiting amount of a composition selected from the group consisting of an antidiabetic composition comprising an antibody that specifically binds with mammalian resistin or a fragment thereof and a pharmaceutically acceptable carrier.

There is also included treating type 2 diabetes. Embodiment 3 contemplates administering to a patient afflicted with type 2 diabetes a resistin-inhibiting amount of a composition selected from the group consisting of an antidiabetic composition comprising an antibody that specifically binds with mammalian resistin or a fragment thereof and a pharmaceutically acceptable carrier.

Further included is treating Syndrome X. Embodiment 2 contemplates aministering to a patient afflicted with Syndrome X a resistin-inhibiting amount of a composition selected from the group consisting of an antidiabetic composition comprising an antibody that specifically binds with mammalian resistin or a fragment thereof and a pharmaceutically acceptable carrier.

The invention also illustrates a method of identifying a compound that affects expression of resistin in a cell. The method comprises contacting a cell with a test compound and comparing the level of resistin expression in the cell with the level of resistin expression in an otherwise identical cell not contacted with the test compound, wherein a higher or lower level of resistin expression in the cell contacted with the test compound compared with the level of resistin expression in the otherwise identical cell not contacted with the test compound is an indication that the test compound affects expression of resistin in a cell.

A compound identified by the aforementioned method is also illustrated in the invention.

In addition, there is illustrated a method of identifying a compound that reduces expression of resistin in a cell. The method comprises contacting a cell with a test compound and comparing the level of resistin expression in the cell with the level of resistin expression in an otherwise identical cell not contacted with the test compound, wherein a lower level of resistin expression in the cell contacted with the test compound compared with the level of resistin expression in the otherwise identical cell not contacted with the test compound is an indication that the test compound reduces expression of resistin in a cell.

A compound identified bv this method of the invention is also illustrated

There is further illustrated a method of determining whether a test compound is a candidate antidiabetic drug candidate. The method comprises contacting a cell comprising a nucleic encoding resistin with a test compound, and comparing the level of expression of resistin in the cell with the level of expression of resistin in an otherwise identical cell which is not contacted with the test compound, whereby a lower level of expression of resistin in the cell contacted with the test compound compared with the level of expression of resistin in the otherwise identical cell not contacted with the test compound is an indication that the test compound is a candidate antidiabetic drug candidate.

There is further illustrated a method of determining whether a test compound is a candidate drug for treatment of Syndrome X. The method comprises contacting a cell comprising a nucleic encoding resistin with a test compound, and comparing the level of expression of resistin in the cell with the level of expression of resistin in an otherwise identical cell which is not contacted with the test compound, whereby a lower level of expression of resistin in the cell contacted with the test compound compared with the level of expression of resistin in the otherwise identical cell not contacted with the test compound is an indication that the test compound is a candidate drug for treatment of Syndrome X.

In addition, there is illustrated a method of determining whether a test compound is a candidate antidiabetic drug candidate. The method comprises contacting a cell comprising a PPARγ receptor and a nucleic encoding resistin with a test compound, and comparing the level of expression of resistin in the cell with the level of expression of resistin in an otherwise identical cell which is not contacted with the test compound, whereby a lower level of expression of resistin in the cell contacted with the test compound compared with the level of expression of resistin in the otherwise identical cell not contacted with the test compound is an indication that the test compound is a candidate antidiabetic drug candidate.

A method of increasing glucose uptake by a cell is illustrated in the invention. The method comprises contacting a cell expressing resistin with a resistin-reducing amount of an anti-resistin compound, thereby increasing glucose uptake by the cell. In one aspect, the cell expressing resistin is selected from the group consisting of an adipocyte, a recombinant cell transfected with an isolated nucleic acid encoding *resistin,* a muscle cell line, a liver cell line, a primary culture cell from skeletal muscle, a primary culture adipocyte cell, and a primary culture hepatocyte.

Further illustrated is a method of increasing insulin-stimulated glucose uptake by a cell. The method comprises contacting a cell expressing resistin with insulin and further contacting the cell with a resistin-reducing amount of an anti-resistin compound, thereby increasing insulin-stimulated glucose uptake by the cell.

In addition, there is illustrated a method of diagnosing type 2 diabetes in a previously undiagnosed mammal. The method comprises obtaining a biological sample from the mammal, assessing the level of resistin in the biological sample, and comparing the level of resistin in the biological sample with the level of resistin in a biological sample obtained from a like mammal not afflicted with type 2 diabetes, wherein a higher level of resistin in the biological sample from the mammal compared with the level of resistin in the biological sample from the like mammal is an indication that the mammal is afflicted with type 2 diabetes, thereby diagnosing type 2 diabetes in the previously undiagnosed mammal.

The biological sample may be selected from the group consisting of a blood sample, a white adipose tissue sample, and a brown adipose tissue sample.

There is also illustrated a method of diagnosing Syndrome X in a previously undiagnosed mammal. The method comprises obtaining a sample from the mammal, assessing the level of resistin in the sample, and comparing the level of resistin in the sample with the level of resistin in a sample obtained from a like mammal not afflicted with Syndrome X, wherein a higher level of resistin in the sample from the mammal compared with the level of resistin in the sample from the like mammal is an indication that the mammal is afflicted with Syndrome X, thereby diagnosing Syndrome X in the previously undiagnosed mammal.

Further is a method of assessing the effectiveness of a treatment for type 2 diabetes in a mammal. The method comprises assessing the level of resistin in a sample obtained from a mammal prior to treatment of the mammal for type 2 diabetes, and comparing the level of resistin in the sample with the level of resistin in a sample obtained from the mammal during the course of or following treatment for type 2 diabetes, wherein a lower level of resistin in the sample obtained prior to treatment compared with the level of resistin in the sample obtained during the course of or following treatment for type 2 diabetes is an indication of the effectiveness of the treatment for type 2 diabetes in the mammal.

In addition, there is illustrated a method of assessing the effectiveness of a treatment for Syndrome X in a mammal. The method comprises assessing the level of resistin in a sample obtained from a mammal prior to treatment of the mammal for Syndrome X, and comparing the level of resistin in the sample with the level of resistin in a sample obtained from the mammal during the course of or following treatment for Syndrome X, wherein a lower level of resistin in the sample obtained prior to treatment compared with the level of resistin in the sample obtained during the course of or following treatment for Syndrome X is an indication of the effectiveness of the treatment for Syndrome X in the mammal.

In addition, the invention illustrates a method of assessing the response in a mammal to TZD. The method comprises assessing the level of resistin in a sample obtained from a mammal prior to administration of TZD to the mammal, administering TZD to the mammal, and assessing the level of resistin in a sample obtained from the mammal during or after administration of TZD, wherein a higher or lower level of resistin in the sample obtained during or after administration of TZD to the mammal compared with the level of resistin in the sample obtained during or after administration of TZD is an indication of the response to TZD in the mammal, thereby assessing the response to TZD in the mammal.

Further illustrated is a method of assessing the response in a mammal to a compound that affects PPARγ-mediated signaling. The method comprises assessing the level of resistin in a sample obtained from a mammal prior to administration of the compound to the mammal, administering the compound to the mammal, assessing the level of resistin in a sample obtained from the mammal during or after administration of the compound, and comparing the level of resistin in the sample obtained during or after administration of the compound to the mammal with the level of resistin in the sample obtained prior to administration of the compound to the mammal, wherein a higher or lower level of resistin in the sample obtained during or after administration of the compound to the mammal compared with the level of resistin in the sample obtained prior to administration of the compound to the mammal is an indication of the response in the mammal to the compound, thereby assessing the response in the mammal to a compound that affects PPARγ-mediated signaling.

In addition, there is illustrated a method of detecting a mutation in a *resistin* allele in a human. The method comprises comparing the nucleic acid sequence encoding *resistin* of a human suspected of having a mutation in a *resistin* allele with the nucleic acid sequence encoding *resistin* obtained from a normal human not having a mutation in a *resistin* allele, wherein any difference between the nucleic acid sequence of the human suspected of having a mutation in the *resistin* allele and the nucleic acid sequence encoding *resistin* of the normal human not having a mutation in the *resistin* allele detects a mutation in the *resistin* allele in the human.

Further illustrated is a method of detecting a mutation in a *resistin* allele in a human. The method comprising comparing the genomic nucleic acid sequence encoding *resistin* of a human suspected of having a mutation in a *resistin* allele with the genomic nucleic acid sequence encoding *resistin* obtained from a normal human not having a mutation in a *resistin* allele, wherein any difference between the genomic nucleic acid sequence of the human suspected of having a mutation in the *resistin* allele and the genomic nucleic acid sequence encoding *resistin* of the normal human not having a mutation in the *resistin* allele detects a mutation in the *resistin* allele in the human.

Also illustrated is a method of treating a human patient afflicted with type 2 diabetes. The method comprises obtaining a biological sample from a human donor, isolating any cells from the biological sample, transfecting the cells with an isolated nucleic acid complementary to mammalian resistin or a fragment thereof, wherein when the nucleic acid is expressed in the cells expression of resistin in the cells is inhibited, and administering the cells to the human patient, wherein the presence of the cells in the human patient effects treatment of the type 2 diabetes.

The human donor may not be suffering from type 2 diabetes and wherein the human donor is syngeneic with the human patient. The human donor may be the human patient. The isolated nucleic acid may be operably linked to a promoter/regulatory sequence.

Further illustrated is a method of treating a human patient afflicted with type 2 diabetes. The method comprises obtaining a biological sample from a human donor, isolating any cells from the biological sample, transfecting the cells with a knock-out targeting vector comprising a first nucleic acid portion encoding a nucleic acid comprising a sequence 5' of the open reading frame encoding resistin and a second nucleic acid portion comprising a nucleic acid sequence 3' of the open reading frame encoding mammalian resistin, wherein when the cells are transfected with the knock-out targeting vector expression of resistin in the cells is inhibited, and administering the cells to the human patient, wherein the presence of the cells in the human patient effects treatment of the type 2 diabetes.

The invention additionally illustrates a method of increasing blood glucose levels in a mammal. The method comprises administering a effective amount of an isolated resistin polypeptide to the mammal, thereby increasing blood glucose levels in the mammal.

In addition, there is illustrated a method of increasing blood sugar level in a mammal. The method comprises administering to the mammal an effective amount of resistin, thereby increasing blood sugar level in the mammal.

There is further illustrated a method of increasing blood sugar level in a mammal, wherein the method comprises administering to the mammal an isolated recombinant cell transfected with an isolated nucleic acid encoding resistin wherein the nucleic acid is expressed in the cell, wherein the presence of the recombinant cells in the mammal effects an increased blond sugar level in the mammal.

Additionally, there is illustrated a method of treating a human patient afflicted with type 2 diabetes, wherein the method comprising administering to the human patient a recombinant cell comprising an isolated nucleic acid complementary to a nucleic acid encoding mammalian resistin or a fragment thereof, wherein the presence of the recombinant cell in the human patient effects treatment of the type 2 diabetes. diabetes.

There is further illustrated a method of treating a human patient afflicted with type 2 diabetes, wherein the method comprising administering to the human patient a recombinant cell comprising the knock-out targeting vector of the invention, wherein the presence of the recombinant cell in the human patient effects treatment of the type 2 diabetes.

Also illustrated is a method of increasing blood sugar level in a mammal. The method comprises administering to the mammal a recombinant cell comprising an isolated nucleic acid comprising mammalian resistin or a fragment thereof, wherein the presence of the recombinant cell in the mammal effects an increased blood sugar level in the mammal

The invention further illustrated a kit for alleviating type 2 diabetes. The kit comprising a resistin-inhibiting amount of a composition comprising an antibody that specifically binds with mammalian resistin or a fragment thereof and a pharmaceutically acceptable carrier, the kit further comprising an applicator, and an instructional material for the use thereof.

Further illustrated is a kit for alleviating type 2 diabetes. The kit comprises a resistin-inhibiting amount of a composition comprising an isolated nucleic acid complementary to nucleic acid encoding mammalian resistin or a fragment thereof, and a pharmaceutically acceptable carrier, the kit further comprising an applicator, and an instructional material for the use thereof.

There is also illustrated a kit for treating type 2 diabetes, wherein the kit comprises a resistin-inhibiting amount of a composition comprising an antibody that specifically binds with mammalian resistin or a fragment thereof and a pharmaceutically acceptable carrier, the kit further comprising an applicator, and an instructional material for the use thereof.

Further illustrated is a kit for treating type 2 diabetes, wherein the kit comprises a resistin-inhibiting amount of a composition comprising an isolated nucleic acid complementary to nucleic acid encoding mammalian resistin or a fragment thereof, and a pharmaceutically acceptable carrier, the kit further comprising an applicator, and an instructional material for the use thereof.

In addition, there is illustrated a kit for alleviating Syndrome X, wherein the kit comprises a resistin-inhibiting amount of a composition comprising an antibody that specifically binds with mammalian resistin or a fragment thereof and a pharmaceutically acceptable carrier, the kit further comprising an applicator, and an instructional material for the use thereof.

Further illustrated is a kit for alleviating Syndrome X, wherein the kit comprises a resistin-inhibiting amount of a composition comprising an isolated nucleic acid complementary to nucleic acid encoding mammalian resistin or a fragment thereof, and a pharmaceutically acceptable carrier, the kit further comprising an applicator, and an instructional material for the use thereof.

Additionally, there is illustrated a kit for treating Syndrome X, wherein the kit comprises a resistin-inhibiting amount of a composition comprising an antibody that specifically binds with mammalian resistin or a fragment thereof and a pharmaceutically acceptable carrier, the kit further comprising an applicator, and an instructional material for the use thereof.

Also illustrated is a kit for treating Syndrome X, the kit comprising a resistin-inhibiting amount of a composition comprising an isolated nucleic acid complementary to nucleic acid encoding mammalian resistin or a fragment thereof, and a pharmaceutically acceptable carrier, the kit further comprising an applicator, and an instructional material for the use thereof.

### BRIEF DESCRIPTION OF THE DRAWINGS

The foregoing summary, as well as the following detailed description of the invention, will be better understood when read in conjunction with the appended drawings. For the purpose of illustrating the invention, the drawings are presented.

In the drawings:

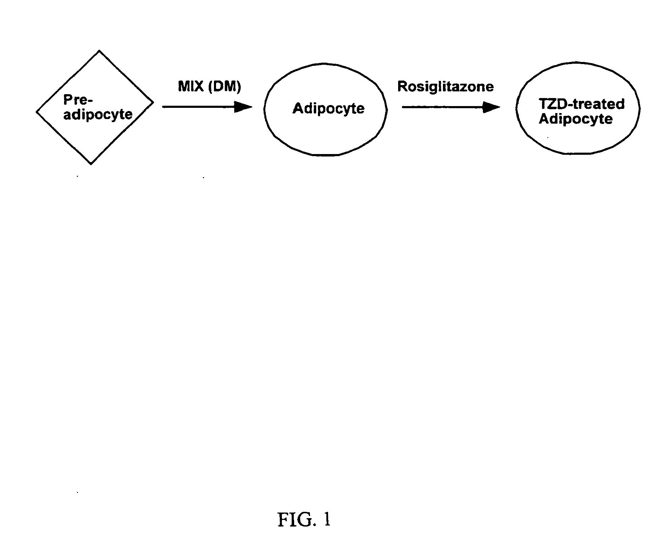

**Figure 1** is a diagram depicting the scheme used to identify and isolate a rosiglitazone down-regulated gene. Resistin cloning strategy is shown in outline form.

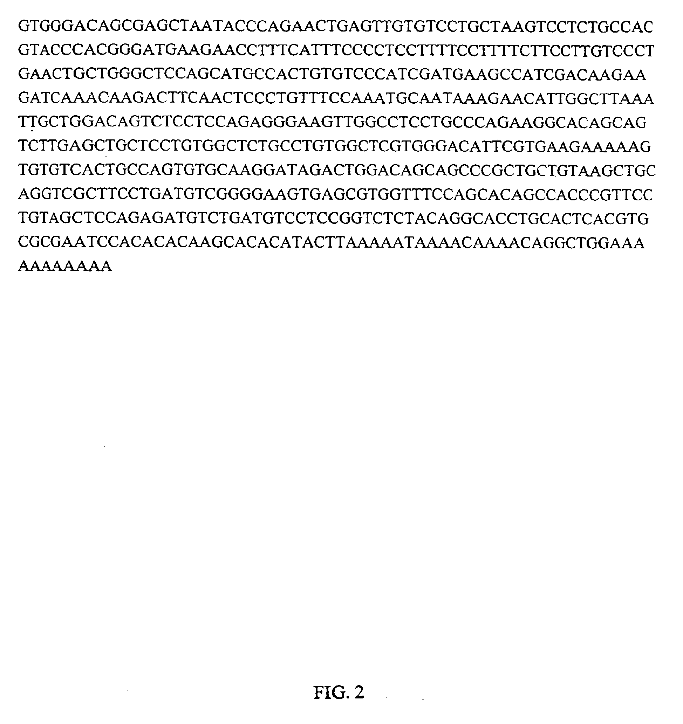

**Figure 2** is a diagram depicting the nucleic acid sequence of mouse *resistin* (SEQ ID NO:1).

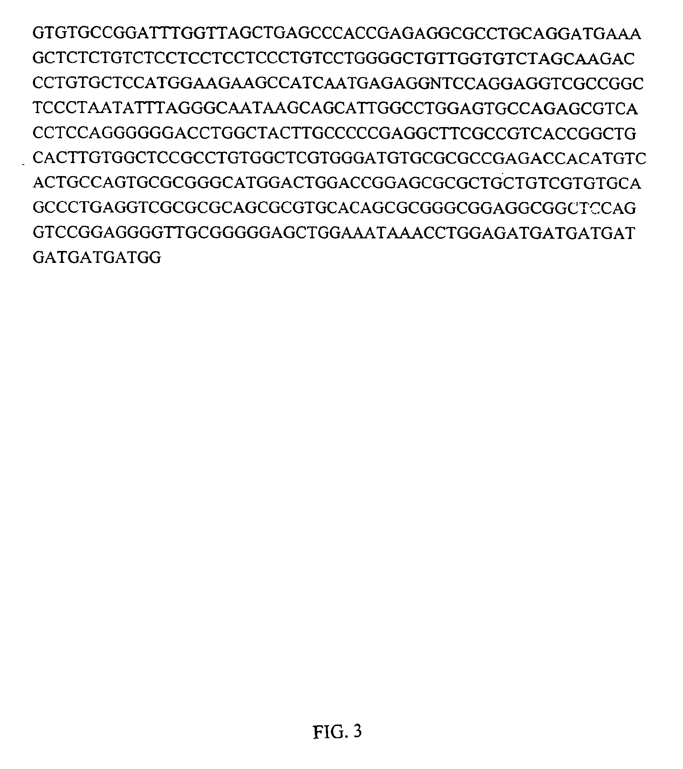

**Figure 3** is a diagram depicting the nucleic acid sequence of human *resistin* (SEQ ID NO:3), which is also referred to herein as human Resistin-like molecule A (hRELM-A). The mouse resistin protein sequence was used in a TBLASTN search of the human EST database and this sequence was identified by the search. The consensus merged cDNA sequence is shown in the middle line between the mouse and human sequences. ESTs have been obtained and the sequence depicted herein has been directly confirmed.

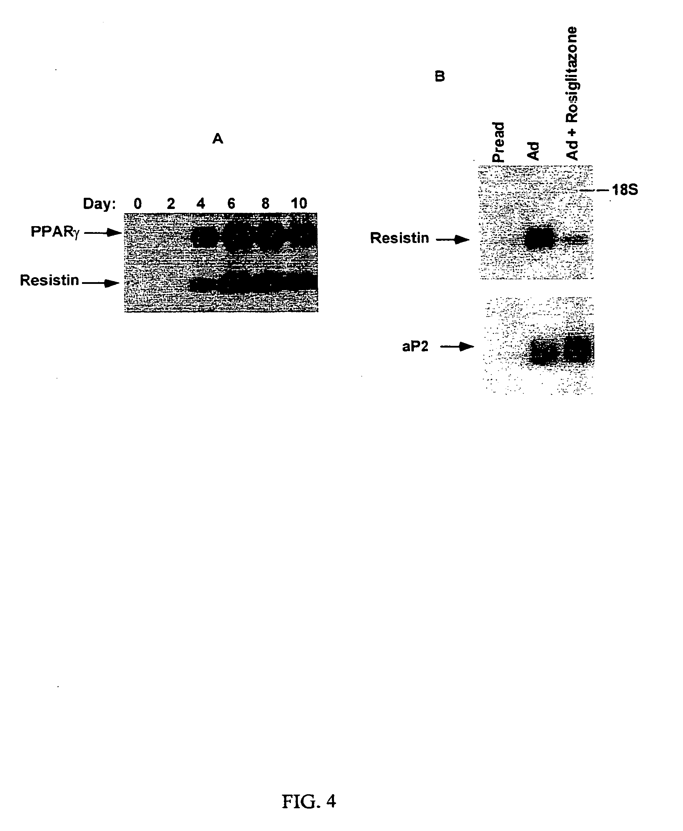

**Figure 4A** is an image depicting a Northern blot analysis demonstrating that *resistin* gene expression, like expression of PPARy, is induced during adipogenesis. Total RNA was obtained from 3T3-L1 adipocytes and preadipocytes and probed by Northern analysis. The image depicts a time course of resistin upregulation during adipocyte differentiation. Cells were differentiated using standard protocol for the indicated times, RNA was obtained, and Northern blot analysis was performed using probes specific for resistin and PPARγ.
**Figure 4B** is an image depicting a Northern blot analysis demonstrating that expression of resistin is down-regulated by rosiglitazone while expression of aF2 is not reduced. The image depicts the effect of 48 hours treatment of adipocytes with rosiglitazone (1 µM). RNA was obtained and probed using Northern analysis using probes specific for resistin and aP2.

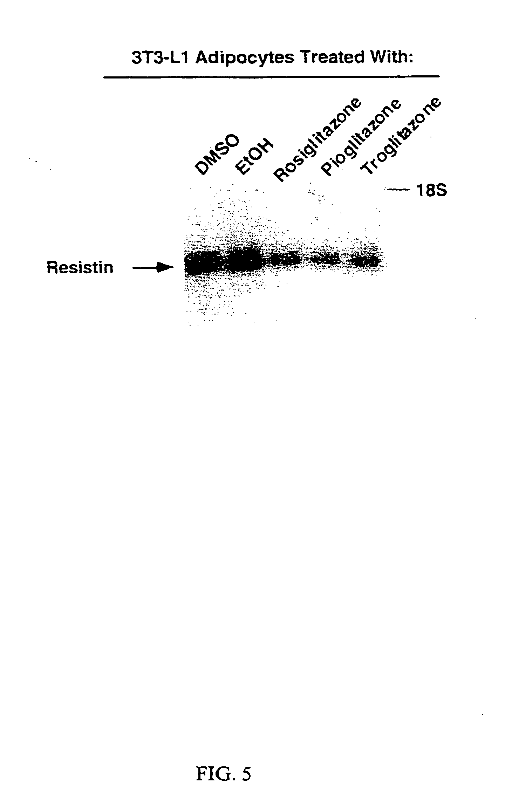

**Figure 5** is an image depicting that *resistin* expression is down-regulated by multiple TZDs (*e.g*. rosiglitazone, pioglitazone, and troglitazone). 3T3-L 1 cells were differentiated to adipocytes then treated with the indicated vehicles (ethanol [EtOH] or dimethylsulfoxide [DMSO]) or with the indicated TZD compounds (each at 1 µM). Then, RNA was prepared and analyzed using Northern blot analysis using resistin cDNA probe.

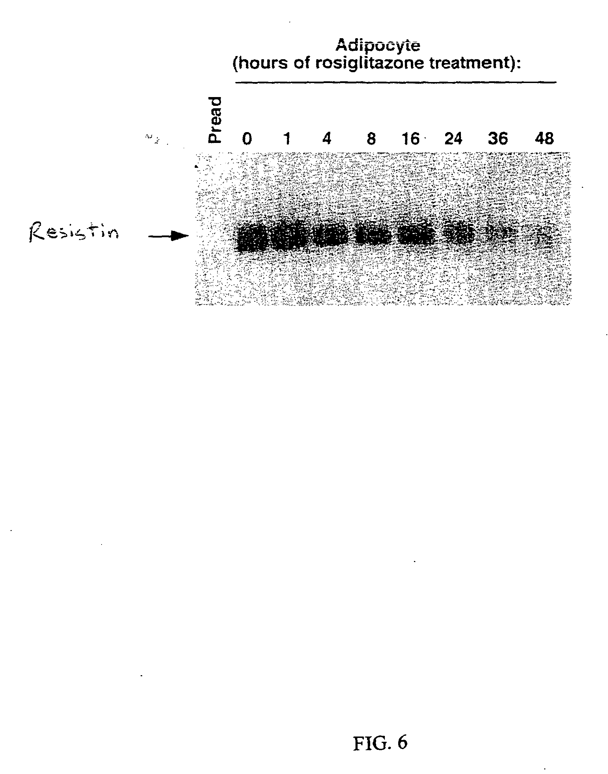

**Figure 6** is an image of a Northern blot depicting the time course of *resistin* expression down-regulation by rosiglitazone. BRL49653 (rosiglitazone) was used at a concentration of 1 µM in DMSO. The Northern blot was probed using mouse *resistin* cDNA. The position of resistin mRNA is indicated in the image. 3T3-L1 cells were differentiated to adipocytes then treated with rosiglitazone (1 µM) for the indicated times (in hours) before RNA was isolated. Resistin expression was examined using Northern analysis using resistin cDNA probe.

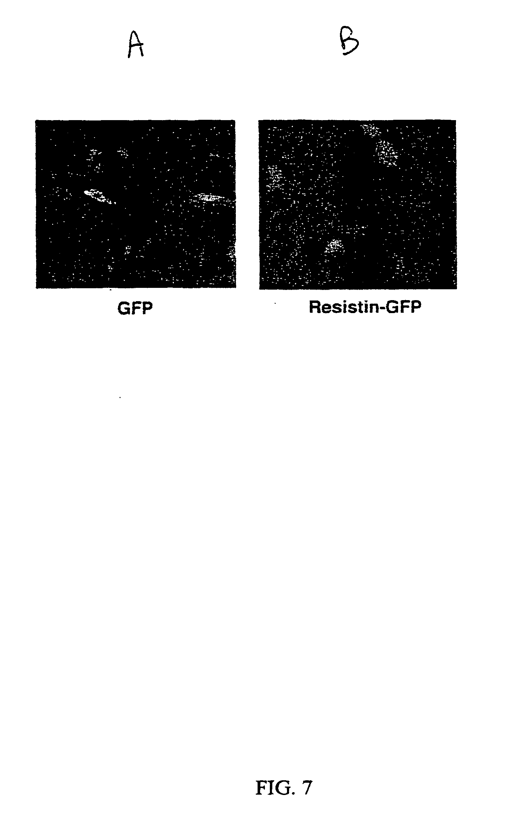

**Figure 7A** is an image depicting the expression and localization of green fluorescent protein (GFP) transfected into 293T cells. The cells were analyzed for green fluorescence by fluorescent microscopy under phase contrast.
**Figure 7B** is an image depicting the expression of nucleic acid encoding resistin further comprising a GFP tag polypeptide transfected into 293T cells. The resistin-GFP fusion protein localized to the Golgi apparatus of 293T transfected cells. Resistin cDNA was covalently linked, in frame, at its C-terminus to green fluorescent protein (GFP) and the fusion protein was subcloned into a eukaryotic expression vector and then transfected into 293T human embryonic kidney cells. The cells were analyzed for green fluorescence by fluorescent microscopy under phase contrast.

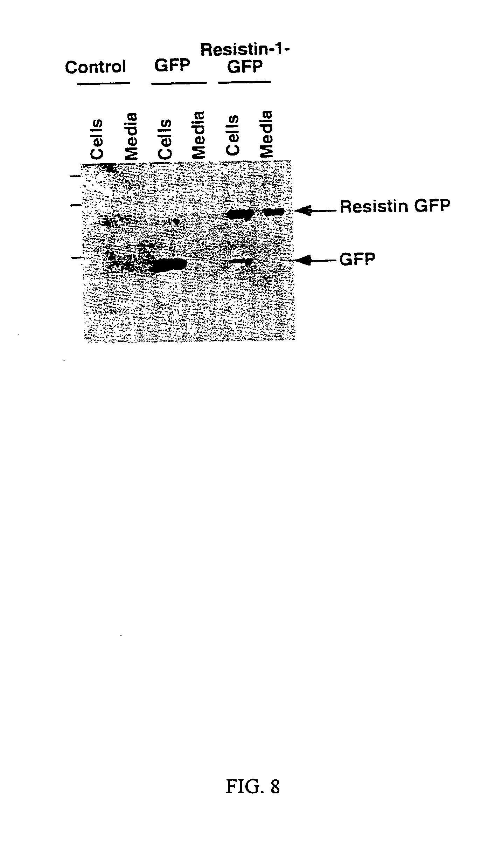

**Figure 8** is an image of a Western blot depicting that *resistin*-GFP fusion protein is secreted into the medium by transfected 293T cells. 293T cells were transfected as described elsewhere herein, then cell extracts were prepared and subjected to SDS-PAGE. Aliquots of media obtained from the cell culture dishes were processed in parallel with the cell extract samples. Proteins were transferred to nitrocellulose filters and the blots were probed with anti-GFP antibody using Western blot analysis.

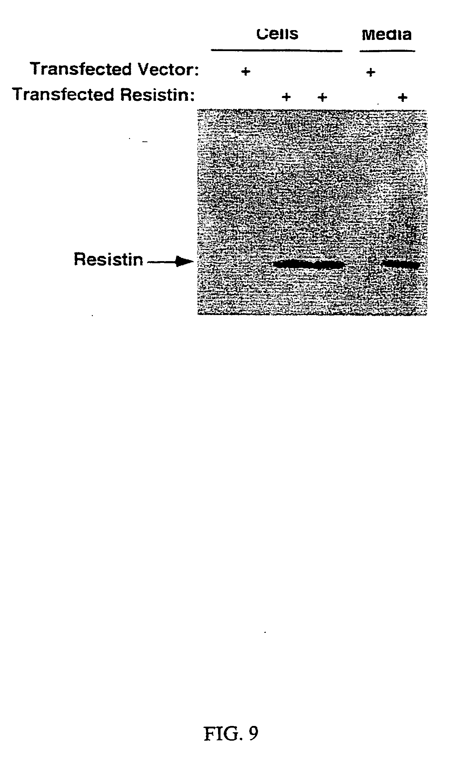

**Figure 9** is an image depicting a Western blot demonstrating that resistin is secreted into the medium by transfected 293T cells. Resistin cDNA was subcloned into pCMX expression vector and transfected into 293T cells. Cell extracts were prepared and subjected to SDS-PAGE. Aliquots of media obtained from the cell culture dishes were processed in parallel with the cell extract samples. Proteins were transferred to nitrocellulose filters and the filters were probed with anti-resistin polyclonal antibody using Western blot analysis.

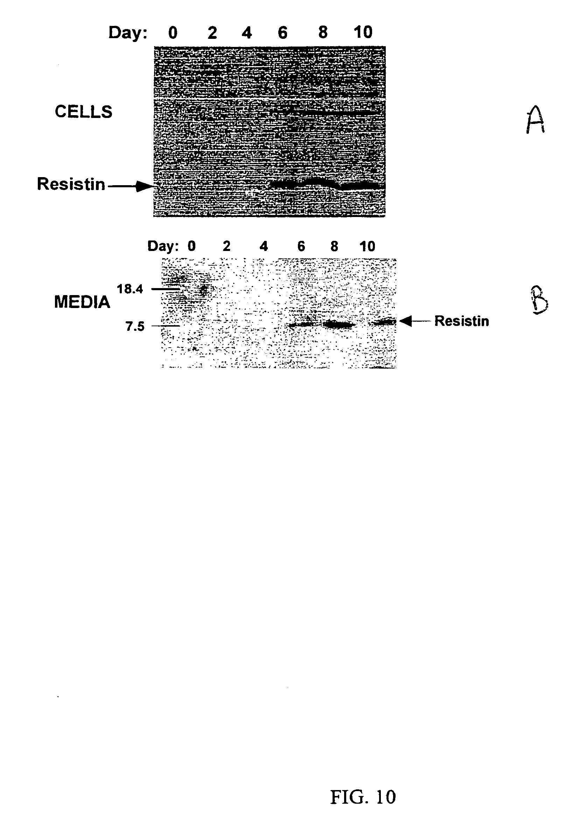

**Figure 10A** is an image depicting a Western blot demonstrating the induction of *resistin* during adipogenesis. 3T3-L1 cells were differentiated using a standard protocol as described elsewhere herein. Cell extracts were prepared and the proteins were separated using SDS-PAGE. The proteins were then subjected to Western blot analysis using anti-resistin antiserum as described elsewhere herein.
**Figure 10B** is an image depicting a Western blot demonstrating the induction of *resistin* during adipogenesis and secretion of the protein into the cell culture medium. 3T3-L1 cells were differentiated using a standard protocol as described elsewhere herein. Aliquots obtained from culture media were electrophoresed using SDS-PAGE and the proteins were subjected to Western blot analysis using anti-resistin antiserum as described elsewhere herein.

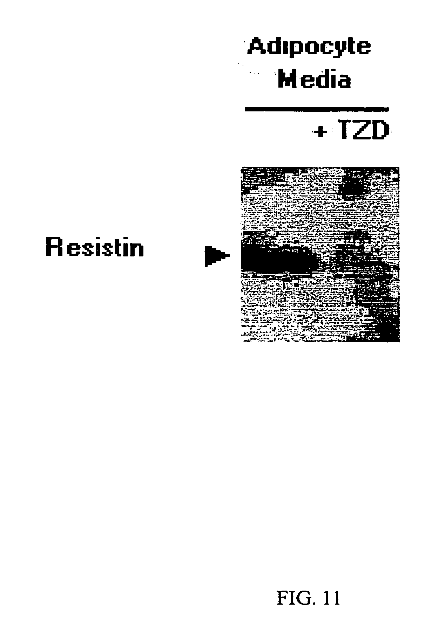

**Figure 11** is an image depicting a Western blot demonstrating that resistin secretion from adipocytes is reduced by exposure of the cells to TZDs. 3T3-L1 adipocytes were exposed to rosiglitazone (1 µM in DMSO) or control medium (DMSO only) for 4 days. Aliquots of media were subjected to Western blot analysis and were immunostained to detect resistin protein.

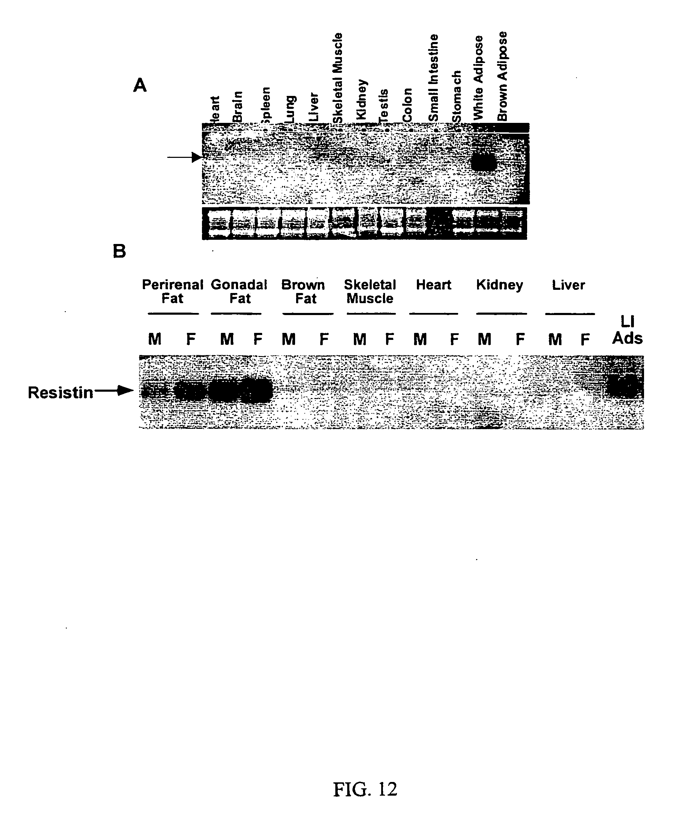

**Figure 12A** is an image depicting a Northern blot depicting the tissue-specific expression of *resistin.* A multiple mouse tissue Northern blot (Clontech Labs.) comprising RNA isolated from heart, brain, spleen, lung, liver, skeletal muscle, kidney, and testis was probed using mouse *resistin* cDNA as described elsewhere herein. Total RNA was prepared from the indicated mouse tissues, and 10 µg of RNA was electrophoresed and subjected to Northern blot analysis using the resistin cDNA probe. Along the bottom edge of the Northern blot is an image depicting an ethidium bromide stain of the same gel used for Northern blotting demonstrating the loading of RNA in each gel well. The location of 28S and 18S RNA markers is indicated for comparison of lane to lane loading variability.
**Figure 12B** is an image depicting a Northern blot demonstrating that resistin gene expression is tissue specific. From left to right, the gel was loaded with RNA isolated from the following sources: perirenal fat, gonadal fat, brown fat, skeletal muscle, heart, kidney, and liver.

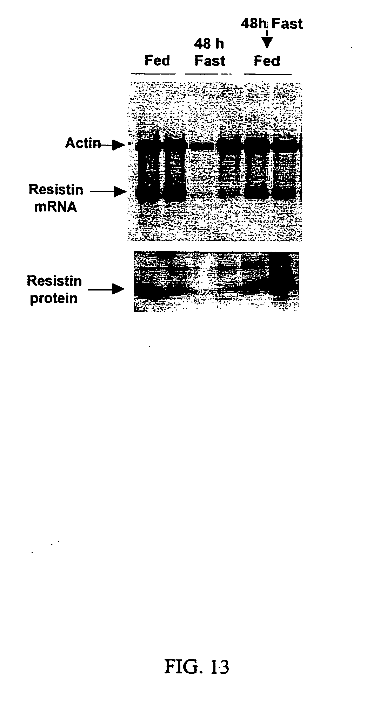

**Figure 13A** is an image depicting a Northern blot demonstrating that *resistin* expression can be reduced by fasting. The animals used in this experiment were either maintained on normal chow or fasted for a period of 48 hours with free access to water. The fasted group were then given access to normal chow *ad libidum* following the fasting period. At the end of the experiment, the animals were euthanized using CO₂ inhalation. The tissues were harvested immediately and RNA was isolated therefrom. The RNA was examined using Northern blot analysis using a probe for resistin or actin.
**Figure 13B** is an image depicting a Western blot demonstrating that the resistin protein level in an animal is reduced by fasting. The animals used in this experiment were either maintained on normal chow or fasted for a period of 48 hours with free access to water. The fasted group were then given access to normal chow *ad libidum* following the fasting period. At the end of the experiment, the animals were euthanized using CO₂ inhalation. The tissues were harvested immediately and proteins were isolated therefrom. The proteins were examined using Western blot analysis using anti-resistin antiserum.

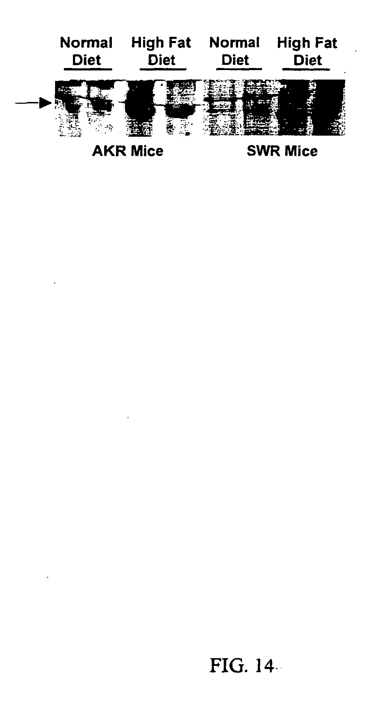

**Figure 14** is an image depicting a Western blot demonstrating that the level of resistin increases in the serum of animals fed a high-fat diet in both AKR and SWR mice. The animals used in this experiment were either maintained on normal chow or high fat chow for a period of 6 weeks with free access to water. At the end of the experiment, the animals were euthanized using CO₂ inhalation. The tissues were harvested immediately and proteins were isolated therefrom. The proteins were examined using Western blot analysis using anti-resistin antiserum.

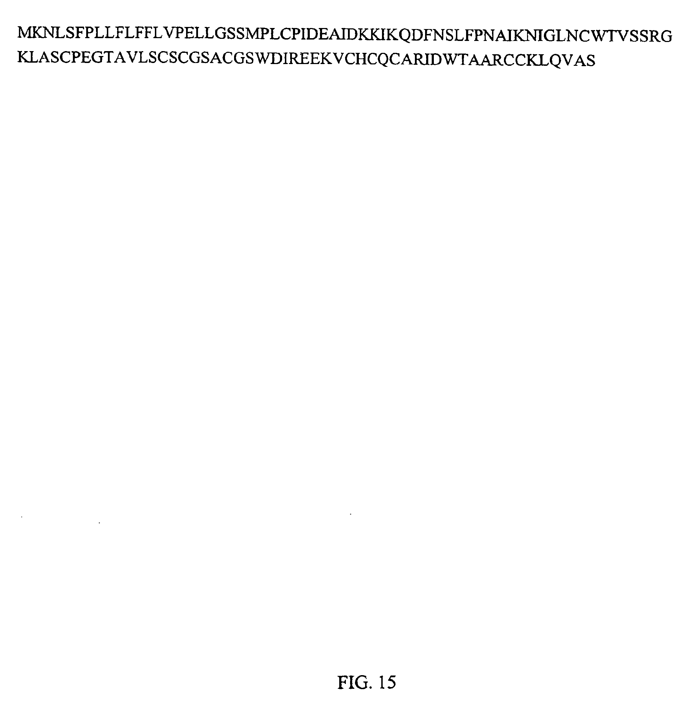

**Figure 15** is a diagram depicting the amino acid sequence of mouse resistin (SEQ ID NO:2).

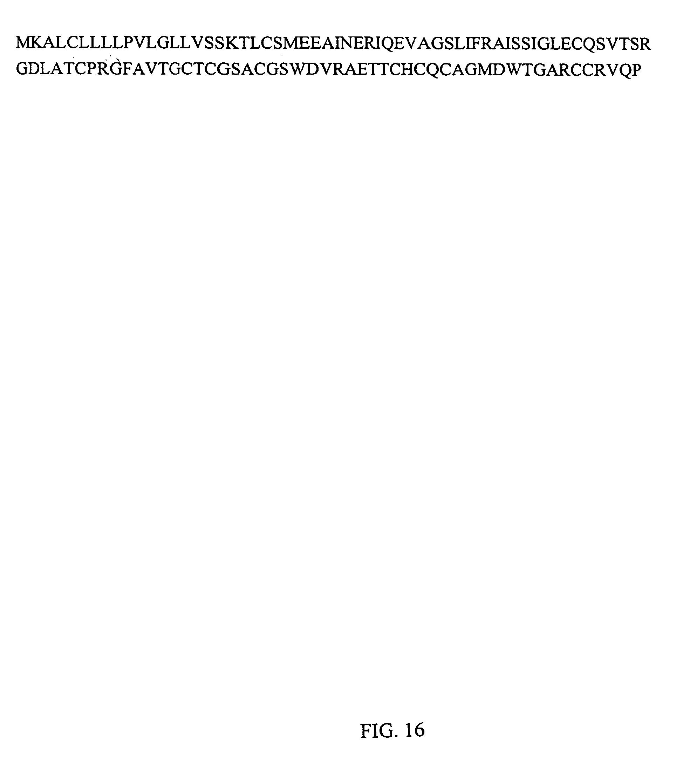

**Figure 16** is a diagram depicting the amino acid sequence of human *resistin* (SEQ ID NO:4), which is also referred to herein as human Resistin-like molecule A (hRELM-A).

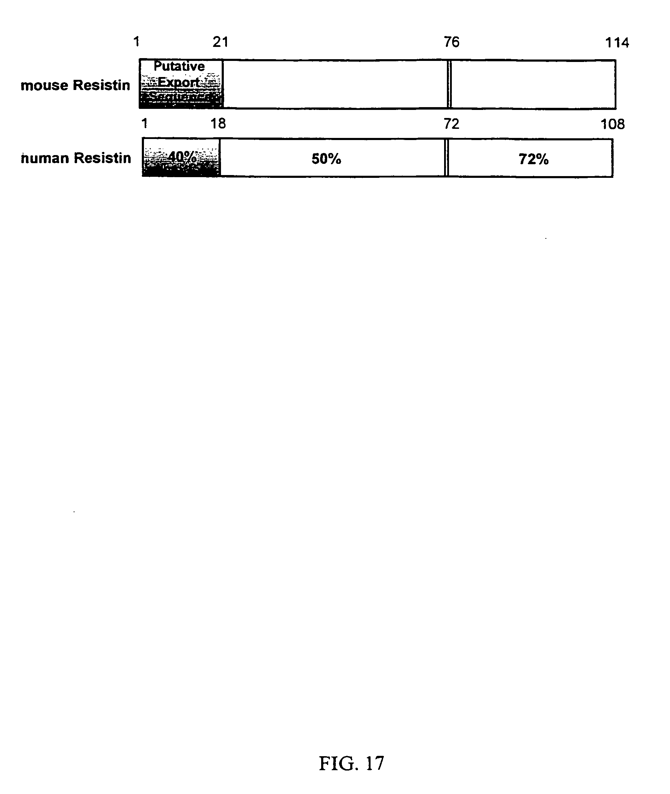

**Figure 17** is a diagram depicting a comparison of the various domains of mouse resistin and human RELM-A proteins. Percent identity is indicated for each domain.

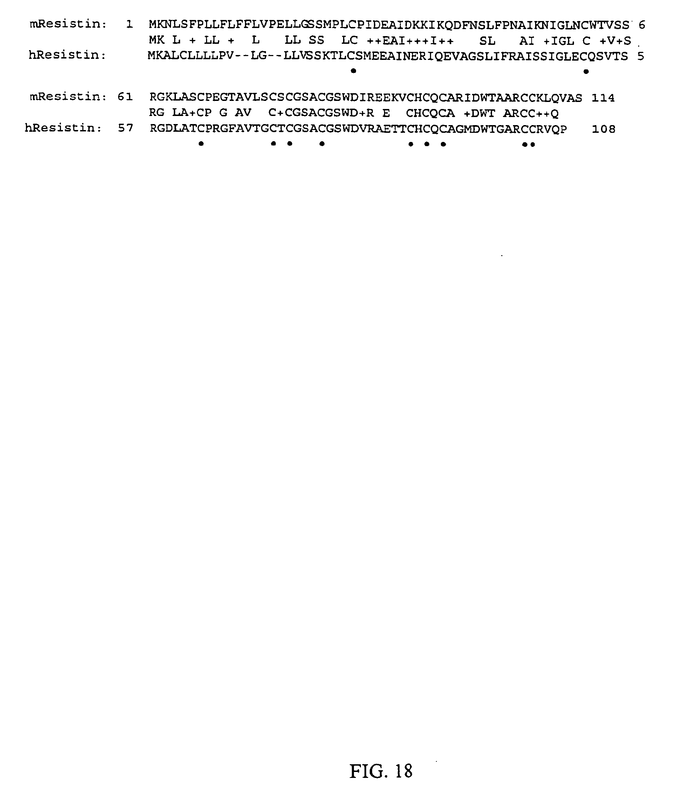

**Figure 18** is an image depicting a comparison of the amino acid sequence of mouse resistin with hRELM-A. The consensus sequence is depicted between the mouse and human sequences. Conservative amino acid changes are indicated by "+". Further, the locations of the invariable cysteine residues are indicated by as "•".

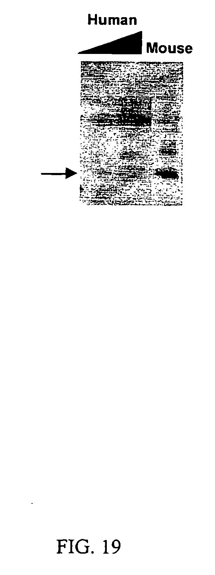

**Figure 19** is an image depicting a Western blot demonstrating the detection of a resistin-immunoreactive protein in human serum. Human serum (5 µl and 10 µl) were electrophoresed in parallel with horse serum. The proteins were examined using Western blot analysis using anti-resistin antiserum which was produced by immunizing rabbits with mouse resistin as described elsewhere herein.

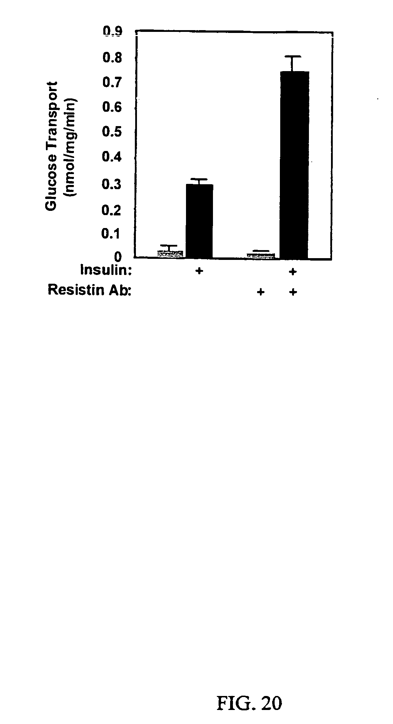

**Figure 20** is a graph depicting the fact that neutralization of resistin using anti-resistin antiserum enhances basal and insulin-stimulated glucose uptake in adipocytes.

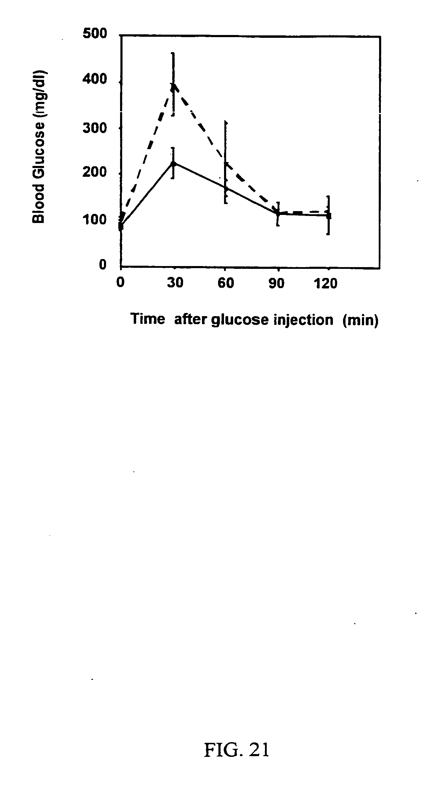

**Figure 21** is a graph depicting the fact that resistin administration reduces glucose tolerance.

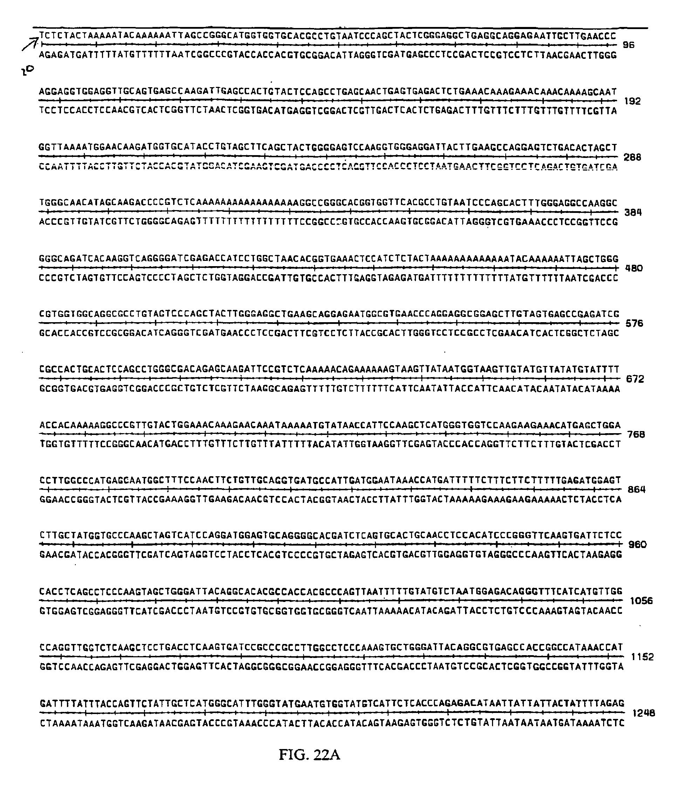

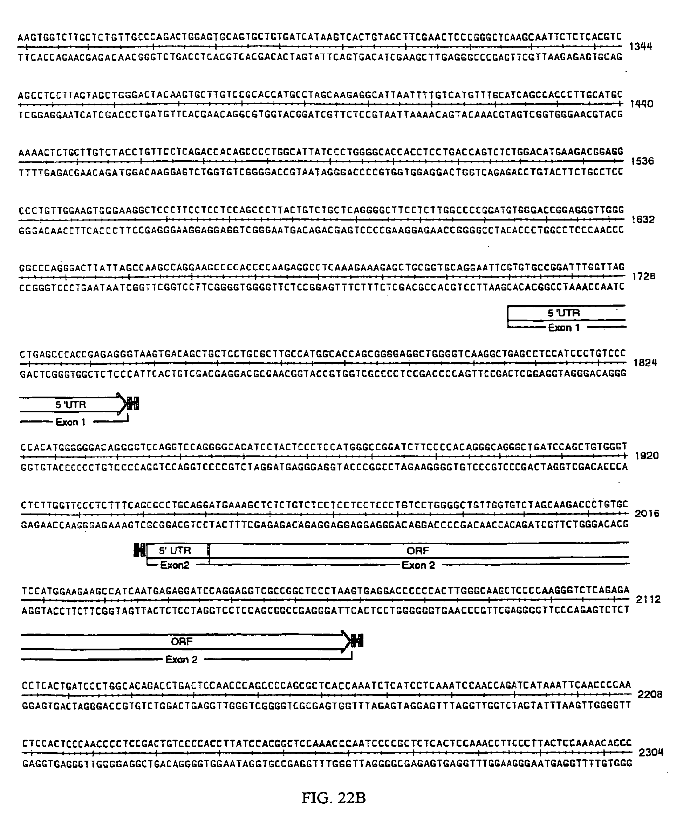

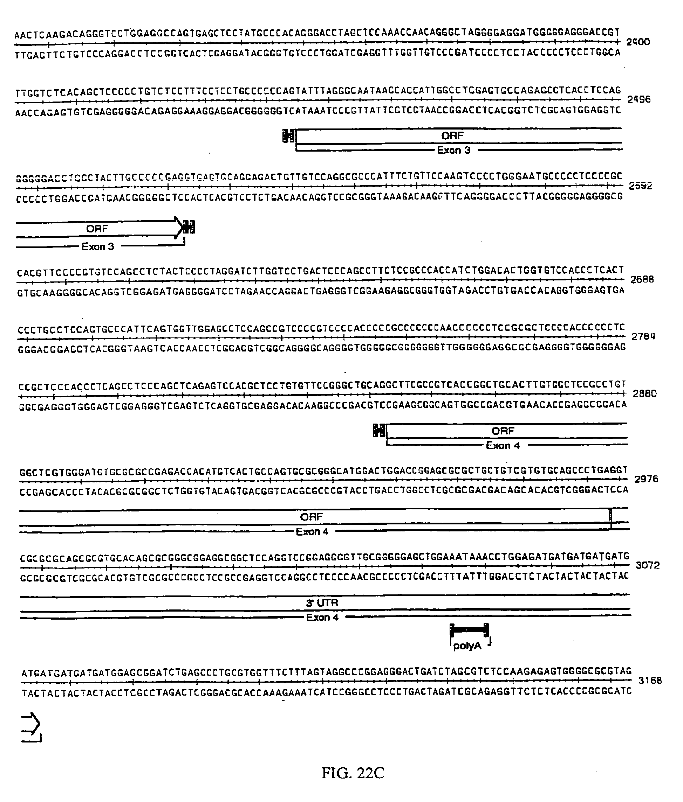

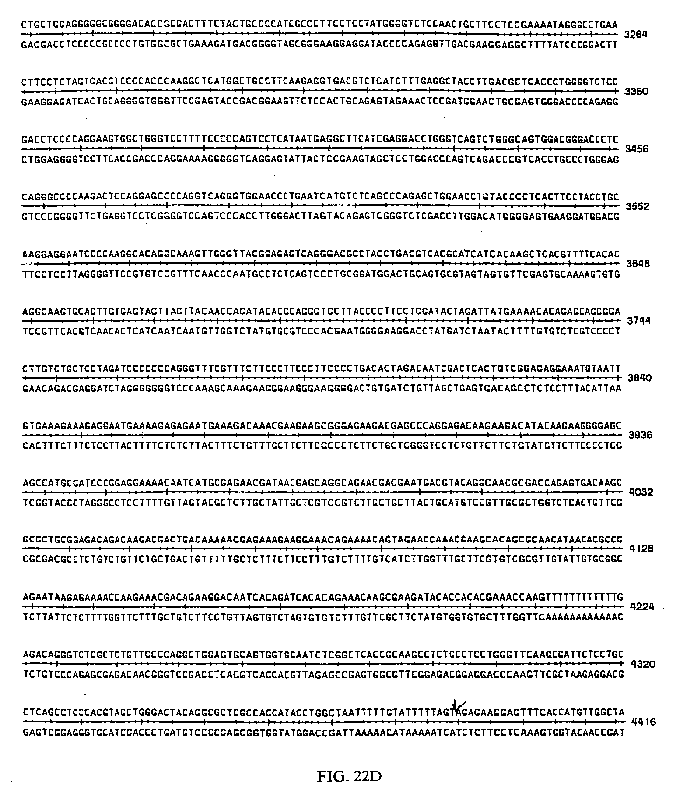

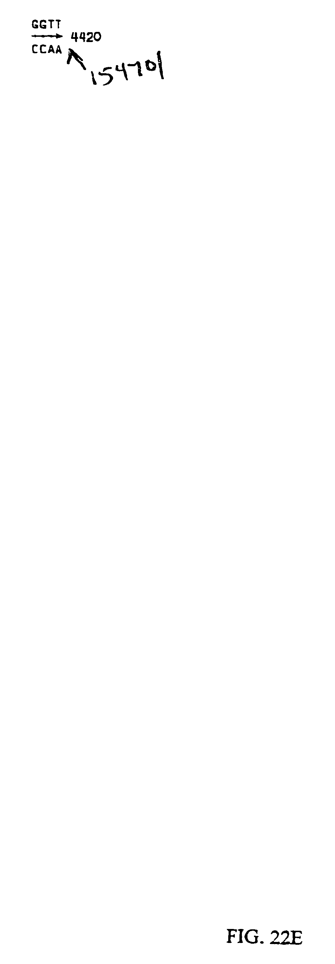

**Figure 22**, comprising Figures A-E, depicts the nucleic acid sequence of human *resistin* gene (SEQ ID NO:5) which comprises from about nucleotide 159120 to about 154701 of the sequence of the contig from human Chromosome 19 (GenBank Acc. No. AC008763). The sequence depicted along the bottom strand of the figures commences at nucleotide 1, which corresponds with nucleotide number 159120 of GenBank Acc. No. AC008763, and ends at about nucleotide 4420, which corresponds with nucleotide number 154701 of GenBank Acc. No. AC008763.

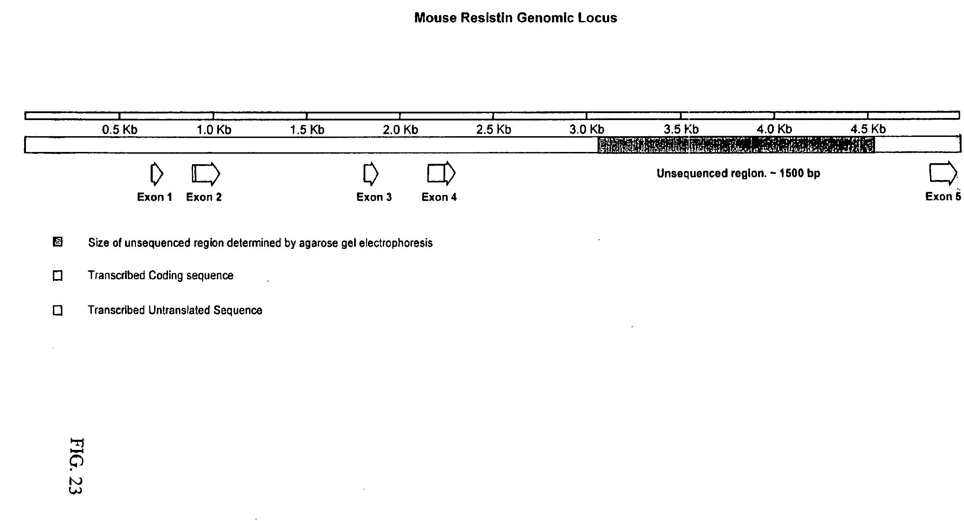

**Figure 23** is a diagram illustrating the organization of the mouse *resistin* genomic locus.

### DETAILED DESCRIPTION OF THE INVENTION

The invention illustrates a novel nucleic acid encoding a mammalian TZD-suppressible gene 1 (TSG-1) now referred to herein as "*resistin*" and protein encoded thereby. It has been discovered, as disclosed herein, that expression of this gene is markedly and specifically reduced by TZDs, which are powerful antidiabetic compounds. This is important because, until the present invention, no target gene(s) which is down-regulated by TZD had been identified. The first such gene to be identified is *resistin* as demonstrated by the data disclosed herein.

As stated previously elsewhere herein, TZDs represent a breakthrough in the treatment of NIDDM. New insights into the mechanism of TZD action in NIDDM are likely to result from basic research into the mode of operation of these compounds. Discovery of new TZD-dependent PPARγ target genes contributes to a conceptual bridge between TZD activation of PPARγ and insulin action. In addition, better understanding of the mechanistic relationship between TZD binding to PPARγ and enhanced insulin action *in vivo* will lead to the development of additional therapies directed to this TZD receptor. For example, phosphorylation of PPARγ negatively regulates its function, suggesting that therapies aimed at increasing the dephosphorylated state might synergize with TZDs in potentiating insulin action.

In addition, several studies have suggested that at least certain TZDs may be too toxic to be useful as antidiabetics. As discussed elsewhere herein, without wishing to be bound by any particular theory, it may be that TZDs are toxic in that they bind to PPARγ thereby deleteriously affecting various cell processes mediated by PPARγ. Therefore, therapeutics based on modulation of *resistin* expression are useful to overcome difficulties associated with AZD binding with PPARγ which can affect a variety of cell processes some of which are required for homeostasis. By providing a more specific target for antidiabetic drugs, regulation of *resistin* expression should overcome these difficulties associated with treatments that affect PPARγ function in a cell.

Furthermore, study of the precise mechanism(s) by which TZDs effect their dramatic antidiabetic activity is of paramount importance in the development of efficacious treatment strategies for this important disease which afflicts so many humans and for which there is no effective cure.

The data disclosed herein suggest that down-regulation of *resistin* expression which results from TZD binding with PPARγ is an important part of the TZD-mediated antidiabetic responses. The instant invention provides *in vitro* and *in vivo* models for the study of the function and role(s) of *resistin* in cell processes, diabetes, and Syndrome X, as well as for the development of therapeutics useful for treating diabetes and Syndrome X.

### Definitions

As used herein, each of the following terms has the meaning associated with it in this section.

The articles "a" and "an" are used herein to refer to one or to more than one (*i.e.,* to at least one) of the grammatical object of the article. By way of example, "an element" means one element or more than one element.

As used herein, the term "adjacent" is used to refer to nucleotide sequences which are directly attached to one another, having no intervening nucleotides. By way of example, the pentanucleotide 5'-AAAAA-3' is adjacent the trinucleotide 5'-TTT-3' when the two are connected thus: 5'-AAAAATTT-3' or 5'-TTTAAAAA-3', but not when the two are connected thus: 5'-AAAAACTTT-3'.

As used herein, amino acids are represented by the full name thereof, by the three letter code corresponding thereto, or by the one-letter code corresponding thereto, as indicated in the following table:

| Full Name | Three-Letter Code | One-Letter Code |
|---|---|---|
| Aspartic Acid | Asp | D |
| Glutamic Acid | Glu | E |
| Lysine | Lys | K |
| Arginine | Arg | R |
| Histidine | His | H |
| Tyrosine | Tyr | Y |
| Cysteine | Cys | C |
| Asparagine | Asn | N |
| Glutamine | Gln | Q |
| Serine | Ser | S |
| Threonine | Thr | T |
| Glycine | Gly | G |
| Alanine | Ala | A |
| Valine | Val | V |
| Leucine | Leu | L |
| Isoleucine | Ile | I |
| Methionine | Met | M |
| Proline | Pro | P |
| Phenylalanine | Phe | F |
| Tryptophan | Trp | W |

As used herein, to "alleviate" type 2 diabetes and/or Syndrome X means reducing the severity of one or more symptoms of type 2 diabetes and/or Syndrome X. This can include, but is not limited to, reducing the amount of insulin required by a human or veterinary patient compared with the amount of insulin required by the patient prior to or in the absence of the method of treatment.

"Antisense" refers particularly to the nucleic acid sequence of the non-coding strand of a double stranded DNA molecule encoding a protein, or to a sequence which is substantially homologous to the non-coding strand. As defined herein, an antisense sequence is complementary to the sequence of a double stranded DNA molecule encoding a protein. It is not necessary that the antisense sequence be complementary solely to the coding portion of the coding strand of the DNA molecule. The antisense sequence may be complementary to regulatory sequences specified on the coding strand of a DNA molecule encoding a protein, which regulatory sequences control expression of the coding sequences.

By the term "applicator" as the term is used herein, is meant any device including, but not limited to, a hypodermic syringe, a pipette, and the like, for administering the *resistin* nucleic acid. protein, and/or composition of the invention to a mammal.

"Biological sample," as that term is used herein, means a sample obtained from an animal that can be used to assess the level of resistin expression, the level of resistin protein present, or both. Such a sample includes, but is not limited to, a blood sample, a white adipose tissue sample, and a brown adipose tissue sample.

By "candidate antidiabetic drug candidate," as the term is used herein, is meant a compound that when contacted with a cell, reduces the level of expression of *resistin* in the cell compared with the level of *resistin* expression in that cell prior to contacting the cell with the compound or which reduces the level of expression in the cell compared with the level of *resistin* expression in an otherwise identical cell which is not contacted with the compound.

By "complementary to a portion or all of the nucleic acid encoding *resistin*" is meant a sequence of nucleic acid which does not encode *resistin* protein. Rather, the sequence which is being expressed in the cells is identical to the non-coding strand of the nucleic acid encoding resistin and thus, does not encode resistin protein.

The terms "complementary" and "antisense" as used herein, are not entirely synonymous. "Antisense" refers particularly to the nucleic acid sequence of the non-coding strand of a double stranded DNA molecule encoding a protein, or to a sequence which is substantially homologous to the non-coding strand. "Complementary" as used herein refers to the broad concept of subunit sequence complementarity between two nucleic acids, *e.g*., two DNA molecules. When a nucleotide position in both of the molecules is occupied by nucleotides normally capable of base pairing with each other, then the nucleic acids are considered to be complementary to each other at this position. Thus, two nucleic acids are complementary to each other when a substantial number (at least 50%) of corresponding positions in each of the molecules are occupied by nucleotides which normally base pair with each other (*e.g*., A:T and G:C nucleotide pairs). As defined herein, an antisense sequence is complementary to the sequence of a double stranded DNA molecule encoding a protein. It is not necessary that the antisense sequence be complementary solely to the coding portion of the coding strand of the DNA molecule. The antisense sequence may be complementary to regulatory sequences specified on the coding strand of a DNA molecule encoding a protein, which regulatory sequences control expression of the coding sequences.

A "coding region" of a gene consists of the nucleotide residues of the coding strand of the gene and the nucleotides of the non-coding strand of the gene which are homologous with or complementary to, respectively, the coding region of an mRNA molecule which is produced by transcription of the gene.

A "coding region" of an mRNA molecule also consists of the nucleotide residues of the mRNA molecule which are matched with an anticodon region of a transfer RNA molecule during translation of the mRNA molecule or which encode a stop codon. The coding region may thus include nucleotide residues corresponding to amino acid residues which are not present in the mature protein encoded by the mRNA molecule (e.g. amino acid residues in a protein export signal sequence).

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. Nucleotide sequences that encode proteins and RNA may include introns.

"Expression vector" refers to a vector comprising a recombinant polynucleotide comprising expression control sequences operatively linked to a nucleotide sequence to be expressed. An expression vector comprises sufficient cisacting elements for expression; other elements for expression can be supplied by the host cell or in an in vitro expression system. Expression vectors include all those known in the art, such as cosmids, plasmids (*e.g*., naked or contained in liposomes) and viruses (*e.g*., retroviruses, adenoviruses, and adeno-associated viruses) that incorporate the recombinant polynucleotide.

A first region of an oligonucleotide "flanks" a second region of the oligonucleotide if the two regions are adjacent one another or if the two regions are separated by no more than about 1000 nucleotide residues, and preferably no more than about 100 nucleotide residues.

As used herein, the term "fragment" as applied to a nucleic acid, may ordinarily be at least about 20 nucleotides in length, typically, at least about 50 nucleotides, more typically, from about 50 to about 100 nucleotides, preferably, at least about 100 to about 200 nucleotides, even more preferably, at least about 200 nucleotides to about 300 nucleotides, yet even more preferably, at least about 300 to about 350, even more preferably, at least about 350 nucleotides to about 500 nucleotides, yet even more preferably, at least about 500 to about 1000, even more preferably, at least about 1000 nucleotides to about 1500 nucleotides, yet even more preferably, at least about 1500 to about 2000, even more preferably, at least about 2000 nucleotides to about 3000 nucleotides, and most preferably, the nucleic acid fragment will be greater than about 3000 nucleotides in length.

As applied to a protein, a "fragment" of resistin is about 20 amino acids in length. More preferably, the fragment of a resistin is about 30 amino acids, even more preferably, at least about 40, yet more preferably, at least about 60, even more preferably, at least about 80, yet more preferably, at least about 100, even more preferably, about 100, and more preferably, at least about 114 amino acids in length.

A "genomic DNA" is a DNA strand which has a nucleotide sequence homologous with a gene. By way of example, both a fragment of a chromosome and a cDNA derived by reverse transcription of a mammalian mRNA are genomic DNAs.

By the term "glucose uptake-enhancing amount" is meant any amount of a substance or molecule that increases the uptake glucose by a cell to a detectable degree. Any glucose uptake assay can be used to assess whether a substance mediates a detectable increase in glucose uptake in a cell. Such assays are exemplified herein; however, the present invention is not limited to any particular glucose uptake assay. Rather, the invention encompasses any glucose uptake assay known in the art or to be developed in the future.

"Homologous" as used herein, refers to the subunit sequence similarity between two polymeric molecules, *e.g*., between two nucleic acid molecules, *e.g*., two DNA molecules or two RNA molecules, or between two polypeptide molecules. When a subunit position in both of the two molecules is occupied by the same monomeric subunit, *e.g*., if a position in each of two DNA molecules is occupied by adenine, then they are homologous at that position. The homology between two sequences is a direct function of the number of matching or homologous positions, *e.g*., if half (*e.g*., five positions in a polymer ten subunits in length) of the positions in two compound sequences are homologous then the two sequences are 50% homologous, if 90% of the positions, *e.g*., 9 of 10, are matched or homologous, the two sequences share 90% homology. By way of example, the DNA sequences 3'ATTGCC5' and 3'TATGGC share 50% homology.

As used herein, "homology" is used synonymously with "identity."

In addition, when the terms "homology" or "identity" are used herein to refer to the nucleic acids and proteins, it should be construed to be applied to homology or identity at both the nucleic acid and the amino acid sequence levels.

A first oligonucleotide anneals with a second oligonucleotide with "high stringency" or "under high stringency conditions" if the two oligonucleotides anneal under conditions whereby only oligonucleotides which are at least about 60%, more preferably at least about 65%, even more preferably at least about 70%, yet more preferably at least about 80%, and preferably at least about 90% or, more preferably, at least about 95% complementary anneal with one another. The stringency of conditions used to anneal two oligonucleotides is a function of, among other factors, temperature, ionic strength of the annealing medium, the incubation period, the length of the oligonucleotides, the G-C content of the oligonucleotides, and the expected degree of non-homology between the two oligonucleotides, if known. Methods of adjusting the stringency of annealing conditions are known (*see*, *e.g*., Sambrook et al., 1989, Molecular Cloning: A Laboratory Manual, Cold Spring Harbor Laboratory, New York).

The determination of percent identity between two nucleotide or amino acid sequences can be accomplished using a mathematical algorithm. For example, a mathematical algorithm useful for comparing two sequences is the algorithm of Karlin and Altschul (1990, Proc. Natl. Acad. Sci. USA 87:2264-2268), modified as in Karlin and Altschul (1993, Proc. Natl. Acad. Sci. USA 90:5873-5877). This algorithm is incorporated into the NBLAST and XBLAST programs of Altschul, et al. (1990, J. Mol. Biol. 215:403-410), and can be accessed, for example, at the National Center for Biotechnology Information (NCBI) world wide web site having the universal resource locator "http://www.ncbi.nlm.nih.gov/BLAST/". BLAST nucleotide searches can be performed with the NBLAST program (designated "blastn" at the NCBI web site), using the following parameters: gap penalty = 5; gap extension penalty = 2; mismatch penalty = 3; match reward = 1; expectation value 10.0; and word size = 11 to obtain nucleotide sequences homologous to a nucleic acid described herein. BLAST protein searches can be performed with the XBLAST program (designated "blastn" at the NCBI web site) or the NCBI "blastp" program, using the following parameters: expectation value 10.0, BLOSUM62 scoring matrix to obtain amino acid sequences homologous to a protein molecule described herein.

To obtain gapped alignments for comparison purposes, Gapped BLAST can be utilized as described in Altschul et al. (1997, Nucleic Acids Res. 25:3389-3402). Alternatively, PSI-Blast or PHI-Blast can be used to perform an iterated search which detects distant relationships between molecules (id.) and relationships between molecules which share a common pattern. When utilizing BLAST, Gapped BLAST, PSI-Blast, and PHI-Blast programs, the default parameters of the respective programs (*e.g*., XBLAST and NBLAST) can be used. See http://www.ncbi.nlm.nih.gov.

The percent identity between two sequences can be determined using techniques similar to those described above, with or without allowing gaps. In calculating percent identity, typically exact matches are counted.

As used herein, the terms "gene" and "recombinant gene" refer to nucleic acid molecules comprising an open reading frame encoding a polypeptide of the invention. Such natural allelic variations can typically result in 1-5% variance in the nucleotide sequence of a given gene. Alternative alleles can be identified by sequencing the gene of interest in a number of different individuals. This can be readily carried out by using hybridization probes to identify the same genetic locus in a variety of individuals. Any and all such nucleotide variations and resulting amino acid polymorphisms or variations that are the result of natural allelic variation and that do not alter the functional activity are intended to be within the scope of the invention.

Moreover, nucleic acid molecules encoding proteins of the invention from other species (homologs), which have a nucleotide sequence which differs from that of the mouse proteins described herein are within the scope of the invention. Nucleic acid molecules corresponding to natural allelic variants and homologs of a cDNA of the invention can be isolated based on their identity to mouse nucleic acid molecules using the mouse cDNAs, or a portion thereof, as a hybridization probe according to standard hybridization techniques under stringent hybridization conditions. For example, a homolog of a mouse resistin protein of the invention can be isolated based on its hybridization with a nucleic acid molecule encoding all or part of mouse resistin under high stringency conditions.

As used herein, an "instructional material" includes a publication, a recording, a diagram, or any other medium of expression which can be used to communicate the usefulness of the nucleic acid, peptide, and/or composition of the invention in the kit for effecting alleviation of the various diseases or disorders recited herein. Optionally, or alternately, the instructional material may describe one or more methods of alleviation the diseases or disorders in a cell or a tissue of a mammal. The instructional material of the kit of the invention may, for example, be affixed to a container which contains the nucleic acid, peptide, and/or composition of the invention or be shipped together with a container which contains the nucleic acid, peptide, and/or composition. Alternatively, the instructional material may be shipped separately from the container with the intention that the instructional material and the compound be used cooperatively by the recipient.

An "isolated nucleic acid" refers to a nucleic acid segment or fragment which has been separated from sequences which flank it in a naturally occurring state, *e.g*., a DNA fragment which has been removed from the sequences which are normally adjacent to the fragment, *e.g*., the sequences adjacent to the fragment in a genome in which it naturally occurs. The term also applies to nucleic acids which have been substantially purified from other components which naturally accompany the nucleic acid, *e.g*., RNA or DNA or proteins, which naturally accompany it in the cell. The term therefore includes, for example, a recombinant DNA which is incorporated into a vector, into an autonomously replicating plasmid or virus, or into the genomic DNA of a prokaryote or eukaryote, or which exists as a separate molecule (*e.g*., as a cDNA or a genomic or cDNA fragment produced by PCR or restriction enzyme digestion) independent of other sequences. It also includes a recombinant DNA which is part of a hybrid gene encoding additional polypeptide sequence.

In the context of the present invention, the following abbreviations for the commonly occurring nucleic acid bases are used. "A" refers to adenosine, "C" refers to cytidine, "G" refers to guanosine, "T" refers to thymidine, and "U" refers to uridine.

By describing two polynucleotides as "operably linked" is meant that a single stranded or double stranded nucleic acid moiety comprises the two polynucleotides arranged within the nucleic acid moiety in such a manner that at least one of the two polynucleotides is able to exert a physiological effect by which it is characterized upon the other. By way of example, a promoter operably linked to the coding region of a gene is able to promote transcription of the coding region.

Preferably, when the nucleic acid encoding the desired protein further comprises a promoter/regulatory sequence, the promoter/regulatory is positioned at the 5' end of the desired protein coding sequence such that it drives expression of the desired protein in a cell. Together, the nucleic acid encoding the desired protein and its promoter/regulatory sequence comprise a "transgene."

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive" promoter is a nucleotide sequence which, when operably linked with a polynucleotide which encodes or specifies a gene product, causes the gene product to be produced in a living human cell under most or all physiological conditions of the cell.

An "inducible" promoter is a nucleotide sequence which, when operably linked with a polynucleotide which encodes or specifies a gene product, causes the gene product to be produced in a living human cell substantially only when an inducer which corresponds to the promoter is present in the cell.

A "tissue-specific" promoter is a nucleotide sequence which, when operably linked with a polynucleotide which encodes or specifies a gene product, causes the gene product to be produced in a living human cell substantially only if the cell is a cell of the tissue type corresponding to the promoter.

A "polyadenylation sequence is a polynucleotide sequence which directs the addition of a poly A tail onto a transcribed messenger RNA sequence.

A "polynucleotide" means a single strand or parallel and anti-parallel strands of a nucleic acid. Thus, a polynucleotide may be either a single-stranded or a double-stranded nucleic acid.

The term "nucleic acid" typically refers to large polynucleotides.

The term "oligonucleotide" typically refers to short polynucleotides, generally, no greater than about 50 nucleotides. It will be understood that when a nucleotide sequence is represented by a DNA sequence (i.e., A, T, G, C), this also includes an RNA sequence (*i.e*., A, U, G, C) in which "U" replaces "T."

Conventional notation is used herein to describe polynucleotide sequences: the left-hand end of a single-stranded polynucleotide sequence is the 5'-end; the left-hand direction of a double-stranded polynucleotide sequence is referred to as the 5'-direction.

The direction of 5' to 3' addition of nucleotides to nascent RNA transcripts is referred to as the transcription direction. The DNA strand having the same sequence as an mRNA is referred to as the "coding strand"; sequences on the DNA strand which are located 5' to a reference point on the DNA are referred to as "upstream sequences"; sequences on the DNA strand which are 3' to a reference point on the DNA are referred to as "downstream sequences."

A "portion" of a polynucleotide means at least at least about twenty sequential nucleotide residues of the polynucleotide. It is understood that a portion of a polynucleotide may include every nucleotide residue of the polynucleotide.

"Primer" refers to a polynucleotide that is capable of specifically hybridizing to a designated polynucleotide template and providing a point of initiation for synthesis of a complementary polynucleotide. Such synthesis occurs when the polynucleotide primer is placed under conditions in which synthesis is induced, i.e., in the presence of nucleotides, a complementary polynucleotide template, and an agent for polymerization such as DNA polymerase. A primer is typically single-stranded, but may be double-stranded. Primers are typically deoxyribonucleic acids, but a wide variety of synthetic and naturally occurring primers are useful for many applications. A primer is complementary to the template to which it is designed to hybridize to serve as a site for the initiation of synthesis, but need not reflect the exact sequence of the template. In such a case, specific hybridization of the primer to the template depends on the stringency of the hybridization conditions. Primers can be labeled with, e.g., chromogenic, radioactive, or fluorescent moieties and used as detectable moieties.

"Probe" refers to a polynucleotide that is capable of specifically hybridizing to a designated sequence of another polynucleotide. A probe specifically hybridizes to a target complementary polynucleotide, but need not reflect the exact complementary sequence of the template. In such a case, specific hybridization of the probe to the target depends on the stringency of the hybridization conditions. Probes can be labeled with, *e.g*., chromogenic, radioactive, or fluorescent moieties and used as detectable moieties.

"Recombinant polynucleotide" refers to a polynucleotide having sequences that are not naturally joined together. An amplified or assembled recombinant polynucleotide may be included in a suitable vector, and the vector can be used to transform a suitable host cell.

A recombinant polynucleotide may serve a non-coding function (*e.g*., promoter, origin of replication, ribosome-binding site, etc.) as well.

A "recombinant polypeptide" is one which is produced upon expression of a recombinant polynucleotide.

"Polypeptide" refers to a polymer composed of amino acid residues, related naturally occurring structural variants, and synthetic non-naturally occurring analogs thereof linked via peptide bonds, related naturally occurring structural variants, and synthetic non-naturally occurring analogs thereof. Synthetic polypeptides can be synthesized, for example, using an automated polypeptide synthesizer.

The term "protein" typically refers to large polypeptides.

The term "peptide" typically refers to short polypeptides.

Conventional notation is used herein to portray polypeptide sequences: the left-hand end of a polypeptide sequence is the amino-terminus; the right-hand end of a polypeptide sequence is the carboxyl-terminus.

As used herein, the term "reporter gene" means a gene, the expression of which can be detected using a known method. By way of example, the Escherichia coli *lacZ* gene may be used as a reporter gene in a medium because expression of the lacZ gene can be detected using known methods by adding the chromogenic substrate *o-*nitrophenyl-β-galactoside to the medium (Gerhardt et al., eds., 1994, Methods for General and Molecular Bacteriology, American Society for Microbiology, Washington, DC, p. 574).

"Resistin-inhibiting amount," as used herein, means any amount of a substance or molecule that detectably decreases the level of resistin expression, amount, and/or activity compared with the level of resisting expression, amount, and/or activity in the absence of the substance or molecule. Thus, any amount that mediates a detectable decrease in: the amount of resistin present, the level of resistin mRNA expression, and/or the ability of resistin to form necessary ligand/receptor interactions, is encompassed in the present invention. The assays by which these conditions are examined are well-known in the art and several are exemplified herein.

By the term "resistin-like activity," as used herein, refers to the ability of a molecule or compound to inhibit insulin stimulation of glucose uptake.

A "restriction site" is a portion of a double-stranded nucleic acid which is recognized by a restriction endonuclease.

A portion of a double-stranded nucleic acid is "recognized" by a restriction endonuclease if the endonuclease is capable of cleaving both strands of the nucleic acid at the portion when the nucleic acid and the endonuclease are contacted.

By the term "specifically binds," as used herein, is meant a compound, *e.g*., a protein, a nucleic acid, an antibody, and the like, which recognizes and binds a specific molecule, but does not substantially recognize or bind other molecules in a sample.

A first oligonucleotide anneals with a second oligonucleotide "with high stringency" if the two oligonucleotides anneal under conditions whereby only oligonucleotides which are at least about 75%, and preferably at least about 90% or at least about 95%, complementary anneal with one another. The stringency of conditions used to anneal two oligonucleotides is a function of, among other factors, temperature, ionic strength of the annealing medium, the incubation period, the length of the oligonucleotides, the G-C content of the oligonucleotides, and the expected degree of non-homology between the two oligonucleotides, if known. Methods of adjusting the stringency of annealing conditions are known (*see, e.g*., Sambrook et al., 1989, Molecular Cloning: A Laboratory Manual, Cold Spring Harbor Laboratory, New York).

"Syndrome X," as the term is used herein, refers to a condition, also known as polymetabolic syndrome, that includes hypertension, hyperlipidemia, and cardiovascular disease, alone or in combination, in association with hyperinsulinemia related to tissue insulin resistance as reviewed by Reaven (1993, Ann. Rev. Med. 4:121-131). This syndrome is often, but not always, a prediabetic condition.

As used herein, the term "transgene" means an exogenous nucleic acid sequence which exogenous nucleic acid is encoded by a transgenic cell or mammal.

A "recombinant cell" is a cell that comprises a transgene. Such a cell may be a eukaryotic cell or a prokaryotic cell. Also, the transgenic cell encompasses, but is not limited to, an embryonic stem cell comprising the transgene, a cell obtained from a chimeric mammal derived from a transgenic ES cell where the cell comprises the transgene, a cell obtained from a transgenic mammal, or fetal or placental tissue thereof, and a prokaryotic cell comprising the transgene.

By the term "exogenous nucleic acid" is meant that the nucleic acid has been introduced into a cell or an animal using technology which has been developed for the purpose of facilitating the introduction of a nucleic acid into a cell or an animal.

By "tag" polypeptide is meant any protein which, when linked by a peptide bond to a protein of interest, may be used to localize the protein, to purify it from a ceii extract, to immobilize it for use in binding assays, or to otherwise study its biological properties and/or function.

As used herein, the term "transgenic mammal" means a mammal, the germ cells of which comprise an exogenous nucleic acid.

As used herein, to "treat" means reducing the frequency with which symptoms of the type 2 diabetes are experienced by a patient.

By the term "vector" as used herein, is meant any plasmid or virus encoding an exogenous nucleic acid. The term should also be construed to include non-plasmid and non-viral compounds which facilitate transfer of nucleic acid into virions or cells, such as, for example, polylysine compounds and the like. The vector may be a viral vector which is suitable as a delivery vehicle for delivery of the TUG-1 protein or nucleic acid encoding a mammalian *resistin,* to the patient, or the vector may be a non-viral vector which is suitable for the same purpose. Examples of viral and non-viral vectors for delivery of DNA to cells and tissues are well known in the art and are described, for example, in Ma et al. (1997, Proc. Natl. Acad. Sci. U.S.A. 94:12744-12746). Examples of viral vectors include, but are not limited to, a recombinant vaccinia virus, a recombinant adenovirus, a recombinant retrovirus, a recombinant adeno-associated virus, a recombinant avian pox virus, and the like (Cranage et al., 1986, EMBO J. 5:3057-3063; International Patent Application No. WO94/17810, published August 18, 1994; International Patent Application No. WO94/23744, published October 27, 1994). Examples of non-viral vectors include, but are not limited to, liposomes, polyamine derivatives of DNA, and the like.

A "knock-out targeting vector," as the term is used herein, means a vector comprising two nucleic acid sequences each of which is complementary to a nucleic acid regions flanking a target sequence of interest which is to be deleted and/or replaced by another nucleic acid sequence. The two nucleic acid sequences therefore flank the target sequence which is to be removed by the process of homologous recombination.

### Description

### 1. Isolated nucleic acids

### A. Sense nucleic acids

The present invention illustrates an isolated nucleic acid encoding mammalian *resistin,* or a fragment thereof, wherein the nucleic acid shares at least about 30% identity with at least one nucleic acid having the sequence of (SEQ ID NO:1) and (SEQ ID NO:3). Alternatively the nucleic acid is about 35% homologous, about 40% homologous, about 45% homologous, about 50% homologous, about 55% homologous, about 60% homologous, about 65% homologous, about 70% homologous, about 75% homologous, about 80% homologous, about 85% homologous, about 90% homologous, about 95% homologous, or, about 99% homologous to at least one of SEO ID NO:1 and SEQ ID NO:3 disclosed herein. The nucleic acid may be at least one of SEQ ID NO:1 and SEQ ID NO:3.

illustrates The present invention illustrates an isolated nucleic acid encoding mouse *resistin* (m*resistin*), or a fragment thereof, wherein the nucleic acid shares at least about 30% homology with m*resistin* (SEQ ID NO:1). Alternatively, the nucleic acid is about 35% homologous, about 40% homologous, about 45% homologous, about 50% homologous, about 55% homologous, about 60% homologous, about 65% homologous, about 70% homologous. about 75% homologous. about 80% homologous, about 85% homologous, about 90% homologous, about 95% homologous, or about 99% homologous to the m*resistin* disclosed herein (SEQ ID NO:1). The nucleic acid may be SEQ ID NO:1.

The present invention illustrates an isolated nucleic acid encoding human *resistin* (h*resistin*), or a fragment thereof, wherein the nucleic acid shares at least about 30% homology with m*resistin* (SEQ ID NO:3). Alternatively, the nucleic acid is about 35% homologous, about 40% homologous, about 45% homologous, about 50% homologous, about 55% homologous, about 60% homologous, about 65% homologous, about 70% homologous, about 75% homologous, about 80% homologous, about 85% homologous, about 90% homologous, about 95% homologous, or, about 99% homologous to the h*resistin* disclosed herein (SEQ ID NO:3). The nucleic acid may be SEQ ID NO:3.

The present invention illustrates an isolated nucleic acid encoding mammalian *resistin,* or a fragment thereof, wherein the protein encoded by the nucleic acid shares at least about 30% homology with the amino acid sequence of at least one of SEQ ID NO:2 and SEQ ID NO:4. Alternatively, the nucleic acid is about 35% homologous, about 40% homologous, about 45% homologous, about 50% homologous, about 55% homologous, about 60% homologous, about 65% homologous, about 70% homologous, about 75% homologous, about 80% homologous, about 85% homologous, about 90% homologous, about 95% homologous, or, about 99% homologous to at least one of SEQ ID NO:2 and SEQ ID NO:4. The *resistin* protein encoded by the nucleic acid may be at least one of SEQ ID NO:2 and SEQ ID NO:4.

The present invention illustrates an isolated nucleic acid encoding mouse *resistin* (m*resistin*), or a fragment thereof, wherein the protein encoded by the nucleic acid shares at least about 30% homology with the amino acid sequence of SEQ ID NO:2. Alternatively, the protein encoded by the nucleic acid is about 35% homologous, about 40% homologous, about 45% homologous, about 50% homologous, about 55% homologous, about 60% homologous, about 65% homologous, about 70% homologous, about 75% homologous, about 80% homologous, about 85% homologous, about 90% homologous, about 95% homologous, or about 99% homologous to the *resistin* disclosed herein (SEO ID NO:2). The *resistin* protein encoded by the nucleic acid may be SEQ ID NO:2.

The present invention illustrates an isolated nucleic acid encoding human *resistin* (h*resistin*), or a fragment thereof, wherein the protein encoded by the nucleic acid shares at least about 30% homology with the amino acid sequence of SEQ ID NO:4. Alternatively, the nucleic acid is about 35% homologous, about 40% homologous, about 45% homologous, about 50% homologous, about 55% homologous, about 60% homologous, about 65% homologous, about 70% homologous, about 75% homologous, about 80% homologous, about 85% homologous, about 90% homologous, about 95% homologous, or about 99% homologous to the human resistin disclosed herein (SEO ID. NO:4). The resistin protein encoded by the nucleic acid may be SEQ ID NO:4.

The isolated nucleic acid may include an RNA or a DNA sequence encoding a resistin protein of the invention, and any modified forms thereof, including chemical modifications of the DNA or RNA which render the nucleotide sequence more stable when it is cell free or when it is associated with a cell. Chemical modifications of nucleotides may also be used to enhance the efficiency with which a nucleotide sequence is taken up by a cell or the efficiency with which it is expressed in a cell.

Once armed with the present invention, it is readily apparent to one skilled in the art that other nucleic acids encoding TZD-suppressible proteins can such as those present in other species of mammals (*e.g*., ape, gibbon, rat, bovine, ovine, equine, porcine, canine, feline, and the like) be obtained by following the procedures described herein in the experimental details section for the isolation of the mouse and human *resistin* nucleic acids encoding resistin polypeptides as disclosed herein (*e.g*., screening of genomic or cDNA libraries), and procedures that are well-known in the art (*e.g*., reverse transcription PCR using mRNA samples) or to be developed.

Further, any number of procedures may be used for the generation of mutant, derivative or variant forms of resistin using recombinant DNA methodology well known in the art such as, for example, that described in Sambrook et al. (1989, Molecular Cloning: A Laboratory Manual, Cold Spring Harbor Laboratory Press, New York) and Ausubel et al. (1997, Current Protocols in Molecular Biology, Green & Wiley, New York).

Procedures for the introduction of amino acid changes in a protein or polypeptide by altering the DNA sequence encoding the polypeptide are well known in the art and are also described in Sambrook et al. (1989, *supra*); Ausubel et al. (1997, *supra*).

The invention illustrates a nucleic acid encoding a mammalian *resistin* wherein the nucleic acid encoding a tag polypeptide is covalently linked thereto. That is, the invention encompasses a chimeric nucleic acid wherein the nucleic acid sequences encoding a tag polypeptide is covalently linked to the nucleic acid encoding at least one of mouse *resistin* and human *resistin.* Such tag polypeptides are well known in the art and include, for instance, green fluorescent protein, myc, myc-pyruvate kinase (myc-PK), His₆, maltose biding protein (MBP), an influenza virus hemagglutinin tag polypeptide, a flag tag polypeptide, and glutathione-S-transferase (GST) tag polypeptide. Any nucleic acid sequence encoding a polypeptide which may function in a manner substantially similar to these tag polypeptides is illustrated.

The nucleic acid comprising a nucleic acid encoding a tag polypeptide can be used to localize resistin within a cell, detect resistin secreted from a cell, and to study the role(s) of resistin in a cell before, during, and/or after exposing the cell to TZD or another test compound. Further, addition of a tag polypeptide facilitates isolation and purification of the "tagged" protein such that the protein of the invention can be produced and purified readily.

### B. Antisense nucleic acids

In certain situations, it may be desirable to inhibit expression of resistin and the invention therefore includes compositions useful for inhibition of resistin expression. Thus, the invention illustrates an isolated nucleic acid complementary to a portion or all of a nucleic acid encoding a mammalian resistin which is in an antisense orientation with respect to transcription. The antisense nucleic acid may be complementary with a nucleic acid having at least about 30% homology with at least one of SEQ ID NO:1 and SEQ ID NO:3, or a fragment thereof. Alternatively, the nucleic acid is about 35% homologous, about 40% homologous, about 45% homologous, about 50% homologous, about 55% homologous, about 60% homologous, about 65% homologous, about 70% homologous, about 75% homologous, about 80% homologous, about 85% homologous, about 90% or homologous, about 95% homologous, about 99% homologous to a nucleic acid complementary to a portion or all of a nucleic acid encoding a mammalian resistin having the sequence of at least one of SEQ ID NO:1 and SEQ ID NO:3, which is in an antisense orientation with respect to transcription. The nucleic acid may be complementary to a portion or all of a nucleic acid that is at least one of SEQ ID NO:1 and SEQ ID NO:3. Such antisense nucleic acid serves to inhibit the expression, function, or both, of resistin.

Alternatively, antisense molecules may be made synthetically and then provided to the cell. Antisense oligomers of between about 10 to about 30, and more preferably about 15 nucleotides, are preferred, since they are easily synthesized and introduced into a target cell. Synthetic antisense molecules include oligonucleotide derivatives known in the art which have improved biological activity compared to unmodified oligonucleotides (*see* Cohen, *supra*; Tullis, 1991, U.S. Patent No. 5,023,243, incorporated by reference herein in its entirety).

### II. Isolated polypeptides

The invention also illustrates an isolated polypeptide comprising a mammalian resistin. The isolated polypeptide comprising a mammalian resistin is at least about 30% homologous to a polypeptide having the amino acid sequence of at least one of (SEQ ID NO:2) and (SEQ ID NO:4). Alternatively, the isolated polypeptide is about 35% homologous, about 40% homologous, about 45% homologous, about 50% homologous, about 55% homologous, about 60% homologous, about 65% homologous, about 70% homologous, about 75% homologous, about 80% homologous, about 85% homologous, about 90% homologous, about 95% homologous, or about 99% homologous to at least one of mouse resistin and human resistin. The isolated polypeptide comprising a mammalian resistin may be at least one of mouse resistin and human resistin. The isolated polypeptide comprising a mammalian resistin may be at least one of SEQ ID NO: 2, and SEQ ID NO:4.

The invention also includes an isolated polypeptide comprising a mammalian resistin. The isolated polypeptide comprising a mammalian resistin may be at least about 30% homologous to a polypeptide having the amino acid sequence of SEQ ID NO:2. Alternatively, the isolated polypeptide comprising a mammalian resistin is at least about 35%, about 40% homologous,

, about 50% homologous, about 60% homologous, about 65% homologous, about 70% homologous, about 75% homologous. about 80% homologous, about 85% homologous, about 90% homologous, about 95% homologous, or, at least about 99% homologous to mouse resistin. The isolated polypeptide comprising a mammalian resistin may be mouse resistin. The isolated polypeptide comprising a mammalian resistin may be SEQ ID NO: 2.

The invention also illustrates an isolated polypeptide comprising a mammalian resistin. The isolated polypeptide comprising a mammalian resistin is at least about 30% homologous to a polypeptide having the amino acid sequence of SEQ ID NO:4. Alternatively, the isolated polypeptide comprising a mammalian resistin is at least about 35%, preferable, about 40% homologous, about 50% homologous, about 60% homologous, about 65% homologous, about 70% homologous, about 75% homologous, about 80% homologous, about 85% homologous, about 90% homologous, about 95% homologous, or at least about 99% homologous to human resistin. The isolated polypeptide comprising a mammalian resistin may be human resistin. The isolated polypeptide comprising a mammalian resistin may be SEQ ID NO:4.

The present invention also illustrates for analogs of proteins or peptides which comprise a resistin protein as disclosed herein. Analogs may differ from naturally occurring proteins or peptides by conservative amino acid sequence differences or by modifications which do not affect sequence, or by both. For example. conservative amino acid changes may be made, which although they alter the primary sequence of the protein or peptide, do not normally alter its function. Conservative amino acid substitutions typically include substitutions within the following groups:
glycine, alanine;
valine, isoleucine, leucine;
aspartic acid, glutamic acid;
asparagine, glutamine;
serine, threonine;
lysine, arginine;
phenylalanine, tyrosine.

Modifications (which do not normally alter primary sequence) include *in vivo,* or *in vitro,* chemical derivatization of polypeptides, *e.g*., acetylation, or carboxylation. Also included are modifications of glycosylation, *e.g*., those made by modifying the glycosylation patterns of a polypeptide during its synthesis and processing or in further processing steps; *e.g.,* by exposing the polypeptide to enzymes which affect glycosylation, *e.g*., mammalian glycosylating or deglycosylating enzymes. Also embraced are sequences which have phosphorylated amino acid residues, *e.g*., phosphotyrosine, phosphoserine, or phosphothreonine.

Also illustrated are polypeptides which have been modified using ordinary molecular biological techniques so as to improve their resistance to proteolytic degradation or to optimize solubility properties or to render them more suitable as a therapeutic agent. Analogs of such polypeptides include those containing residues other than naturally occurring L-amino acids, *e.g*., D-amino acids or non-naturally occurring synthetic amino acids. The peptides of the invention are not limited to products of any of the specific exemplary processes listed herein.

The present invention should also be construed to encompass "mutants," "derivatives." and "variants" of the peptides of the invention (or of the DNA encoding the same) which mutants, derivatives and variants are resistin peptides which are altered in one or more amino acids (or, when referring to the nucleotide sequence encoding the same, are altered in one or more base pairs) such that the resulting peptide (or DNA) is not identical to the sequences recited herein, but has the same biological property as the peptides disclosed herein, in that the peptide has biological/biochemical properties of the resistin peptide of the present invention.

A biological property of a resistin protein should be construed but not be limited to include, the ability of the peptide to be suppressed by TZDs (*e.g*., the level of the protein, its secretion from a cell, or both, are decreased by TZD), the ability of resistin to be secreted from a cell, and the ability of resistin to antagonize insulin action on glucose uptake.

Further, the invention illustrates naturally occurring variants or recombinantly derived mutants of resistin sequences, which variants or mutants render the protein encoded thereby either more, less, or just as biologically active as the full-length clones of the invention.

The nucleic acids, and peptides encoded thereby, are useful tools for elucidating the function(s) of resistin in a cell. Further, nucleic and amino acids comprising mammalian resistin are useful diagnostics which can be used, for example, to identify a compound that affects resistin expression and is a potential antidiabetic drug candidate. The nucleic acids, the proteins encoded thereby, or both, can be administered to a mammal to increase or decrease expression of resistin in the mammal. This can be beneficial for the mammal in situations where under or over-expression of resistin in the mammal mediates a disease or condition associated with altered expression of resistin compared with normal expression of resistin in a healthy mammal. Additionally, the nucleic and amino acids can be used to produce recombinant cells and transgenic non-human mammals which are useful tools for the study of TZD action, the identification of novel antidiabetic therapeutics, and for elucidating the cellular role(s) of resistin, among other things. Further, the nucleic and amino acids can be used diagnostically, either by assessing the level of gene expression or protein expression, to assess severity and prognosis of type 2 diabetes and Syndrome X, reflecting endogenous and exogenous substances leading to activation of endogenous PPARγ. The nucleic acids and proteins are also useful in the development of assays to assess the efficacy of a treatment for type 2 diabetes and/or Syndrome X. That is, the nucleic acids and polypeptides can be used to detect the effect of various therapies on *resistin* expression, thereby ascertaining the effectiveness of the therapies.

### III. Vectors

The invention illustrates an isolated nucleic acid encoding a mammalian resistin operably linked to a nucleic acid comprising a promoter/regulatory sequence such that the nucleic acid is preferably capable of directing expression of the protein encoded by the nucleic acid. Thus, the invention illustrates expression vectors and methods for the introduction of exogenous DNA into cells with concomitant expression of the exogenous DNA in the cells such as those described, for example, in Sambrook et al. (1989, *supra*), and Ausubel et al. (1997, *supra*).

Expression of resistin either alone or fused to a detectable tag polypeptide in cells which either do not normally express resistin or which do not express resistin fused with a tag polypeptide, may be accomplished by generating a plasmid, viral, or other type of vector comprising the desired nucleic acid operably linked to a promoter/regulatory sequence which serves to drive expression of the protein, with or without tag, in cells in which the vector is introduced. Many promoter/regulatory sequences useful for driving constitutive expression of a gene are available in the art and include, but are not limited to, for example, the cytomegalovirus immediate early promoter enhancer sequence, the SV40 early promoter, both of which were used in the experiments disclosed herein, as well as the Rous sarcoma virus promoter, and the like. Moreover, inducible and tissue specific expression of the nucleic acid encoding resistin may be accomplished by placing the nucleic acid encoding resistin, with or without a tag, under the control of an inducible or tissue specific promoter/regulatory sequence. Examples of tissue specific or inducible promoter/regulatory sequences which are useful for his purpose include, but are not limited to the MMTV LTR inducible promoter, and the SV40 late enhancer/promoter. In addition, promoters which are well known in the art which are induced in response to inducing agents such as metals, glucocorticoids, and the like, are also contemplated in the invention. Thus, it will be appreciated that the invention includes the use of any promoter/regulatory sequence, which is either known or unknown, and which is capable of driving expression of the desired protein operably linked thereto.

Expressing resistin using a vector allows the isolation of large amounts of recombinantly produced protein. Further, where the lack or decreased level of resistin expression causes a disease, disorder, or condition associated with such expression, the expression of resistin driven by a promoter/regulatory sequence can provide useful therapeutics including, but not limited to, gene therapy whereby resistin is provided. A disease, disorder or condition associated with a decreased level of expression, level of protein, or decreased activity of the protein, for which administration of resistin can be useful can include, but is not limited to, a condition associated with a low blood sugar state characterized by high insulin levels. These diseases, disorders or conditions can include, *inter alia,* insulinoma (due to, *e.g*., pancreatic insulin-secreting tumors), nesidioblastosis (congenital hyperinsulinemia), and other congenital syndromes of hyperinsulinism. Therefore, the invention illustrates not only methods of inhibiting resistin expression, translation, and/or activity, but it also includes methods relating to increasing resistin expression, protein level, and/or activity.

Selection of any particular plasmid vector or other DNA vector is not a limiting factor in this invention and a wide plethora vectors is well-known in the art. Further, it is well within the skill of the artisan to choose particular promoter/regulatory sequences and operably link those promoter/regulatory sequences to a DNA sequence encoding a desired polypeptide. Such technology is well known in the art and is described, for example, in Sambrook, *supra,* and Ausubel, *supra.*

The invention thus illustrates a vector vector comprising an isolated nucleic acid encoding a mammalian resistin. The incorporation of a desired nucleic acid into a vector and the choice of vectors is well-known in the art as described in, for example, Sambrouk et al., *supra,* and Ausubel et al., *supra.*

The invention also illustrates cells, viruses, proviruses, and the like, containing such vectors. Methods for producing cells comprising vectors and/or exogenous nucleic acids are well-known in the art. *See, e.g.,* Sambrook et al., *supra*; Ausubel et al., *supra.*

The nucleic acids encoding resistin may be cloned into various plasmid vectors. Instead, the present invention illustrates a wide plethora of vectors which are readily available and/or well-known in the art.

### IV. Antisense molecules and ribozymes

Further, the invention illustrates a recombinant cell comprising an antisense nucleic acid which cell is a useful model for the study of diabetes and/or hyperglycemia and for elucidating the role(s) of resistin in such processes. That is, the suppression of resistin by TZD indicates that resistin is involved in mediating the antidiabetic effect(s) of TZDs. Accordingly, a transgenic cell comprising an antisense nucleic acid complementary to resistin is a useful tool for the study of the mechanism(s) of action of resistin and its role(s) in the cell and for the identification of therapeutics that ameliorate the effect(s) of resistin expression.

One skilled in the art will appreciate that one way to decrease the levels of resistin mRNA and/or protein in a cell is to inhibit expression of the nucleic acid encoding the protein. Expression of resistin may be inhibited using, for example, antisense molecules, and also by using Ribozymes or double-stranded RNA as described in, for example, Wianny and Kernicka-Goetz (2000, Nature Cell Biol. 2:70-75).

Antisense molecules and their use for inhibiting gene expression are well known in the art (*see, e.g*., Cohen, 1989, In: Oligodeoxyribonucleotides, Antisense Inhibitors of Gene Expression, CRC Press). Antisense nucleic acids are DNA or RNA molecules that are complementary, as that term is defined elsewhere herein, to at least a portion of a specific mRNA molecule (Weintraub, 1990, Scientific American 262:40). In the cell, antisense nucleic acids hybridize to the corresponding mRNA. forming a double-stranded molecule thereby inhibiting the translation of genes.

The use of antisense methods to inhibit the translation of genes is known in the art, and is described, for example, in Marcus-Sakura (1988, Anal. Biochem. 172:289). Such antisense molecules may be provided to the cell via genetic expression using DNA encoding the antisense molecule as taught by Inoue (1993, U.S. Patent No. 5,190,931).

Alternatively, antisense molecules may be made synthetically and then provided to the cell. Antisense oligomers of between about 10 to about 30, and more preferably about 15 nucleotides, are preferred, since they are easily synthesized and introduced into a target cell. Synthetic antisense molecules contemplated by the invention include oligonucleotide derivatives known in the art which have improved biological activity compared to unmodified oligonucleotides (*see* Cohen, *supra*; Tullis, 1991, U.S. Patent No. 5,023,243, incorporated by reference therein in its entirety).

Ribozymes and their use for inhibiting gene expression are also well known in the art (*see*, *e.g*., Cech et al., 1992, J. Biol. Chem. 267:17479-17482; Hampel et al., 1989, Biochemistry 28:4929-4933; Eckstein et al., International Publication No. WO 92/07065; Altman et al., U.S. Patent No. 5,168,053, incorporated by reference herein in its entirety). Ribozymes are RNA molecules possessing the ability to specifically cleave other single-stranded RNA in a manner analogous to DNA restriction endonucleases. Through the modification of nucleotide sequences encoding these RNAs, molecules can be engineered to recognize specific nucleotide sequences in an RNA molecule and cleave it (Cech, 1988, J. Amer. Med. Assn. 260:3030). A major advantage of this approach is that, because they are sequence-specific, only mRNAs with particular sequences are inactivated.

There are two basic types of ribozymes, namely, tetrahymena-type (Hasselhoff, 1988, Nature 334:585) and hammerhead-type. Tetrahymena-type ribozymes recognize sequences which are four bases in length, while hammerhead-type ribozymes recognize base sequences 11-18 bases in length. The longer the sequence, the greater the likelihood that the sequence will occur exclusively in the target mRNA species. Consequently, hammerhead-type ribozymes are preferable to tetrahymena-type ribozymes for inactivating specific mRNA species, and 18-base recognition sequences are preferable to shorter recognition sequences which may occur randomly within various unrelated mRNA molecules.

Ribozymes useful for inhibiting the expression of resistin may be designed by incorporating target sequences into the basic ribozyme structure which are complementary to the mRNA sequence of the resistin encoded by *resistin* or having at least about 80% homology to at least one of SEQ ID NO:1 and SEQ ID NO:3. Ribozymes targeting resistin may be synthesized using commercially available reagents (Applied Biosystems, Inc., Foster City, CA) or they may be genetically expressed from DNA encoding them.

### V. Recombinant cells and transgenic non-human mammals

The invention illustrates a recombinant cell comprising , *inter alia,* an isolated nucleic acid encoding resistin, an antisense nucleic acid complementary thereto, a nucleic acid encoding an antibody that specifically binds resistin, and the like. In one aspect, the recombinant cell can be transiently transfected with a plasmid encoding a portion of the nucleic acid encoding resistin. The nucleic acid need not be integrated into the cell genome nor does it need to be expressed in the cell. Moreover, the cell may be a prokaryotic or a eukaryotic cell and the invention should not be construed to be limited to any particular cell line or cell type. Such cells include, but are not limited to, fibroblasts, hepatocytes, skeletal muscle cells, and adipocytes.

The recombinant cell comprising an isolated nucleic acid encoding mammalian resistin may be used to produce a transgenic non-human mammal. That is, the exogenous nucleic acid, or transgene as it is also referred to herein, of the invention is introduced into a cell, and the cell is then used to generate the non-human transgenic mammal. The cell into which the transgene is introduced is preferably an embryonic stem (ES) cell. However, the invention should not be construed to be limited solely to ES cells comprising the transgene of the invention nor to cells used to produce transgenic animals. Rather, a transgenic cell may include, any cell derived from a transgenic animal comprising a transgene, a cell comprising the transgene derived from a chimeric animal derived from the transgenic ES cell, and any other comprising the transgene which may or may not be used to generate a non-human transgenic mammal.

Further, it is important to note that the purpose of transgene-comprising, *i.e*., recombinant, cells is not limited to the generation of transgenic mammals. Rather, any cell is included type into which a nucleic acid encoding a mammalian resistin is introduced, including, without limitation, a prokaryotic cell and a eukaryotic cell comprising an isolated nucleic acid encoding mammalian resistin.

When the cell is a eukaryotic cell, the cell may be any eukaryotic cell which, when the transgene is introduced therein, and the protein encoded by the desired gene is no longer expressed therefrom, a benefit is obtained. Such a benefit may include the fact that there has been provided a system in which lack of expression of the desired gene can be studied *in vitro* in the laboratory or in a mammal in which the cell resides, a system wherein cells comprising the introduced gene deletion can be used as research, diagnostic and therapeutic tools, and a system wherein animal models are generated which are useful for the development of new diagnostic and therapeutic tools for selected disease states in a mammal including, for example, type 2 diabetes, Syndrome X, and prediabetic conditions. Insulin resistance is also a characteristic of polycystic ovarian syndrome, so that resistin may be useful in the diagnosis and therapy of this condition as well.

Alternatively, the invention illustrates a eukaryotic cell which, when the transgene of the invention is introduced therein, and the protein encoded by the desired gene is expressed therefrom where it was not previously present or expressed in the cell or where it is now expressed at a level or under circumstances different than that before the transgene was introduced, a benefit is obtained. Such a benefit may include the fact that there has been provided a system in the expression of the desired gene can be studied *in vitro* in the laboratory or in a mammal in which the cell resides, a system wherein cells comprising the introduced gene can be used as research, diagnostic and therapeutic tools, and a system wherein animal models are generated which are useful for the development of new diagnostic and therapeutic tools for selected disease states in a mammal.

Such cell expressing an isolated nucleic acid encoding *resistin* can be used to provide resistin to a cell, tissue, or whole animal where a higher level of resistin can be useful to treat or alleviate a disease, disorder or condition associated with low blood sugar states characterized by high insulin levels. Such diseases, disorders or conditions can include, but are not limited to, insulinoma, (due to, *e.g*., pancreatic insulin-secreting tumors), nesidioblastosis (congenital hyperinsulinemia). and other congenital syndromes of hyperinsulinism. Therefore, the invention illustrates a cell expressing resistin to increase or induce resistin expression, translation, and/or activity, where increasing resistin expression, protein level, and/or activity can be useful to treat or alleviate a disease, disorder or condition.

One of ordinary skill would appreciate, based upon the disclosure provided herein, that a "knock-in" or "knock-out" vector comprises at least two sequences homologous to two portions of the nucleic acid which is to be replaced or deleted, respectively. The two sequences are homologous with sequences that flank the gene; that is, one sequence is homologous with a region at or near the 5' portion of the coding sequence of the nucleic acid encoding *resistin* and the other sequence is further downstream from the first. One skilled in the art would appreciate, based upon the disclosure provided herein, that the present invention is not limited to any specific flanking nucleic acid sequences. Instead, the targeting vector may comprise two sequences which remove some or all (*i.e.,* a "knock-out" vector) or which insert (*i.e*., a "knock-in" vector) a nucleic acid encoding resistin, or a fragment thereof, from or into a mammalian genome, respectively. The crucial feature of the targeting vector is that it comprise sufficient portions of two sequences located towards opposite, *i.e*.. 5' and 3', ends of the *resistin* open reading frame (ORF) in the case of a "knock-out" vector, to allow deletion/insertion by homologous recombination to occur such that all or a portion of the nucleic acid encoding resistin is deleted from or inserted into a location on a mammalian chromosome.

The design of transgenes and knock-in and knock-out targeting vectors is well-known in the art and is described in standard treatises such as Sambrook et al. (1989, Molecular Cloning: A Laboratory Manual, Cold Spring Harbor Laboratory, New York), and in Ausubel et al. (1997, Current Protocols in Molecular Biology, John Wiley & Sons, New York), and the like. The upstream and downstream portions flanking or within the *resistin* coding region to be used in the targeting vector may be easily selected based upon known methods and following the teachings disclosed herein based on the disclosure provided herein including the nucleic and amino acid sequences of both mouse and human *resistin.* Armed with these sequences, one of ordinary skill in the art would be able to construct the transgenes and knock-out vectors.

The invention further illustrates a knock-out targeting vector comprising a nucleic acid encoding a selectable marker such as, for example, a nucleic acid encoding the *neo^{R}* gene thereby allowing the selection of transgenic a cell where the nucleic acid encoding *resistin,* or a portion thereof, has been deleted and replaced with the neomycin resistance gene by the cell's ability to grow in the presence of G418. However. other selectable markers well-known in the art may be used in the knock-out targeting vector to allow selection of recombinant cells where the *resistin* gene has been deleted and/or inactivated and replaced by the nucleic acid encoding the selectable marker of choice. Methods of selecting and incorporating a selectable marker into a vector are well-known in the art and are describe in, for example, Sambrook et al. (1989, Molecular Cloning: A Laboratory Manual, Cold Spring Harbor Laboratory, New York), and in Ausubel et al. (1997, Current Protocols in Molecular Biology, John Wiley & Sons, New York).

As noted herein, the invention illustrates a non-human transgenic mammal comprising an exogenous nucleic acid inserted into a desired site in the genome thereof thereby deleting the coding region of a desired endogenous target gene, *i.e*., a knock-out transgenic mammal. Further, the invention illustrates a transgenic non-human mammal wherein an exogenous nucleic acid encoding resistin is inserted into a site the genome, *i.e*., a "knock-in" transgenic mammal. The knock-in transgene inserted may comprise various nucleic acids encoding, for example, a tag polypeptide, a promoter/regulatory region operably linked to the nucleic acid encoding *resistin* not normally present in the cell or not typically operably linked to *resistin.*

The generation of the non-human transgenic mammal of the invention is preferably accomplished using the method which is now described. However, other methods can be used to generate the desired knock-out mammal.

In the preferred method of generating a non-human transgenic mammal, ES cells are generated comprising the transgene of the invention and the cells are then used to generate the knock-out animal essentially as described in Nagy and Rossant (1993, In: Gene Targeting, A Practical Approach, pp.146-179, Joyner ed., IRL Press). ES cells behave as normal embryonic cells if they are returned to the embryonic environment by injection into a host blastocyst or aggregate with blastomere stage embryos. When so returned, the cells have the full potential to develop along all lineages of the embryo. Thus, it is possible, to obtain ES cells, introduce a desired DNA therein, and then return the cell to the embryonic environment for development into mature mammalian cells, wherein the desired DNA may be expressed.

Precise protocols for the generation of transgenic mice are disclosed in Nagy and Rossant (1993, In: Gene Targeting, A Practical Approach, Joyner ed. IRL Press, pp. 146-179). and are therefore not repeated herein. Transfection or transduction of ES cells in order to introduce the desired DNA therein is accomplished using standard protocols, such as those described, for example, in Sambrook et al. (1989, Molecular Cloning: A Laboratory Manual, Cold Spring Harbor Laboratory, New York), and in Ausubel et al. (1997, Current Protocols in Molecular Biology, John Wiley & Sons, New York). Preferably, the desired DNA contained within the transgene of the invention is electroporated into ES cells, and the cells are propagated as described in Soriano et al. (1991, Cell 64:693-702).

Introduction of an isolated nucleic acid into the fertilized egg of the mammal is accomplished by any number of standard techniques in transgenic technology (Hogan et al., 1986, Manipulating the Mouse Embryo: A Laboratory Manual, Cold Spring Harbor, NY). Most commonly, the nucleic acid is introduced into the embryo by way of microinjection.

Once the nucleic acid is introduced into the egg, the egg is incubated for a short period of time and is then transferred into a pseudopregnant mammal of the same species from which the egg was obtained as described, for example, in Hogan et al. (1986, Manipulating the Mouse Embryo: A Laboratory Manual, Cold Sparing Harbor, NY). Typically, many eggs are injected per experiment, and approximately two-thirds of the eggs survive the procedure. About twenty viable eggs are then transferred into pseudopregnant animals, and usually four to ten of the viable eggs so transferred will develop into live pups.

Any mammalian *resistin* gene may be used in the methods described herein to produce a transgenic mammal or a transgenic cell harboring a transgene comprising a deletion of all or part of that mammalian *resistin* gene. Preferably, a murine *resistin* gene (SEQ ID NO: 1) or a mammalian homolog, such as a human *resistin* (SEQ ID NO:3) gene, is used.

The transgenic mammal can be any species of mammal. Thus, the invention should be construed to include generation of transgenic mammals encoding the chimeric nucleic acid, which mammals include mice, hamsters, rats, rabbits, pigs, sheep and cattle. The methods described herein for generation of transgenic mice can be analogously applied using any mammalian species. Preferably, the transgenic mammal of the invention is a rodent and even more preferably, the transgenic mammal of the invention is a mouse. By way of example, Lukkarinen et al. (1997, Stroke 28:639-645), teaches that gene constructs which enable the generation of transgenic mice also enable the generation of other transgenic rodents, including rats. Similarly, nullizygous mutations in a genetic locus of an animal of one species can be replicated in an animal of another species having a genetic locus highly homologous to the first species.

To identify the transgenic mammals of the invention, pups are examined for the presence of the isolated nucleic acid using standard technology such as Southern blot hybridization, PCR, and/or RT-PCR. Expression of the nucleic acid in the cells and in the tissues of the mammal is also assessed using ordinary technology described herein. Further, the presence or absence of resistin in the circulating blood of the transgenic animal can be determined , for example, as disclosed herein (*e.g*., Western blot analysis), or using standard methods for protein detection that are well-known in the art.

Cells obtained from the transgenic mammal of the invention, which are also considered "transgenic cells" as the term is used herein, encompass such as cells as those obtained from the *resistin* (+/-) and (-/-) transgenic non-human mammal described elsewhere herein, are useful systems for modeling diseases and symptoms of mammals which are believed to be associated with altered levels of glucose such as diabetes and hyperglycemia and/or diseases which are ameliorated or treated using TZDs or are associated with an altered level of resistin expression. Moreover, as a marker of a pathway(s) that improves insulin action, resistin expression levels are also useful indicators in assessment of hypertension and obesity in addition to diabetes.

Particularly suitable are cells derived from a tissue of the non-human knock-out or knock-in transgenic mammal described herein, wherein the transgene comprising the *resistin* gene is expressed or inhibits expression of resistin in various tissues. By way of example, cell types from which such cells are derived include fibroblasts, endothelial, adipocyte, and myoblast cells of (1) the *resistin* (+/+), (+/-) and (-/-) non-human transgenic liveborn mammal, (2) the resistin (+/+), (-/-) or (+/-) fetal animal, and (3) placental cell lines obtained from the *resistin* (+/+), (-/-) and (+/-) fetus and liveborn mammal.

One skilled in the art would appreciate, based upon this disclosure, that cells comprising decreased levels of resistin protein, decreased level of resistin activity, or both, include, but are not limited to, cells expressing inhibitors of resistin expression (*e.g*., antisense or ribozyme molecules, as well as activated PPARγ, and the like), other transcription factors that inhibit the resistin promoter (*e.g*., dominant negative C/EBPα), or reagents specifically targeted to the resistin promoter sequence.

Methods and compositions useful for maintaining mammalian cells in culture are well known in the art, wherein the mammalian cells are obtained from a mammal including, but not limited to, cells obtained from a mouse such as the transgenic mouse described herein.

The recombinant cell can be used to study the effect of qualitative and quantitative alterations in resistin levels on adipocyte/muscle cell signal transduction systems and glucose metabolism. Further, the recombinant cell can be used to produce resistin for use for therapeutic and/or diagnostic purposes. That is, a recombinant cell expressing resistin can be used to produce large amounts of purified and isolated resistin that can be administered to treat or alleviate a disease, disorder or condition associated with or caused by a decreased level of resistin. Further, administration of resistin can be useful to treat or alleviate a low blood sugar state characterized by high insulin levels. Such low blood sugar states include, but are not limited to, insulinoma, nesidioblastosis, and other congenital hyperinsulinism syndromes. Alternatively, the recombinant cells expressing resistin can be administered in ex vivo and *in vivo* therapies where administering the recombinant cells thereby administers the protein to a cell, a tissue, and/or an animal. Additionally, the recombinant cells are useful for the discovery of resistin receptor and resistin signaling pathways.

The recombinant cell may be used to study the effects of elevated or decreased resistin levels on cell homeostasis and glucose metabolism since resistin has been hypothesized to play a role in glucose metabolism, Syndrome X, and type 2 diabetes and hyperglycemia.

The recombinant cell, wherein the cell has been engineered such that it does not express resistin, or expresses reduced or altered resistin lacking biological activity, can also be used in ex vivo and *in vivo* cell therapies where either an animal's own cells (*e.g*., adipocytes, hepatocytes, muscle cells, and the like) or those of a syngeneic matched donor are recombinantly engineered as described elsewhere herein (*e.g*., by insertion of an antisense nucleic acid or a knock-out vector such that resistin expression and/or protein levels are thereby reduced in the recombinant cell), and the recombinant cell is administered to the recipient animal. In this way, recombinant cells that express resistin at a reduced level can be administered to an animal whose own cells express increased levels of resistin thereby treating or alleviating a disease, disorder or condition associated with or mediated by increased resistin expression as disclosed elsewhere herein.

The transgenic mammal, rendered diabetic either genetically by cross-breeding or chemically via streptozotocin, can be used to study the pathogenesis of diabetic complication and the possible role of resistin therein.

Further, the transgenic mammal and/or cell may be used to study the subcellular localization of resistin.

Also, the transgenic mammal (both +/- and -/- live bom and fetuses) and/or cell of the invention may be used to study to role(s) of resistin in glucose metabolism and to elucidate the target(s) of resistin action as well as any receptor(s) that bind with resistin to mediate its effect(s) in the cell.

### VI. Antibodies

The invention includes an antibody that specifically binds resistin, or an immunogenic fragment thereof, as defined in embodiment 6.

In one embodiment, the antibody is a rabbit polyclonal antibody to mouse resistin. More specifically, the antibody is directed to mouse resistin comprising amino acid residues sequence of (SEQ ID NO:2). In another embodiment, the antibody is a rabbit polyclonal antibody directed to human resistin comprising amino acid residues sequence of (SEQ ID NO:4).

The antibodies are generated by immunizing rabbits according to standard immunological techniques well-known in the art (*see*, *e.g.,* Harlow et al., 1988, In: Antibodies, A Laboratory Manual, Cold Spring Harbor, Any). Such techniques include immunizing an animal with a chimeric protein comprising a portion of another protein such as a maltose binding protein or glutathione (GSH) tag polypeptide portion and a portion comprising the respective mouse and/or human resistin amino acid residues. The chimeric proteins are produced by cloning the appropriate nucleic acids encoding resistin (*e.g*., [SEQ ID NO:1] and [SEQ ID NO:3]) into a plasmid vector suitable for this purpose, such as but not limited to, pMAL-2 or pCMX.

However, the invention should not be construed as being limited solely to these antibodies or to these portions of the protein antigens. Rather, the invention should be construed to include other antibodies, as that term is defined elsewhere herein, to mouse and human resistin, or portions thereof_{.} Further, the present invention should be construed to encompass antibodies perform in a manner substantially similar to those described herein in that, *inter alia,* the antibodies bind to resistin and they are able to bind resistin present on Western blots and in immunofluorescence microscopy of a cell transiently transfected with a nucleic acid encoding at least a portion of resistin.

One skilled in the art would appreciate, based upon the disclosure provided herein, that the antibody can specific bind with any portion of the protein and the full-length protein can be used to generate antibodies specific therefor. However, the present invention is not limited to using the full-length protein as an immunogen. Rather, the present invention includes using an immunogenic portion of the protein to produce an antibody that specifically binds with mammalian resistin. That is, the invention includes immunizing an animal using an immunogenic portion, or antigenic determinant, of the protein.

The antibodies can be produced by immunizing an animal such as, but not limited to, a rabbit or a mouse, with a protein of the invention, or a portion thereof, or by immunizing an animal using a protein comprising at least a portion of resistin, or a fusion protein including a tag polypeptide portion comprising, for example, a maltose binding protein tag polypeptide portion, covalently linked with a portion comprising the appropriate resistin amino acid residues. One skilled in the art would appreciate, based upon the disclosure provided herein, that smaller fragments of these proteins can also be used to produce antibodies that specifically bind resistin.

One skilled in the art would appreciate, based upon the disclosure provided herein, that various portions of an isolated resistin polypeptide can be used to generate antibodies to either highly conserved regions of resistin or to non-conserved regions of the polypeptide. As disclosed elsewhere herein, resistin comprises various conserved domains including, but not limited to, a putative signal peptide from about amino acid residue 1 to about amino acid residue 20 (18 in human resistin); and a putative secreted portion comprising from about amino acid residue 21 (19 in human resistin) to about amino acid residue 114 (108 in human resistin). The secreted portion is characterized by conserved cysteine residues at amino acid positions 26, 55, 67, 76, 78, 82, 93, 95, 97, 107, and 108, in mouse resistin (SEQ ID NO:2). These cysteine residues are also present in human resistin, without gaps when the sequences are aligned, at amino acid positions 22, 51, 63, 72, 74, 78, 89, 91, 93, 103, and 104, in human resistin (SEQ ID NO:4).

Once armed with the sequence of resistin and the detailed analysis localizing the various conserved and non-conserved domains of the protein, the skilled artisan would understand, based upon the disclosure provided herein, how to obtain antibodies specific for the various portions of a mammalian resistin polypeptide using methods well-known in the art or to be developed.

Further, the skilled artisan, based upon the disclosure provided herein, would appreciate that the non-conserved regions of a protein of interest can be more immunogenic than the highly conserved regions which are conserved among various organisms. Further, immunization using a non-conserved immunogenic portion can produce antibodies specific for the non-conserved region thereby producing antibodies that do not cross-react with other proteins which can share one or more conserved portions.

One skilled in the art would appreciate, based upon the disclosure provided herein, which portions of resistin are less homologous with other proteins sharing conserved domains. However, the present invention is not limited to any particular domain; instead, the skilled artisan would understand that other non-conserved regions of the resistin proteins of the invention can be used to produce the antibodies of the invention as disclosed herein.

The polypeptide is 95% homologous, and most preferably, about 99% homologous to at least one of mouse resistin and human resistin. More preferably, the polypeptide that specifically binds with an antibody specific for mammalian resistin is at least one of mouse resistin and human resistin. Most preferably, the polypeptide that specifically binds with an antibody that specifically binds with a mammalian resistin is at least one of SEQ ID NO: 2, and SEQ ID NO:4.

The invention encompasses polyclonal, monoclonal, synthetic antibodies, and the like. One skilled in the art would understand, based upon the disclosure provided herein, that the crucial feature of the antibody of the invention is that the antibody bind specifically with resistin. That is, the antibody of the invention recognizes resistin, or a fragment thereof (*e.g*., an immunogenic portion or antigenic determinant thereof), on Western blots, in immunostaining of cells, and immunoprecipitates resistin using standard methods well-known in the art.

One skilled in the art would appreciate, based upon the disclosure provided herein, that the antibodies can be used to localize the relevant protein in a cell and to study the role(s) of the antigen recognized thereby in cell processes. Moreover, the antibodies can be used to detect and or measure the amount of protein present in a biological sample using well-known methods such as, but not limited to, Western blotting and enzyme-linked immunosorbent assay (ELISA). Moreover, the antibodies can be used to immunoprecipitate and/or immuno-affinity purify their cognate antigen using methods well-known in the art. In addition, the antibody can be used to decrease the level of resistin in a cell thereby inhibiting the effect(s) of resistin in a cell. Thus, by administering the antibody to a cell or to the tissues of an animal or to the animal itself, the required resistin receptor/ligand interactions are therefore inhibited such that the effect of resistin mediated signaling are also inhibited. One skilled in the art would inhibiting resistin ligand/receptor interaction using an anti-resistin antibody include, but are not limited to, increased basal and insulin-stimulated glucose uptake by adipocytes, increased insulin-stimulated glucose uptake in skeletal muscle, decreasing blood glucose levels in an intact organism, lowering serum insulin levels in an intact organism, and the like.

The generation of polyclonal antibodies is accomplished by inoculating the desired animal with the antigen and isolating antibodies which specifically bind the antigen therefrom using standard antibody production methods such as those described in, for example, Harlow et al. (1988, In: Antibodies, A Laboratory Manual, Cold Spring Harbor, NY).

Monoclonal antibodies directed against full length or peptide fragments of a protein or peptide may be prepared using any well known monoclonal antibody preparation procedures, such as those described, for example, in Harlow et al. (1988, In: Antibodies, A Laboratory Manual, Cold Spring Harbor, NY) and in Tuszynski et al. (1988, Blood, 72:109-115). Quantities of the desired peptide may also be synthesized using chemical synthesis technology. Alternatively, DNA encoding the desired peptide may be cloned and expressed from an appropriate promoter sequence in cells suitable for the generation of large quantities of peptide. Monoclonal antibodies directed against the peptide are generated from mice immunized with the peptide using standard procedures as referenced herein.

Nucleic acid encoding the monoclonal antibody obtained using the procedures described herein may be cloned and sequenced using technology which is available in the art, and is described, for example, in Wright et al. (1992, Critical Rev. Immunol. 12:125-168), and the references cited therein. Further, the antibody of the invention may be "humanized" using the technology described in Wright et al. (*supra*), and in the references cited therein, and in Gu et al. (1997, Thrombosis and Hematocyst 77:755-759).

To generate a phage antibody library, a cDNA library is first obtained from mRNA which is isolated from cells, *e.g*., the hybridoma, which express the desired protein to be expressed on the phage surface, *e.g*., the desired antibody. cDNA copies of the mRNA are produced using reverse transcriptase. cDNA which specifies immunoglobulin fragments are obtained by PCR and the resulting DNA is cloned into a suitable bacteriophage vector to generate a bacteriophage DNA library comprising DNA specifying immunoglobulin genes. The procedures for making a bacteriophage library comprising heterologous DNA are well known in the art and are described, for example, in Sambrook et al., *supra.*

Bacteriophage which encode the desired antibody, may be engineered such that the protein is displayed on the surface thereof in such a manner that it is available for binding to its corresponding binding protein, *e.g*., the antigen against which the antibody is directed. Thus, when bacteriophage which express a specific antibody are incubated in the presence of a cell which expresses the corresponding antigen, the bacteriophage will bind to the cell. Bacteriophage which do not express the antibody will not bind to the cell. Such panning techniques are well known in the art and are described for example, in Wright et al. (*supra*).

Processes such as those described above, have been developed for the production of human antibodies using M13 bacteriophage display (Burton et al., 1994, Adv. Immunol. 57:191-280). Essentially, a cDNA library is generated from mRNA obtained from a population of antibody-producing cells. The mRNA encodes rearranged immunoglobulin genes and thus, the cDNA encodes the same. Amplified cDNA is cloned into M 13 expression vectors creating a library of phage which express human Fab fragments on their surface. Phage which display the antibody of interest are selected by antigen binding and are propagated in bacteria to produce soluble human Fab immunoglobulin. Thus, in contrast to conventional monoclonal antibody synthesis, this procedure immortalizes DNA encoding human immunoglobulin rather than cells which express human immunoglobulin.

The procedures just presented describe the generation of phage which encode the Fab portion of an antibody molecule. However, phage which encode single chain antibodies (seFv/phage antibody libraries) are also included in the invention. Fab molecules comprise the entire Ig light chain, that is, they comprise both the variable and constant region of the light chain, but include only the variable region and first constant region domain (CH1) of the heavy chain. Single chain antibody molecules comprise a single chain of protein comprising the Ig Fv fragment. An Ig Fv fragment includes only the variable regions of the heavy and light chains of the antibody, having no constant region contained therein. Phage libraries comprising scFv DNA may be generated following the procedures described in Marks et al. (1991, J. Mol. Biol. 222:581-597). Panning of phage so generated for the isolation of a desired antibody is conducted in a manner similar to that described for phage libraries comprising Fab DNA.

The invention may include synthetic phage display libraries in which the heavy and light chain variable regions may be synthesized such that they include nearly all possible specificities (Barbas, 1995, Nature Medicine 1:837-839; de Kruif et al. 1995, J. Mol. Biol. 248:97-105).

The invention also illustrates methods of contacting a cell with a resistin antagonist thereby causing the cell to exhibit decreased resistance to insulin. The cells contacted with the resistin antagonist can be derived from a patient (*i.e*., endogenous) or can be from another donor animal (*i.e*., heterologous). Where the patient's own cells are used, such treatment can be performed ex vivo or *in vivo*, and the treated cells can be readministered to the patient.

### VII. Compositions

The invention illustrates a composition comprising an isolated nucleic complementary to a nucleic acid, or a portion thereof, encoding a mammalian resistin which is in an antisense orientation with respect to transcription. The composition may comprise a pharmaceutically acceptable carrier.

The invention illustrates composition comprising an isolated mammalian resistin polypeptide as described herein. The composition may comprise a pharmaceutically-acceptable carrier.

The invention also illustrates a composition comprising an antibody that specifically binds resistin. The composition may comprise a pharmaceutically-acceptable carriere (see also embodiment 8)

The invention further illustrates a composition comprising an isolated nucleic acid encoding a mammalian resistin. The composition may comprise a pharmaceutically acceptable carrier.

The compositions can be used to administer resistin to a cell, a tissue, or an animal or to inhibit expression of resistin in a cell, a tissue, or an animal. The compositions are useful to treat a disease, disorder or condition mediated by altered expression of resistin such that decreasing or increasing resistin expression or the level of the protein in a cell, tissue, or animal, is beneficial to the animal. That is, where a disease, disorder or condition in an animal is mediated by or associate with altered level of resistin expression or protein level, the composition can be used to modulate such expression or protein level of resistin.

For administration to the mammal, a polypeptide, or a nucleic acid encoding it, and/or an antisense nucleic acid complementary to all or a portion thereof, can be suspended in any pharmaceutically acceptable carrier, for example, HEPES buffered saline at a pH of about 7.8.

Other pharmaceutically acceptable carriers which are useful include, but are not limited to, glycerol, water, saline, ethanol and other pharmaceutically acceptable salt solutions such as phosphates and salts of organic acids. Examples of these and other pharmaceutically acceptable carriers are described in Remington's Pharmaceutical Sciences (1991, Mack Publication Co., New Jersey).

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or di-glycerides.

Pharmaceutical compositions that are useful in the methods illustrated by the invention may be administered, prepared, packaged, and/or sold in formulations suitable for oral, rectal, vaginal, parenteral, topical, pulmonary, intranasal, buccal, ophthalmic, or another route of administration. Other contemplated formulations include projected nanoparticles, liposomal preparations, resealed erythrocytes containing the active ingredient, and immunologically-based formulations.

The compositions may be administered via numerous routes, including, but not limited to, oral, rectal, vaginal, parenteral, topical, pulmonary, intranasal, buccal, or ophthalmic administration routes. The route(s) of administration will be readily apparent to the skilled artisan and will depend upon any number of factors including the type and severity of the disease being treated, the type and age of the veterinary or human patient being treated, and the like.

Pharmaceutical compositions that are useful in the methods illustrated by the invention may be administered systemically in oral solid formulations, ophthalmic, suppository, aerosol, topical or other similar formulations. In addition to the compound such as heparan sulfate, or a biological equivalent thereof, such pharmaceutical compositions may contain pharmaceutically-acceptable carriers and other ingredients known to enhance and facilitate drug administration. Other possible formulations, such as nanoparticles, liposomes, resealed erythrocytes, and immunologically based systems may also be used to administer resistin and/or a nucleic acid encoding the same according to the methods of the invention.

Compounds which are identified using any of the methods described herein may be formulated and administered to a mammal for treatment of diabetes and/or hyperglycemia are now described.

The invention regards, the preparation and use of pharmaceutical compositions comprising a compound useful for treatment of diabetes or hyperglycemia as an active ingredient (see embodiments 1-5 and 7). Such a pharmaceutical composition may consist of the active ingredient alone, in a form suitable for administration to a subject, or the pharmaceutical composition may comprise the active ingredient and one or more pharmaceutically acceptable carriers, one or more additional ingredients, or some combination of these. The active ingredient may be present in the pharmaceutical composition in the form of a physiologically acceptable ester or salt, such as in combination with a physiologically acceptable cation or anion, as is well known in the art.

As used herein, the term "pharmaceutically acceptable carrier" means a chemical composition with which the active ingredient may be combined and which, following the combination, can be used to administer the active ingredient to a subject.

As used herein, the term "physiologically acceptable" ester or salt means an ester or salt form of the active ingredient which is compatible with any other ingredients of the pharmaceutical composition, which is not deleterious to the subject to which the composition is to be administered.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

Although the descriptions of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, mammals including commercially relevant mammals such as cattle, pigs, horses, sheep, cats, and dogs.

Pharmaceutical compositions that are useful in the methods illustrated by the invention may be prepared, packaged, or sold in formulations suitable for oral, rectal, vaginal, parenteral, topical, pulmonary, intranasal, buccal, ophthalmic, intrathecal or another route of administration. Other contemplated formulations include projected nanoparticles, liposomal preparations, resealed erythrocytes containing the active ingredient, and immunologically-based formulations.

A pharmaceutical composition may be prepared, packaged, or sold in bulk, as a single unit dose, or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered. By way of example, the composition may comprise between 0.1 % and 100% (w/w) active ingredient.

In addition to the active ingredient, a pharmaceutical composition of the invention may further comprise one or more additional pharmaceutically active agents. Particularly contemplated additional agents include anti-emetics and scavengers such as cyanide and cyanate scavengers.

Controlled- or sustained-release formulations of a pharmaceutical composition of the invention may be made using conventional technology.

A formulation of a pharmaceutical composition suitable for oral administration may be prepared, packaged, or sold in the form of a discrete solid dose unit including, but not limited to, a tablet, a hard or soft capsule, a cachet, a troche, or a lozenge, each containing a predetermined amount of the active ingredient. Other formulations suitable for oral administration include, but are not limited to, a powdered or granular formulation, an aqueous or oily suspension, an aqueous or oily solution, or an emulsion.

As used herein, an "oily" liquid is one which comprises a carbon-containing liquid molecule and which exhibits a less polar character than water.

A tablet comprising the active ingredient may, for example, be made by compressing or molding the active ingredient, optionally with one or more additional ingredients. Compressed tablets may be prepared by compressing, in a suitable device, the active ingredient in a free-flowing form such as a powder or granular preparation, optionally mixed with one or more of a binder, a lubricant, an excipient, a surface active agent, and a dispersing agent. Molded tablets may be made by molding, in a suitable device, a mixture of the active ingredient, a pharmaceutically acceptable carrier, and at least sufficient liquid to moisten the mixture. Pharmaceutically acceptable excipients used in the manufacture of tablets include, but are not limited to, inert diluents, granulating and disintegrating agents, binding agents, and lubricating agents. Known dispersing agents include, but are not limited to, potato starch and sodium starch glycollate. Known surface active agents include, but are not limited to, sodium lauryl sulphate. Known diluents include, but are not limited to, calcium carbonate, sodium carbonate, lactose, microcrystalline cellulose, calcium phosphate, calcium hydrogen phosphate, and sodium phosphate. Know granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Tablets may be non-coated or they may be coated using known methods to achieve delayed disintegration in the gastrointestinal tract of a subject, thereby providing sustained release and absorption of the active ingredient. By way of example, a material such as glyceryl monostearate or giyceryi distearate may be used to coat tablets. Further by way of example, tablets may be coated using methods described in U.S. Patents numbers 4,256,108; 4,160,452; and 4,265,874 to form osmotically-controlled release tablets. Tablets may further comprise a sweetening agent, a flavoring agent, a coloring agent, a preservative, or some combination of these in order to provide pharmaceutically elegant and palatable preparation.

Hard capsules comprising the active ingredient may be made using a physiologically degradable composition, such as gelatin. Such hard capsules comprise the active ingredient, and may further comprise additional ingredients including, for example, an inert solid diluent such as calcium carbonate, calcium phosphate, or kaolin.

Soft gelatin capsules comprising the active ingredient may be made using a physiologically degradable composition, such as gelatin. Such soft capsules comprise the active ingredient, which may be mixed with water or an oil medium such as peanut oil, liquid paraffin, or olive oil.

Liquid formulations of a pharmaceutical composition which are suitable for oral administration may be prepared, packaged, and sold either in liquid form or in the form of a dry product intended for reconstitution with water or another suitable vehicle prior to use.

Liquid suspensions may be prepared using conventional methods to achieve suspension of the active ingredient in an aqueous or oily vehicle. Aqueous vehicles include, for example, water and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropylmethylcellulose. Known dispersing or wetting agents include, but are not limited to, naturally-occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (*e.g*., polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin and acacia. Known preservatives include, but are not limited to, methyl, ethyl, or n-propyl-para- hydroxybenzoates, ascorbic acid, and sorbic acid. Known sweetening agents include, for example, glycerol, propylene glycol, sorbitol, sucrose, and saccharin. Known thickening agents for oily suspensions include, for example, beeswax, hard paraffin, and cetyl alcohol.

Liquid solutions of the active ingredient in aqueous or oily solvents may be prepared in substantially the same manner as liquid suspensions, the primary difference being that the active ingredient is dissolved, rather than suspended in the solvent. Liquid solutions of the pharmaceutical composition of the invention may comprise each of the components described with regard to liquid suspensions, it being understood that suspending agents will not necessarily aid dissolution of the active ingredient in the solvent. Aqueous solvents include, for example, water and isotonic saline. Oily solvents include, for example, almond oil, oily esters, ethyl alcohol, vegetable oils such as arachis, olive, sesame, or coconut oil, fractionated vegetable oils, and mineral oils such as liquid paraffin.

Powdered and granular formulations of a pharmaceutical preparation may be prepared using known methods. Such formulations may be administered directly to a subject, used, for example, to form tablets, to fill capsules, or to prepare an aqueous or oily suspension or solution by addition of an aqueous or oily vehicle thereto. Each of these formulations may further comprise one or more of dispersing or wetting agent, a suspending agent, and a preservative. Additional excipients, such as fillers and sweetening, flavoring, or coloring agents, may also be included in these formulations.

A pharmaceutical composition may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally-occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

A pharmaceutical composition may be prepared, packaged, or sold in a formulation suitable for rectal administration. Such a composition may be in the form of, for example, a suppository, a retention enema preparation, and a solution for rectal or colonic irrigation.

Suppository formulations may be made by combining the active ingredient with a non-irritating pharmaceutically acceptable excipient which is solid at ordinary room temperature (*i.e*.. about 20°C) and which is liquid at the rectal temperature of the subject (*i.e*., about 37°C in a healthy human). Suitable pharmaceutically acceptable excipients include, but are not limited to, cocoa butter, polyethylene glycols, and various glycerides. Suppository formulations may further comprise various additional ingredients including, but not limited to, antioxidants and preservatives.

Retention enema preparations or solutions for rectal or colonic irrigation may be made by combining the active ingredient with a pharmaceutically acceptable liquid carrier. As is well known in the art, enema preparations may be administered using, and may be packaged within, a delivery device adapted to the rectal anatomy of the subject. Enema preparations may further comprise various additional ingredients including, but not limited to, antioxidants and preservatives.

A pharmaceutical composition may be prepared, packaged, or sold in a formulation suitable for vaginal administration. Such a composition may be in the form of, for example, a suppository, an impregnated or coated vaginally-insertable material such as a tampon, a douche preparation, or gel or cream or a solution for vaginal irrigation.

Methods for impregnating or coating a material with a chemical composition are known in the art, and include, but are not limited to methods of depositing or binding a chemical composition onto a surface, methods of incorporating a chemical composition into the structure of a material during the synthesis of the material (*i.e*., such as with a physiologically degradable material), and methods of absorbing an aqueous or oily solution or suspension into an absorbent material, with or without subsequent drying.

Douche preparations or solutions for vaginal irrigation may be made by combining the active ingredient with a pharmaceutically acceptable liquid carrier. As is well known in the art, douche preparations may be administered using, and may be packaged within, a delivery device adapted to the vaginal anatomy of the subject. Douche preparations may further comprise various additional ingredients including, but not limited to, antioxidants, antibiotics, antifungal agents, and preservatives.

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry (*i.e*., powder or granular) form for reconstitution with a suitable vehicle (*e.g*., sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or di-glycerides. Other parentally-administrable formulations which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt.

Formulations suitable for topical administration include, but are not limited to, liquid or semi-liquid preparations such as liniments, lotions, oil-in-water or water-in-oil emulsions such as creams, ointments or pastes, and solutions or suspensions. Topically-administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

A pharmaceutical composition may be prepared, packaged, or sold in a formulation suitable for pulmonary administration via the buccal cavity. Such a formulation may comprise dry particles which comprise the active ingredient and which have a diameter in the range from about 0.5 to about 7 nanometers, and preferably from about 1 to about 6 nanometers. Such compositions are conveniently in the form of dry powders for administration using a device comprising a dry powder reservoir to which a stream of propellant may be directed to disperse the powder or using a self-propelling solvent/powder-dispensing container such as a device comprising the active ingredient dissolved or suspended in a low-boiling propellant in a sealed container. Preferably, such powders comprise particles wherein at least 98% of the particles by weight have a diameter greater than 0.5 nanometers and at least 95% of the particles by number have a diameter less than 7 nanometers. More preferably, at least 95% of the particles by weight have a diameter greater than 1 nanometer and at least 90% of the particles by number have a diameter less than 6 nanometers. Dry powder compositions preferably include a solid fine powder diluent such as sugar and are conveniently provided in a unit dose form.

Low boiling propellants generally include liquid propellants having a boiling point of below 65°F at atmospheric pressure. Generally the propellant may constitute 50 to 99.9% (w/w) of the composition, and the active ingredient may constitute 0.1 to 20% (w/w) of the composition. The propellant may further comprise additional ingredients such as a liquid non-ionic or solid anionic surfactant or a solid diluent (preferably having a particle size of the same order as particles comprising the active ingredient).

Pharmaceutical compositions formulated for pulmonary delivery may also provide the active ingredient in the form of droplets of a solution or suspension. Such formulations may be prepared, packaged, or sold as aqueous or dilute alcoholic solutions or suspensions, optionally sterile, comprising the active ingredient, and may conveniently be administered using any nebulization or atomization device. Such formulations may further comprise one or more additional ingredients including, but not limited to, a flavoring agent such as saccharin sodium, a volatile oil, a buffering agent, a surface active agent, or a preservative such as methylhydroxybenzoate. The droplets provided by this route of administration preferably have an average diameter in the range from about 0.1 to about 200 nanometers.

The formulations described herein as being useful for pulmonary delivery are also useful for intranasal delivery of a pharmaceutical composition of the invention.

Another formulation suitable for intranasal administration is a coarse powder comprising the active ingredient and having an average particle from about 0.2 to 500 micrometers. Such a formulation is administered in the manner in which snuff is taken, *i.e*., by rapid inhalation through the nasal passage from a container of the powder held close to the nares.

Formulations suitable for nasal administration may, for example, comprise from about as little as 0.1% (w/w) and as much as 100% (w/w) of the active ingredient, and may further comprise one or more of the additional ingredients described herein.

A pharmaceutical composition may be prepared, packaged, or sold in a formulation suitable for buccal administration. Such formulations may, for example, be in the form of tablets or lozenges made using conventional methods, and may, for example, 0.1 to 20% (w/w) active ingredient, the balance comprising an orally dissolvable or degradable composition and, optionally, one or more of the additional ingredients described herein. Alternately, formulations suitable for buccal administration may comprise a powder or an aerosolized or atomized solution or suspension comprising the active ingredient. Such powdered, aerosolized, or aerosolized formulations, when dispersed, preferably have an average particle or droplet size in the range from about 0. 1 to about 200 nanometers, and may further comprise one or more of the additional ingredients described herein.

A pharmaceutical composition may be prepared, packaged, or sold in a formulation suitable for ophthalmic administration. Such formulations may, for example, be in the form of eye drops including, for example, a 0.1-1.0% (w/w) solution or suspension of the active ingredient in an aqueous or oily liquid carrier. Such drops may further comprise buffering agents, salts, or one or more other of the additional ingredients described herein. Other ophthalmalmically-administrable formulations which are useful include those which comprise the active ingredient in microcrystalline form or in a liposomal preparation.

As used herein, "additional ingredients" include, but are not limited to, one or more of the following: excipients; surface active agents; dispersing agents; inert diluents; granula 1g and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents: filters; emulsifying agents; antioxidants; antibiotics; antifungal agents; stabilizing agents; and pharmaceutically acceptable polymeric or hydrophobic materials. Other "additional ingredients" which may be included in the pharmaceutical compositions of the invention are known in the art and described, for example in Genaro, ed. (1985, Remington's Pharmaceutical Sciences, Mack Publishing Co., Easton, PA), which is incorporated herein by reference.

Typically, dosages of the compound of the invention which may be administered to an animal, preferably a human, will vary depending upon any number of factors, including but not limited to, the type of animal and type of disease state being treated, the age of the animal and the route of administration.

The compound can be administered to an animal as frequently as several times daily, or it may be administered less frequently, such as once a day, once a week, once every two weeks, once a month, or even lees frequently, such as once every several months or even once a year or less. The frequency of the dose will be readily apparent to the skilled artisan and will depend upon any number of factors, such as, but not limited to, the type and severity of the disease being treated, the type and age of the animal, etc.

### VIII. Methods

### A. Methods of identifying useful compounds

The present invention further illustrates a method of identifying a compound that affects expression of resistin in a cell. The method comprises contacting a cell with a test compound and comparing the level of expression of resistin in the cell so contacted with the level of expression of resistin in an otherwise identical cell not contacted with the compound. If the level of expression of resistin is higher or lower in the cell contacted with the test compound compared to the level of expression of resistin in the otherwise identical cell not contacted with the test compound, this is an indication that the test compound affects expression of resistin in a cell.

Similarly, the present invention illustrates a method of identifying a compound that reduces expression of resistin in a cell. The method comprises contacting a cell with a test compound and comparing the level of expression of resistin in the cell contacted with the compound with the level of expression of resistin in an otherwise identical cell, which is not contacted with the compound. If the level of expression of resistin is lower in the cell contacted with the compound compared to the level in the cell that was not contacted with the compound, then that is an indication that the test compound affects reduces expression of resistin in a cell.

One skilled in the art would appreciate, based on the disclosure provided herein, that the level of expression of resistin in the cell may be measured by determining the level of expression of mRNA encoding resistin such as is exemplified herein using BRL49653 as a test compound to identify resistin itself. Alternatively, the level of expression of mRNA encoding resistin can be determined by using immunological methods to assess resistin production from such mRNA as exemplified herein using Western blot analysis using an anti-resistin antibody of the invention. Further, nucleic acid-based detection methods, such as Northern blot and PCR assays and the like, can be used as well. In addition, methods that assess the level of resistin activity in a cells, as exemplified by the glucose uptake assay disclosed elsewhere herein, can also be used to assess the level of resistin in a cell. Thus, one skilled in the art would appreciate, based upon the extensive disclosure and reduction to practice provided herein, that there are a plethora of methods that are well-known in the art, which can be used to asses the level of expression of resistin in a cell including those disclosed herein and others which may be developed in the future.

One skilled in the art would also appreciate, based on the disclosure provided herein, that, preferably, the cell used to identify a compound that affects resistin expression should, but not need not, comprise a functional PPARγ receptor. This is because, as discussed previously elsewhere herein, there is strong evidence that TZD binding with the PPARγ receptor mediates the reduction of expression of resistin. Thus, in order to identify other compounds having antidiabetic effects which are also mediated through interactions involving the PPARγ receptor, such receptor should be available in the cell used for such screening assays. Cells comprising an endogenous functional PPARγ receptor include, but are not limited to, 3T3-L 1 cells as exemplified elsewhere herein, 3T3-442A preadipoctytes, JEG-3 choriocarcinoma cells, as well as any cells engineered to express PPARγ by ectopic expression of an exogenous isolated nucleic acid encoding PPARγ. Additional cells include, *inter alia,* primary cultures of adipocytes obtained from any mammalian species, including but not limited to, mouse, rat and human.

However, the cell used to identify a compound that affects resistin expression need not comprise a functional PPARγ receptor such that compounds which do not act via PPARγ receptor, but which affect expression of resistin, can also be identified using the method of the invention. Thus, using a cell without endogenous PPARγ, it may be possible to suppress resistin by mechanisms other than those mediated by PPARγ activation thereby identifying a compound which reduces resistin expression via alternative pathways.

In addition, a cell that does not comprise a functional PPARγ receptor can be transfected with an appropriate vector such that a functional PPARγ receptor is expressed in the transfectant cell. The transfected cell can then be used according to the method described above, to identify a compound that affects resistin expression via an effect associated with or mediated by PPARγ receptor.

The resistin-based screening method represents an important improvement upon other screening methods that are based upon induction of genes that are induced during adipocyte differentiation (*e.g*., screening methods based upon expression of aP2 as described in Burris et al., 1999, Mol. Endocrinol. 13:410-417). This is because, unlike the method of the invention which uses suppression of resistin expression to identify useful compounds, other methods based on genes induced during adipocyte differentiation do not distinguish between compounds that simply lead to increased numbers of fat cells and those compounds that activate the antidiabetic pathway mediated by PPARγ, resistin, their ligands and receptors, respectively, or all of these molecules. Thus, the present invention illustrates resistin-based assays which are more accurate than other methods which are not specific for potential antidiabetic drug candidate compounds.

The present invention illustrates a method of determining whether a test compound is a candidate antidiabetic drug candidate. The method comprises contacting a cell with a test compound and comparing the level of expression of resistin in the cell with the level of expression of resistin in an otherwise identical cell not contacted with the test compound. If the level of expression of resistin in the cell contacted with the test compound is lower than the level of expression of resistin in the otherwise identical cell not contacted with the test compound, this is an indication that the test compound is a candidate antidiabetic drug candidate.

Similarly, the present invention illustrates a method of determining whether a test compound is a candidate drug for treatment of Syndrome X. The method comprises contacting a cell with a test compound and comparing the level of expression of resistin in the cell with the level of expression of resistin in an otherwise identical cell not contacted with the test compound. If the level of expression of resistin in the cell contacted with the test compound is lower than the level of expression of resistin in the otherwise identical cell not contacted with the test compound, this is an indication that the test compound is a candidate drug for treatment of Syndrome X.

These methods of identifying candidate drugs for type 2 diabetes and/or Syndrome X, are based on the fact that there is a correlation between lower levels of resistin expression and increased glucose uptake such as is desired for the treatment of diseases, disorders or conditions associated with or mediated by decreased glucose uptake such as, but not limited to, type 2 diabetes and Syndrome X. Thus, compounds that mediate a reduction in resistin expression in a cell are candidate therapeutic drugs for treatment of diseases, disorders or conditions mediated by decreased glucose uptake.

Similarly, because, as disclosed herein, the antidiabetic effect of TZDs appears to be mediate by TZD binding of PPARγ receptor which, in turn, mediates the reduction in resistin expression, the present invention illustrates a method of determining whether a test compound is a candidate antidiabetic drug candidate. This method comprises contacting a cell comprising a PPARγ receptor and a nucleic encoding resistin with a test compound. The level of expression of resistin in the cell contacted with the compound is compared with the level of expression of resistin in an otherwise identical cell which is not contacted with the said test compound. As disclosed elsewhere herein, a lower level of expression of resistin in the cell contacted with the test compound compared with the level of expression of resistin in the cell not contacted with the test compound, indicates that the test compound is a candidate antidiabetic drug candidate. Thus, in order to identify other compounds, besides TZDs, having antidiabetic effects which are mediated through interactions involving the PPARγ receptor, such receptor should be available in the cell used for such screening assays. Cells comprising an endogenous functional PPARγ receptor include, but are not limited to, 3T3-L1 cells as exemplified elsewhere herein.

Further, one skilled in the art would appreciate based on the disclosure provided herein that, as disclosed in the examples below, a cell which lacks endogenous resistin expression can be transfected with a vector comprising an isolated nucleic acid encoding *resistin* whereby expression of resistin is effected in the cell. The transfected cell is then contacted with the test compound thereby allowing the determination of whether the compound affects the expression of resistin. Therefore, one skilled in the art armed with the present invention would be able to, by selectively transfecting a cell lacking detectable levels of resistin using resistin-expressing vectors, identify a compound which selectively affects resistin expression.

B. Uses directed to treating or alleviating a disease associated with resistin (embodiments 1-5) The invention illustrates alleviating type 2 diabetes. The approach comprises administering an antisense nucleic acid complementary to a nucleic acid encoding resistin to a patient afflicted with type 2 diabetes. Expression of resistin is specifically and markedly reduced by binding of a ligand, *e.g*., TZD, to PPARγ. This binding, in turn, mediates a strong antidiabetic response which includes uptake of glucose in the absence or presence of insulin. Indeed, the data disclosed herein demonstrate that inhibition of resistin expression increases the glucose uptake effect of insulin.

These data, along with the correlation between a high fat diet and increased levels of resisting in circulating blood, indicate that resistin plays an important role in type 2 diabetes and other diseases disorders or conditions associated with decreased glucose uptake. Thus, reduction of resistin expression mediates or is associated with the antidiabetic effects of TZDs. Therefore, antisense nucleic acid that inhibit resistin expression can be administered to an animal afflicted with type 2 diabetes, or Syndrome X, as a method of alleviating type 2 diabetes, Syndrome X, or both.

Antisense nucleic acids that inhibit expression of resistin may thus also be used for the manufacture of a medicament for treatment of type 2 diabetes.

One skilled in the art would understand, based upon the disclosure provided herein, that because reducing expression of resistin mediates a beneficial effect. *e.g*., decreased resistin Expression mediates increased glucose uptake by adipocytes, which is otherwise lacking or deficient in type 2 diabetics and those suffering from Syndrome X, a method of alleviating type 2 diabetes would include administering to a patient afflicted with type 2 diabetes a glucose uptake-enhancing amount of the composition of an antibody that inhibits resistin activity in a cell.

More specifically, the data disclosed herein demonstrate that antibody binding with resistin decreases the biological effect of resistin and mediates increased glucose uptake by cells. This effect is beneficial in treating, alleviating, or both, type 2 diabetes and Syndrome X since glucose uptake is affected in those diseases. Therefore, increasing glucose uptake, in this instance by decreasing resistin expression, is a useful treatment for type 2 diabetes and Syndrome X, and other diseases, disorders or conditions associated with altered glucose uptake.

One skilled in the art would understand, based upon the disclosure provided herein, that since reduced resistin expression mediates a beneficial effect, including increased glucose uptake by a cell, that methods of decreasing expression of resistin, decreasing the level of resistin polypeptide present in the cell, and/or decreasing the activity of resistin in a cell (using, *e.g*., antisense nucleic acids, ribozymes, antibodies, and the like), can be used to treat and/or alleviate a disease, disorder or condition associated with altered glucose uptake where a higher level of uptake would provide a benefit. Thus, whether an antisense nucleic acid or a blocking antibody is administered, the crucial feature is that the expression of resistin be reduced in a cell.

Techniques for inhibiting expression of a nucleic acid in a cell are well known in the art and encompass such methods as disclosed herein (*e.g*., inhibition using an antibody, an antisense nucleic acid, and the like). Other techniques useful for inhibiting expression of a nucleic acid encoding *resistin* include, but are not limited to, using nucleotide reagents that target specific sequences of the resistin promoter, cell treatments leading to expression or activation of transcription factors (*e.g*., PPARγ) that down-regulate expression of the resistin gene, cell treatments leading to reduced expression or de-activation of transcription factors that up-regulate resistin gene expression (*e.g*., C/EBPα).

Further, approaches that inhibit the biological activity of resistin (*e.g*., cytokine-like signaling via ligand/receptor interactions) are also contemplated herein to treat or alleviate type 2 diabetes, Syndrome X, and any disease associated with or mediated by altered glucose uptake and/or resistin receptor/ligand interactions. The invention includes an approach or increasing glucose uptake by a cell. The approach based upon the surprising results disclosed herein demonstrating that inhibition of resistin expression, translation, and or function (*e.g*., antibody binding with resistin can inhibit required ligand/receptor interactions), mediated an increase in both basal and approach insulin-stimulated glucose uptake by cells. Thus, the approach comprises administering an amount of anti-resistin antibody that sufficiently inhibits resistin binding to its receptor or performing any of its regular functions, to a cell expressing resistin in order to increase glucose uptake, thereby providing an effective method of treating type 2 diabetes, Syndrome X, and other diseases, disorders or conditions associated with or mediated by decreased glucose uptake.

Further, the effect of anti-resistin on glucose uptake, resistin expression, or both, can be determined in a wide variety of cells that express resistin such as, *e.g*., an adipocyte, a recombinant cell transfected with an isolated nucleic acid encoding *resistin,* and the like. The effect of anti-resistin on glucose uptake can also be determined using cell types that are responsive to insulin, such as, but not limited to muscle cell lines including L6 and C2C12 cells and liver cell lines including, but not limited to, Hep3B and HepG2 cells. The effects of anti-resistin can also be assessed in primary cultures of skeletal muscle or adipocytes or hepatocytes from any mammalian species, including, but not limited to, mouse, rat and human.

The invention further includes a method of increasing insulin-stimulated glucose uptake by a cell. The method comprises contacting a cell that expresses resistin (either endogenously or after being receiving an isolated nucleic acid encoding resistin) with insulin and further contacting said cell with a resistin-reducing amount of an anti-resistin compound (*e.g*., anti-resistin antibodies, antisense complementary to nucleic acid encoding resistin, and the like), thereby increasing insulin-stimulated glucose uptake by said cell. AS pointed out previously elsewhere herein, the method is based on the fact, demonstrated for the first time herein, that inhibition of resistin (*e.g*., reduction of expression, amount, and/or activity of resistin in a cell) mediates an increased uptake, both basal and insulin-stimulated, of glucose into a cell.

One skilled in the art would understand, based upon the disclosure provided herein, that it may be useful to increase the level or activity of resistin in a cell. That is, it can be useful to treat or alleviate a disease, disorder of condition associated with or mediated by decreased expression, level, or activity of resistin by administering resistin. Such diseases, disorders or conditions include, but are not limited to, a condition characterized by low blood sugar states further characterized by high insulin levels. Such states include, among others, insulinoma, nesidioblastosis, and other congenital syndromes of hyperinsulinism.

Whether expression of resistin, levels of the polypeptide, or its activity, is increased or decreased, one skilled in the art would appreciate, based on this disclosure, that methods of reducing or inducing resistin of the invention encompass administering a recombinant cell that either expresses or lacks expression of resistin.

In another embodiment of the invention, an individual suffering from a disease, disorder or a condition that is associated with or mediated by resistin expression can be treated by supplementing, augmenting and/or replacing defective cells with cells that lack resistin expression. The cells can be derived from cells obtained from a normal syngeneic matched donor or cells obtained from the individual to be treated. The cells may be genetically modified to inhibit resistin expression.

An example of a disease, disorder or a condition associated with or mediated by resistin expression is type 2 diabetes and Syndrome X.

In addition to replacing defective cells with repaired cells or normal cells from matched donors, the method of the invention may also be used to facilitate expression of a desired protein that when secreted in the an animal, has a beneficial effect. That is, cells may be isolated, furnished with a gene encoding resistin and introduced into the donor or into a syngeneic matched recipient. Expression of the resistin exerts a therapeutic effect, *e.g*., in an individual afflicted with low blood sugar states characterized by high insulin levels. That is, the data disclosed herein demonstrate that administration of resistin reduces glucose tolerance and increases blood glucose levels. Thus, administration of resistin can be used to increase blood sugar levels in a mammal such as to effect treatment of a low blood sugar state in the mammal (*e.g*., insulinoma, nesidioblastosis, and other hyperinsulinism syndromes.

This aspect relates to gene therapy in which therapeutic amounts of resistin are administered to an individual.

Recombinant cells transfected with either nucleic acid encoding resistin, antisense nucleic acids or a knock-out targeting vector of the invention, can be used as cell therapeutics to treat a disease, disorder or a condition characterized by expression of resistin or the lack thereof.

In particular, a gene construct that comprises a heterologous gene which encodes resistin is introduced into cells. These recombinant cells are used to purify isolated resistin, which was then administered to an animal causing diabetic-levels of blood sugar. One skilled in the art would understand, based upon the disclosure provided herein, that instead of administering an isolated resistin polypeptide, resistin can be administered to a mammal in need thereof by administering to the mammal the recombinant cells themselves. This will benefit the recipient individual who will benefit when the protein is expressed and secreted by the recombinant cell into the recipient's system.

Gene constructs comprising nucleotide sequences are introduced into cells. That is, the cells, referred to herein as "recombinant cells," are genetically altered to introduce a nucleic acid encoding resistin or a nucleic acid that inhibits resistin expression in and/or secretion by the recombinant cell thereby mediating a beneficial effect on an recipient to which the recombinant cell is administered. According to some aspects of the invention, cells obtained from the same individual to be treated or from another individual, or from a non-human animal, can be genetically altered to replace a defective gene and/or to introduce a gene whose expression has a beneficial effect on the individual or to inhibit resistin expression which can have a beneficial effect on the individual.

In some aspects , an individual suffering from a disease, disorder or a condition can be treated by supplementing, augmenting and/or replacing defective or deficient nucleic acid encoding resistin by providing an isolated recombinant cells containing gene constructs that include normal, functioning copies of a nucleic acid encoding resistin. This aspect relates to gene therapy in which the individual is provided with a nucleic encoding resistin for which they are deficient in presence and/or function. The isolated nucleic acid encoding resistin provided by the cell compensates for the defective resistin expression of the individual, because, when the nucleic acid is expressed in the individual, a protein is produced which serves to alleviate or otherwise treat the disease, disorder or condition in the individual. Such nucleic acid preferably encodes a resistin polypeptide that is secreted from the recombinant cell.

In all cases in which a gene construct encoding resistin is transfected into a cell, the nucleic acid is operably linked to an appropriate promoter/regulatory sequence which is required to achieve expression of the nucleic acid in the recombinant cell. Such promoter/regulatory sequences include but are not limited to, constitutive and inducible and/or tissue specific and differentiation specific promoters, and are discussed elsewhere herein. Constitutive promoters include, but are not limited to, the cytomegalovirus immediate early promoter and the Rous sarcoma virus promoter. In addition, housekeeping promoters such as those which regulate expression of housekeeping genes may also be used. Other promoters include those which are preferentially expressed in cells of the central nervous system, such as, but not limited the promoter for the gene encoding glial fibrillary acidic protein. In addition, promoter/regulatory elements may be selected such that gene expression is inducible. For example, a tetracycline inducible promoter may be used (Freundlich et al., 1997, Meth. Enzymol. 283:159-173).

The gene construct is preferably provided as an expression vector which includes the coding sequence of a mammalian resistin of the invention operably linked to essential promoter/regulatory sequences such that when the vector is transfected into the cell, the coding sequence is expressed by the cell. The coding sequence is operably linked to the promoter/regulatory elements necessary for expression of the sequence in the cells. The nucleotide sequence that encodes the protein may be cDNA, genomic DNA, synthesized DNA or a hybrid thereof or an RNA molecule such as mRNA.

The gene construct, which includes the nucleotide sequence encoding resistin operably linked to the promoter/regulatory elements, may remain present in the cell as a functioning episomal molecule or it may integrate into the chromosomal DNA of the cell. Genetic material may be introduced into cells where it remains as separate genetic material in the form of a plasmid. Alternatively, linear DNA which can integrate into a host cell chromosome may be introduced into the cell. When introducing DNA into the cell, reagents which promote DNA integration into chromosomes may be added. DNA sequences which are useful to promote integration may also be included in the DNA molecule. Alternatively, RNA may be introduced into the cell.

In order for genetic material in an expression vector to be expressed, the promoter/regulatory elements must be operably linked to the nucleotide sequence that encodes the protein. In order to maximize protein production, promoter/regulatory sequences may be selected which are well suited for gene expression in the desired cells. Moreover, codons may be selected which are most efficiently transcribed in the cell. One having ordinary skill in the art can produce recombinant genetic material as expression vectors which are functional in the desired cells.

It is also contemplated that promoter/regulatory elements may be selected to facilitate tissue specific expression of the protein. Thus, for example, specific promoter/regulatory sequences may be provided such that the heterologous gene will only be expressed in the tissue where the recombinant cells are implanted. One skilled in the art would understand, based upon the disclosure provided herein, that the preferred tissues where the expression or lack of expression of resistin is to be targeted include, but are not limited to, white adipose tissue, brown adipose tissue, blood, hepatic tissue, and skeletal muscle. In addition, promoter/regulatory elements may be selected such that gene expression is inducible. For example, a tetracycline inducible promoter may be used (Freundlich et al., 1997, Meth. Enzymol. 283:159-173).

The nucleic acid encoding resistin preferably includes a signal sequence as disclosed elsewhere herein (*e.g*., amino acids 1 to 20 of mouse resistin [SEQ ID NO:2] and amino acids 1 to 18 of human resistin [SEQ ID NO:4]), which directs the transport and secretion of the resistin encoded by the isolated nucleic acid in the recombinant cell. The signal sequence is generally processed and removed upon secretion of the mature resistin protein from the cell.

In addition to providing cells with recombinant genetic material that either corrects a genetic defect in the cells, that encodes a protein which is otherwise not present in sufficient quantities and/or functional condition so that the genetic material corrects a genetic defect in the individual, and/or that encodes a protein which is useful as beneficial in the treatment or prevention of a particular disease, disorder or condition associated therewith, and that inhibits expression of resistin in the cell (*e.g*., a knock-out targeting vector, an antisense nucleic acid, and the like), genetic material can also be introduced into the recombinant cells used in the present invention to provide a means for selectively terminating such cells should such termination become desirable. Such means for targeting recombinant cells for destruction may be introduced into recombinant cells.

Recombinant cells can be furnished with genetic material which renders them specifically susceptible to destruction. For example, recombinant cells may be provided with a gene that encodes a receptor that can be specifically targeted with a cytotoxic agent. An expressible form of a gene that can be used to induce selective cell death can be introduced into the recombinant cells. In such a system, cells expressing the protein encoded by the gene are susceptible to targeted killing under specific conditions or in, the presence or absence of specific agents. For example, an expressible form of a herpes virus thymidine kinase (herpes tk) gene can be introduced into the recombinant cells and used to induce selective cell death. When the introduced genetic material that includes the herpes tk gene is introduced into the individual, herpes tk will be produced. If it is desirable or necessary to kill the implanted recombinant cells, the drug gangcyclovir can be administered to the individual which will cause the selective killing of any cell producing herpes tk. Thus, a system can be provided which allows for the selective destruction of implanted recombinant cells.

One skilled in the art would understand, based upon the disclosure provided herein, that the present invention encompasses production of recombinant cells to either provide resistin to or inhibit resistin expression in a mammal. That is, the cells can be used to administer resistin to an animal or to deliver a molecule (*e.g*., a knock-out targeting vector, an antisense nucleic acid, a ribozyme, and antibody that specifically binds with resistin, and the like).

Administration of resistin to an animal can be used as a model system to study the mechanism of action of resistin or to develop model systems useful for the development of diagnostics and/or therapeutics for diseases, disorders or conditions associated with resistin expression.

Further, the delivery of resistin to an animal mediated by administration of recombinant cells expressing and secreting resistin can also be used to treat or alleviate a disease, disorder or condition where increasing the level of resistin mediates a therapeutic effect. More specifically, administration of resistin to an animal by administering a recombinant cell expressing a nucleic acid encoding resistin can be useful for treatment of blood sugar states characterized by high insulin levels, including, but not limited to, insulinoma, nesidioblastosis, and other congenital syndromes of hyperinsulinism.

Alternatively, administration of recombinant cells comprising a nucleic acid the expression of which inhibits or reduces resistin expression, activity, and/or secretion from a cell, can be used as a model for the development of diagnostics and/or therapeutics useful for diseases, disorders or conditions associated with or mediated by resistin expression, activity, and/or secretion. The present invention encompasses that the recombinant cells can produce the molecule that inhibits resistin expression thereby providing such molecule to the animal. Alternatively, without wishing to be bound by any particular theory, the recombinant cells themselves, which are otherwise functional adipocytes, hepatocytes, muscle cells, and the like, except for the inability to express resistin, can perform the functions of otherwise identical but non-recombinant cells, without being subject to the resistin signaling pathway.

Cells, both obtained from an animal, from established cell lines that are commercially available or to be developed, or primary cells cultured *in vitro,* can be transfected using well known techniques readily available to those having ordinary skill in the art. Thus, the present invention is not limited to obtaining cells from a donor animal or from the patient animal itself. Rather, the invention includes using any cell that can be engineered using a nucleic acid of the invention such that the recombinant cell either expresses resistin (where it did not express resistin prior to being engineered, or where the cell produced resistin an a different level prior to the introduction of the nucleic acid into the cell) or the recombinant cell does not express resistin or expresses it at a lower level (where it expressed resistin before or expressed resistin at a different level prior to introduction of the nucleic acid into the cell).

Nucleic acids can be introduced into the cells using standard methods which are employed for introducing a gene construct into cells which express the protein encoded by the gene or which express a molecule that inhibits resistin expression. In some embodiments, cells are transfected by calcium phosphate precipitation transfection, DEAE dextran transfection, electroporation, microinjection, liposome-mediated transfer, chemical-mediated transfer, ligand mediated transfer or recombinant viral vector transfer.

Recombinant adenovirus vectors may be used to introduce DNA having a desired sequence into the cell. In some embodiments, recombinant retrovirus vectors are used to introduce DNA having a desired sequence into the cell. In some embodiments, standard calcium phosphate, DEAE dextran or lipid carrier mediated transfection techniques are employed to incorporate a desired DNA into dividing cells. Standard antibiotic resistance selection techniques can be used to identify and select transfected cells. In some embodiments, DNA is introduced directly into cells by microinjection. Similarly, well known electroporation or particle bombardment techniques can be used to introduce foreign DNA into cells. A second gene is usually co-transfected with and/or covalently linked to the nucleic acid encoding resistin, or knock-out targeting vector or antisense molecule thereto. The second gene is frequently a selectable antibiotic-resistance gene. Transfected recombinant cells can be selected by growing the cells in an antibiotic that kills cells that do not take up the selectable gene. In most cases where the two genes are unlinked and co-transfected, the cells that survive the antibiotic treatment contain and express both genes.

Where an isolated resistin polypeptide, an antibody that specifically binds with resistin, a resistin antisense nucleic acid, and/or recombinant cells are administered to an animal either to increase or reduce the level of resistin present in the animal, one skilled in the art would understand, based upon the disclosure provided herein, that the amount of the polypeptide, nucleic acid, antibody, or cell to be administered to the animal can be titrated by assessing the level of resistin and/or sugar present in the blood or by determining the level of expression of resistin or the level of resistin polypeptide or nucleic acid encoding resistin present in the tissues of the animal.

Methods for assessing blood sugar level are well known in the art and are also disclosed herein. Further, methods for assessing the level of resistin (*e.g*., using anti-resistin antibodies in Western blot or other immune-based analyses such as ELISA) and/or methods for assessing the level of resistin expression in a cell and/or tissues (*e.g*., using Northern blot analysis, and the like) are disclosed herein or are well known to those skilled in the art. Such assays can be used to determine the "effective amount" of resistin, nucleic acid, antibody, antisense nucleic acid, ribozyme, recombinant cell, and the like, to be administered to the animal in order to reduce or increase the level of resistin and/or blood sugar amount to a desired level.

### C. Methods of diagnosis and assessment of therapies

The present invention illustrates methods of diagnosis certain diseases, disorders, or conditions (*e.g*., type 2 diabetes and Syndrome X) which are associated with or mediated by expression of resistin.

The invention illustrates method of diagnosing type 2 diabetes in a previously undiagnosed patient mammal. The method comprises obtaining a biological sample from the mammal and comparing the level of resistin (expression, amount, activity) in the sample with the level of resistin in a sample from a normal person who is not afflicted with type 2 diabetes. A higher level of resistin in the sample from the patient compared with the level of resistin in the sample obtained from a person not afflicted with type 2 diabetes is an indication that the patient is afflicted with type 2 diabetes. This is because, as disclosed elsewhere herein, the amount of resistin in a sample is inversely correlated with the ability of a cell to take up glucose.

In one aspect, the biological sample is selected from the group consisting of a blood sample, a white adipose tissue sample, and a brown adipose tissue sample.

Likewise, the invention illustrates a method of diagnosing Syndrome X in a previously undiagnosed patient mammal. The method comprises obtaining a sample from the patient and comparing the level of resistin (expression, amount, activity) with the level of resistin in a sample from an individual not afflicted with Syndrome X. A higher level of resistin in the sample from the patient mammal indicates that the mammal is afflicted with Syndrome X. This is because, as disclosed previously elsewhere herein, the level of resistin is correlated with a decreased ability for glucose uptake in a cell. Thus, one skilled in the art would appreciate, based upon the disclosure provided herein, that a higher level of resistin in a sample indicates that the source of the sample is afflicted with a disease, disorder or condition associated with decreased glucose uptake such as, but not limited to, type 2 diabetes and Syndrome X.

The invention further illustrates a method of diagnosing type 2 diabetes and syndrome X by analyzing the genomic *resistin* DNA. That is, one skilled in the art, once armed with the disclosure provided herein including the nucleic and amino acid sequences of resistin as well as the genomic organization of the *resistin* gene including its localization to human Chromosome 19, would easily develop methods for detecting mutations, rearrangements, and the like, in the *resistin* gene thereby diagnosing a mutation in the gene in a mammal, including, but not limited to, a mouse, a rat, and a human.

The invention illustrates a method of assessing the effectiveness of a treatment for type 2 diabetes in a mammal. The method comprises assessing the level of resistin expression, amount, and/or activity, before, during and after a specified course of treatment for a disease, disorder or condition mediated by or associated with decreased glucose uptake (*e.g*., type 2 diabetes and Syndrome X, among others). This is because, as stated previously elsewhere herein, resistin expression, amount and/or activity is associated with or mediates decreased glucose uptake which is feature of certain disease states. Thus, assessing the effect of a course of treatment upon resistin expression/amount/activity indicates the efficacy of the treatment such that a lower level of resistin expression, amount, or activity indicates that the treatment method is successful.

One skilled in the art would understand, based upon the disclosure provided herein, that the level of resistin is an indicator of the response to TZD in a mammal. This is because TZD, presumably by its binding of PPARγ, mediates a decrease in the level of resistin expression. Thus, the effect, if any, in the level of resistin expression in a mammal undergoing a course of treatment with TZD is an indicator of the effectiveness of the TZD treatment. Therefore, the present invention illustrates a rapid, simple, and effective method for assessing the effectiveness of a therapeutic course of TZD by simply assessing the level of resistin expression in a sample obtained from the mammal being treated with the compound.

The invention further illustrates a method of assessing the response in a mammal to a compound that affects PPARγ-mediated signaling. This method comprises assessing the level of resistin in a sample obtained from a mammal prior to administration of a compound that affects PPARγ-mediated signaling (*e.g*., TZDs, non-TZD compounds that bind with and alter the activity of PPARγ, including prostaglandin J2 derivatives, and the like. The level prior to treatment is then compared to the level of resistin (expression, amount, activity) in a sample obtained during or after the administration of the compound. Since resistin expression is correlated to the effect of certain compounds mediated by PPARγ-mediated signaling, a higher or lower level of resistin expression when the two samples are compared, is an indication that the compound affects PPARγ-mediated signaling which, in turn, affects resistin expression. Therefore, the level of resistin expression is a simple and effective marker for assessing the effect of a substance on PPARγ-mediated signaling.

The data disclosed herein should allow the identification and characterization of the resistin-receptor. This is useful since antagonism of the resistin receptor should improve insulin action, which is useful in treatment of obesity, and other insulin-resistant states leading to type 2 diabetes as well as Syndrome X.

### D. Methods of detecting mutations in the resistin gene locus (illustration)

The methods can be used to detect mutations in a nucleic acid of the invention in order to determine if a human has a mutated gene since such a human is potentially at risk for a disease, disorder, or condition associated with the expression or activity of hresistin (*e.g*., type 2 diabetes, syndrome X, and polycystic ovarian disease). This is because it is well-known that most mutations are deleterious such that a human having a mutation in the nucleic acid encoding *resistin* is more likely than not to be negatively impacted by such mutation which is potentially associated with altered expression or activity of the protein encoded by the mutated nucleic acid.

The methods include detecting, in a biological sample (*e.g*., blood, white adipose tissue, and brown adipose tissue) obtained from the human, the presence or absence of a mutation characterized by at least one of an alteration of a nucleic acid encoding a human resistin (hresistin) of the invention, or the altered expression of the gene encoding *hresistin.* For example, such mutations can be detected by ascertaining the existence of at least one of: 1) a deletion of one or more nucleotides from the nucleic acid encoding *hresistin,* 2) an addition of one or more nucleotides to the nucleic acid encoding *hresistin;* 3) a substitution of one or more nucleotides of the nucleic acid encoding *hresistin;* 4) a chromosomal rearrangement of the nucleic acid encoding *hresistin;* 5) an alteration in the level of a messenger RNA transcript of the nucleic acid encoding *hresistin*; 6) an aberrant modification of the gene, such as of the methylation pattern of the genomic DNA; 7) a non-wild type splicing pattern of a messenger RNA transcript of the nucleic acid encoding *hresistin;* 8) a non-wild type level of the protein encoded by the nucleic acid encoding *hresistin;* 9) an allelic loss of the nucleic acid encoding *hresistin;* and 10) an inappropriate post-translational modification of the protein encoded by the nucleic acid encoding *hresistin.*

As described herein, there are a large number of assay techniques known in the art which can be used for detecting such mutations in a nucleic acid encoding a known protein. Thus, once armed with the teachings set forth herein, including the nucleic and amino acid sequences of human (SEQ ID NOs:3 and 4, respectively) and murine (SEQ ID NOs:1 and 2, respectively) resistin, and the genomic sequence of human *resistin* (SEQ ID NO:5), which is depicted in Figures 22A-E, and comprises from about nucleotide 159120 to 154701 of GenBank Acc. No. AC008763, as well as the genomic arrangement of the entire sequence (depicted for mouse *resistin* in Figure 23), and the localization of human resistin to human Chromosome 19, one skilled in the art would be able to detect a mutation in the *resistin* gene.

Detection of the mutation involves the use of an primer in a polymerase chain reaction (PCR; *see, e.g*., U.S. Patent Nos. 4,683,195 and 4,683,202), such as anchor PCR or RACE PCR, or, alternatively, in a ligation chain reaction (LCR: *see*, *e.g*., Landegran et al., 1988, Science 241:1077-1080; and Nakazawa et al., 1994, Proc. Natl. Acad. Sci. USA 91:360-364), the latter of which can be particularly useful for detecting point mutations in a gene (*see. e.g*., Abravaya et al., 1995, Nucleic Acids Res. 23:675-682). This method can include the steps of collecting a biological sample from a patient, isolating nucleic acid (*e.g*., genomic, mRNA, or both) from the cells of the biological sample, contacting the nucleic acid sample with one or more primers which specifically hybridize with the selected gene under conditions such that hybridization and amplification of the gene (if present) occurs, and detecting the presence or absence of an amplification product. The method can also include detecting the size of the amplification product and comparing the length to the length of a corresponding product obtained in the same manner from a control sample. PCR, LCR, or both, can be used as a preliminary amplification step in conjunction with any of the techniques used for detecting mutations described herein.

Alternative amplification methods include: self-sustained sequence replication (Guatelli et al., 1990, Proc. Natl. Acad. Sci. USA 87:1874-1878), transcriptional amplification system (Kwoh, et al., 1989, Proc. Natl. Acad. Sci. USA 86:1173-1177), Q-Beta Replicase (Lizardi et al., 1988, Bio/Technology 6:1197), or any other nucleic acid amplification method, followed by the detection of the amplified molecules using any of a variety of techniques well known to those of skill in the art. These detection schemes are especially useful for detection of nucleic acid molecules if such molecules are present in very low numbers.

Mutations in a selected gene can be identified in a sample by detecting alterations in restriction enzyme cleavage patterns. For example, sample and control DNA is isolated, (optionally) amplified, digested with one or more restriction endonucleases, and fragment length sizes are determined by gel electrophoresis and compared. Differences in fragment length sizes between sample and control DNA (*i.e*., restriction fragment length polymorphism, RFLP) indicates occurrence of mutations or other sequence differences in the sample DNA compared with control, wild type DNA.

Moreover, sequence specific ribozymes (*see*. *e.g*., U.S. Patent No. 5,498,531) can be used to detect the presence of specific mutations by development or loss of a ribozyme cleavage site.

Genetic mutations are identified by hybridizing a sample and control nucleic acids, *e.g*., DNA or RNA, with high density arrays containing hundreds or thousands of oligonucleotides probes (Cronin et al.,1996, Human Mutation 7:244-255; Kozal et al., 1996, Nature Med. 2:753-759).

In addition, any of a variety of sequencing methods known in the art can be used to directly sequence the selected gene and detect mutations by comparing the sequence of the sample nucleic acids with the corresponding wild-type (control) sequence (*see*, *e.g*., Maxam and Gilbert, 1977, Proc. Natl. Acad. Sci. USA 74:560; Sanger, 1977, Proc. Natl. Acad. Sci. USA 74:5463). It is also contemplated that any of a variety of automated sequencing procedures can be used when performing the diagnostic assays (as reviewed in 1995, Bio/Techniques 19:448). Such automated sequencing methods include mass spectrometry (*see*, *e.g*., PCT Publication No. WO 94/16101; Cohen et al., 1996, Adv. Chromatogr. 36:127-162; Griffin et al., 1993, Appl. Biochem. Biotechnol. 38:147-159).

Other methods for detecting mutations in a selected gene include methods involving protection from cleavage agents to detect mismatched bases in RNA / RNA or RNA / DNA heteroduplexes as described in, *e.g*., Myers et al. (1985, Science 230:1242). In essence, hybridizing RNA or DNA containing wild-type sequence with potentially mutant RNA or DNA obtained from a tissue sample and subsequent treatment of the duplexes formed with an agent(s) (*e.g*., S1 nuclease, hydroxylamine or osmium tetroxide with piperidine, DNA mismatch enzymes such as mutY from *E. coli* or mammalian thymidine DNA glycosylase) that cleaves single-stranded regions of duplex detects base pair mismatches between the control and sample strands. Following digestion of the mismatched regions, the resulting material is separated by size on denaturing polyacrylamide gels to determine the site of the mutated or mismatched region (*see*, *e.g*., Cotton et al., 1988, Proc. Natl. Acad. Sci. USA 85:4397; Saleeba et al., 1992, Methods EnzymoL 217:286-295).

Alterations in electrophoretic mobility are used to identify mutations in genes. For example, single strand conformation polymorphism (SSCP) analysis can be used to detect differences in electrophoretic mobility between mutant and wild type nucleic acids as described in Orita et al. (1989, Proc. Natl. Acad. Sci. USA 86:2766), Cotton (1993, Mutat. Res, 285:125-144), and Hayashi (1992, Genet. Anal. Tech. Appl. 9:73-79).

The movement of mutant or wild-type fragments in polyacrylamide gels containing a gradient of denaturant is assayed using denaturing gradient gel electrophoresis (DGGE), as described by Myers et al. (1985, Nature 313:495).

Examples of other techniques for detecting point mutations include, but are not limited to, selective oligonucleotide hybridization, selective amplification, and selective primer extension (*see*, *e.g*., Saiki et al., 1986, Nature 324:163; Saiki et al., 1989, Proc. Natl. Acad. Sci. USA 86:6230).

Alternatively, allele specific amplification technology can be used in conjunction with the methods of the invention as described in, for example, Gibbs et al. (1989, Nucleic Acids Res. 17:2437-2448), Prossner (1993, Tibtech 11:238), Gasparini et al. (1992, Mol. Cell Probes 6:1), and Barany (1991, Proc. Natl. Acad. Sci. USA 88:189).

Additional methods of detecting a mutation include fluorescent *in situ* hybridization (FISH), as disclosed elsewhere herein, and similar methods well-known in the art.

The methods described herein can be performed, for example, using pre-packaged diagnostic kits comprising at least one probe nucleic acid or antibody reagent described herein. Such kits can be used, for example, to diagnose a human patient exhibiting a disease, disorder, or condition involving a nucleic acid encoding hresistin. Furthermore, any cell type or tissue in which the polypeptide of the invention is expressed, *e.g*., a blood sample, as well as cells and/or tissue from the white adipose tissue, brown adipose tissue, as well as hepatocytes and muscle cells, and the like, can be used in the prognostic assays described herein.

### IX. Kits

The invention illustrates various kits which comprise a compound, such as a nucleic acid encoding resistin, an antibody that specifically binds resistin, a nucleic acid complementary to a nucleic acid encoding resistin but in an antisense orientation with respect to transcription, and/or compositions of the invention, an applicator, and instructional materials which describe use of the compound to perform the methods of the invention. Although exemplary kits are described below, the contents of other useful kits will be apparent to the skilled artisan in light of the present disclosure. Each of these kits is included within the invention.

The invention illustrates a kit for alleviating type 2 diabetes. The kit is used pursuant to the methods disclosed in the invention. Briefly, the kit may be used to contact a cell with a nucleic acid complementary to a nucleic acid encoding resistin where the nucleic acid is in an antisense orientation with respect to transcription to reduce expression of resistin, or with an antibody that specifically binds with resistin, wherein the decreased expression, amount, or activity of resistin mediates an antidiabetic effect. Moreover, the kit comprises an applicator and an instructional material for the use of the kit. These instructions simply embody the examples provided herein.

The kit illustrates a pharmaceutically-acceptable carrier. The composition is provided in an appropriate amount as set forth elsewhere herein. Further, the route of administration and the frequency of administration are as previously set forth elsewhere herein.

In one aspect, the invention illustrates a kit for alleviating Syndrome X. The kit is used pursuant to the methods disclosed in the invention. Briefly, the kit may be used to contact a cell with a nucleic acid complementary to a nucleic acid encoding resistin where the nucleic acid is in an antisense orientation with respect to transcription, or with an antibody that specifically binds with resistin, in order to reduce expression of resistin wherein the decreased expression, amount, or activity of resistin mediates an antidiabetic effect. Moreover, the kit comprises an applicator and an instructional material for the use of the kit. These instructions simply embody the examples provided herein.

The invention illustrates kits for treating type 2 diabetes and for treating Syndrome X. These kits, as discussed previously elsewhere herein, comprise a resistin-inhibiting amount of a nucleic acid complementary to a nucleic acid encoding resistin where the nucleic acid is in an antisense orientation with respect to transcription, or with an antibody that specifically binds with resistin. These resistin-inhibiting substances reduce expression of resistin, and, in turn, the decreased expression, amount, or activity of resistin mediates an antidiabetic effect, *e.g*., increased glucose uptake.

The invention is further described in detail by reference to the following experimental examples. These examples are provided for purposes of illustration

### Example: Resistin, a novel insulin sensitivity modulator

The experiments presented in this example may be summarized as follows.

A novel TZD-suppressible gene, *resistin* (previously referred to as TSG-1 for TZD-suppressible gene 1), has been identified. The data disclosed herein demonstrate that expression of *Resistin* is markedly and specifically suppressed in a cell contacted by the powerful antidiabetic compounds TZDs.

The data disclosed herein suggest that resistin is the mechanistic link between obesity, insulin resistance, and type 2 diabetes (NIDDM), and is a downstream target of TZDs. More specifically, the data disclosed herein demonstrate that *resistin* is expressed only in fat cells. Further, the data demonstrate that resistin is secreted by fat cells, and that the protein can be detected circulating in the bloodstream.

The data disclosed herein further demonstrate that serum resistin levels are increased by high fat diets associated with diabetes and obesity, and that serum resistin levels are decreased by fasting. Neutralization of resistin dramatically improves insulin-stimulated glucose uptake, which is the main antidiabetic function of insulin. Resistin is also dramatically down-regulated by thiazolidinediones (TZDs), a novel class of antidiabetic compounds that function by activating a nuclear hormone receptor called peroxisome proliferator activated receptor γ (PPARγ), which is highly expressed in adipose tissue (fat).

Thus, the data disclosed herein demonstrate that resistin is a potential diagnostic tool for type 2 diabetes as well as syndrome X, both of which diseases are characterized by insulin resistance, hypertension, and cardiovascular disease. The data disclosed herein also indicate that resistin is a potential target for novel therapies for type 2 diabetes, which therapies function by decreasing resistin levels, decreasing resistin biological activity, or both, or therapies based on antagonism of the cellular receptor for resistin thereby inhibiting resistin/receptor interactions involved in type 2 diabetes and/or syndrome X.

The Materials and Methods used in the experiments presented in this example are now described.

### Cloning and isolation of mouse and human resistin cDNAs

To clone and identify resistin, 3T3-L1 cells were differentiated into adipocytes using a standard protocol using differentiation medium (DM) containing dexamethasone, insulin, isobutylmethylxanthine, and fetal bovine serum as described in Green and Kehinde (1975, Cell 5:19-27) and Green and Meuth (1974, Cell 3:127-133). On the seventh day of culture, the cells were treated with either dimethylsulfoxide (DMSO) or 1 micromolar BRL49653 (rosiglitazone) in DMSO. Forty-eight hours later, total RNA was prepared from each cell population using standard methods as described in, for example, Sambrook et al. (1989, Molecular Cloning: A Laboratory Manual, Cold Spring Harbor Laboratory Press, New York) and Ausubel et al. (1997, Current Protocols in Molecular Biology, Green & Wiley, New York). cDNA was then prepared using 1 microgram total RNA from each population using a Cap finder cDNA synthesis kit (Clontech Laboratories, Inc., Palo Alto, CA) following the manufacturer's instructions.

The Clontech PCR-select kit was then used to select cDNAs derived from RNAs that were present in control DMSO-only treated cells but which were absent from RNAs expressed in rosiglitazone-treated cells. These cDNAs were subcloned into an appropriate vector, *i.e*., TA cloning vector (Clontech Laboratories, Inc., Palo Alto, CA), to produce a bacterial library. The library was plated on 96-well plates and then the plates were screened in duplicate using labeled cDNA from untreated 3T3-L1 adipocytes of from adipocytes treated with rosiglitazone.

Clones whose expression was reduced in the rosiglitazone treated cDNA sample were grown-up, and down-regulation by rosiglitazone was confirmed using Northern blot analysis according to standard methods as described in, *e.g*., Sambrook et al., *supra,* and Ausubel et al., *supra.*

The original resistin clone isolated as described previously elsewhere herein was sequenced and the sequence information obtained was used to search the GenBank database using the BLAST algorithm server. Searching GenBank identified an expressed sequence tags (EST) but the sequence was otherwise novel. The ESTs so identified were merged to arrive at the full-length cDNA sequence (SEQ ID NO:1). Further, the ESTs were obtained and sequenced thereby confirming the full-length sequence of *resistin* (SEQ ID NO:1).

The sequence of mouse *resistin* was used to search the GenBank databases and resulted in the identification of several ESTs but the full sequence was novel.

A search of the various databases using the human *resistin* sequence identified two ESTs (GenBank Acc. Nos. AA311223 and N41594) sharing homology with the human sequence. Further, a BLASTP search of the pertinent databases demonstrated that the only known protein sharing the highest degree of identity with human resistin was human ultra high-sulfur keratin 1 (GenBank Acc. No. S 18946). This protein only shares about 30% homology over 43 of the 114 amino acid residues of human resistin thereby demonstrating less than 30% identity with human resistin.

A TBLASTN search of GenBank, EMBL, DDBJ and PDB databases demonstrated that the human *resistin* gene is located on Chromosome 19 as evidenced by the fact that the sequence is present in a contig (GenBank Acc. No. AC008763) from this region of the human genome. The human *resistin* sequence (SEQ ID NO:5) spans from about nucleotide 159120 to about 154701 of the sequence of AC008763.

### Recombinant cells expressing resistin and preparations of resistin polypeptide

293T cells were transfected using Fugene (Boehringer Mannheim, indianapolis, IN) with either empty vector (pIRES-EYFP) or pTSG-IRES-EYFP (Clontech) which contained the 345 bp open reading frame of mouse *resistin.* Also, pTK-hygro was co-transfected for stable selection using hygromycin. Pools of greater than 15 clones were selected to generate a stable cell line. Media (Dulbecco's Modified Eagles Media supplemented with 10% fetal bovine serum) was collected every 24 hours from stable cell lines expressing either empty vector or resistin. This collected conditioned media (CM) was stored in aliquots at -20°C until ready for use.

### Glucose Transport Assay

3T3-L1 adipocytes (day 8) were serum starved for 12 hours in 0.2% fatty acid free bovine serum albumin (BSA) in Dulbecco's modified essential media (DMEM) followed by a glucose step-down in KRP buffer (136 mM NaCl, 4.7 mM KCl, 10mM NaPO₄, 0.9 mM MgSO₄, 0.9 mM CaCl₂) containing 0.2% fatty acid free BSA for 45 minutes immediately prior to stimulation with insulin (Sigma Chemical Co., St. Louis, MO). The cells were stimulated for 15 minutes with 100 nm insulin with the addition of 100 µM 2-deoxyglucose and 0.5 µCi/mL ³H-2-deoxyglucose after 11 minutes. Cytochalasin B (10µM) (Sigma) was added at the time of stimulation to allow for subtraction of non-specific binding. The transport assay was terminated by washing the cells with ice-cold phosphate buffered saline (PBS). The cells were solubilized using 0.05% sodium dodecyl sulfate (SDS) in PBS.

Glucose uptake was determined by scintillation counting of the radioactivity present in the cell extracts. The amount of protein in the cell extracts was determined using Pierce BCA microplate assay kit Pierce Chemical Co., Rockford, IL per the manufacturer's instructions. Specific activity is shown as nmol/mg/min. Anti-resistin rabbit polyclonal antibody (IgG) or non-specific rabbit IgG control were added at the concentrations indicated at the time of serum starvation.

### Generation of resistin rabbit polyclonal sera

The 345 bp open reading frame (ORF) of resistin (SEQ ID NO:1) was subcloned into pGEX-4T expression vector (Invitrogen, Carlsbad, CA) to allow for the production of a fusion protein (resistin-GST) in bacteria. The resultant fusion protein was purified from bacteria inclusion bodies and used as an antigen for rabbit polyclonal sera production per standard methods well-known in the art.

### Fasting and Feeding experiments

Animals were either maintained on normal chow or fasted for a period of 48 hours with free access to water. The fasted and re-fed group were then given access to normal chow *ad libidum* following the fasting period. At the end of the experiment, animals were euthanized using CO₂ inhalation and the tissues were immediately harvested for RNA or protein analysis.

### Western blot analysis

Tissue or cell extracts were homogenized using whole cell lysis buffer (20 mM Tris, 150 mM NaCl, 1 mM EDTA, 10% glycerol, 0.5% NP40) containing protease inhibitors (Complete, Boehringer Mannheim, Indianapolis, IN). Protein concentration was determined using Pierce BCA microplate assay kit as described elsewhere herein. Total cell protein extracts were subjected to electrophoresis using a 15% SDS-PAGE in Laemmli loading buffer containing 20% β-mercaptoethanol (Sigma Chemical Co., St. Louis, MO). Proteins were transferred to Immobilon-P membranes (Millipore) at 60V for 2 hours. The membranes were blocked in 1% BSA in Tris buffered saline (TBS; 0.1M Tris, 0.15M NaCl) containing 0.15% Tween-20 (TTBS). Both primary and secondary antibodies were diluted in blocking buffer. The immunostained proteins were visualized using an enhanced chemiluminescence (ECL) kit (Amersham, Arlington Heights, IL) per the manufacturer's instructions.

cDNAs were subcloned into the TA cloning vector, arrayed on 96 well plates, and screened in duplicate using labeled cDNA from adipocytes or from adipocytes treated with rosiglitazone. Clones whose expression was reduced in the rosiglitazone treated sample were grown up, and down-regulation by rosiglitazone was confirmed by Northern blot analysis as described elsewhere herein.

### Administration of resistin to animals

Prior to injection into animals, 30 ml of conditioned media harvested from recombinant 293T cells expressing resistin was concentrated into 5 ml using an Ultrafree-15 centrifugal filter device (Millipore).

Male and female mice, 9 weeks of age, were administered 200 microliters of concentrated conditioned media intraperitonially just prior to the dark cycle. Mice were consequently fasted overnight. Twelve hours later, a second intraperitional dose of concentrated CM was administered to each animal. Two hours after the second administration, the mice were given a glucose challenge of 10 microliters per gram of body weight of 20 % glucose in PBS solution. Blood glucose levels were determined using a Fast-Take glucometer (Johnson & Johnson) at thirty minute intervals for a two hour period.

The Results of the experiments presented in this example are now described.

### Cloning and identification of resistin as a putative PPARγ-regulated insulin sensitivity modulator

The abundance of PPARγ in mature adipocytes suggested that this cell type is a biological target of TZDs. Given the link between adipose mass and diabetes, the fact that PPARγ is adipogenic further suggested that TZDs regulate a different set of genes in the mature adipocytes as compared with the adipogenic gene program per se. Most known PPARγ target genes are induced during adipogenesis and expression of those genes is maintained at high level in the mature adipocyte. Without wishing to be bound by any particular theory, it was hypothesized that TZD treatment down-regulated an adipocyte gene that normally contributes to insulin resistance.

The hypothesis that TZD down-regulates expression of a gene involved in insulin resistance was tested using the PCR-based differential screen outlined in Figure 1, the results of which are disclosed herein. That is, murine 3T3-L1 cells were differentiated using standard conditions (*i.e*., culturing the cells in a differentiation medium comprising dexamethasone, isobutylmethylxanthine, insulin, and fetal calf serum). On day 7 cells, the differentiated cells were exposed to TZD (rosiglitazone at a concentration of 1 micromolar in DMSO) or continued in post-differentiation medium containing DMSO. The cells were allowed to grow in the absence or presence of rosiglitazone or DMSO-only for 48 hours.

cDNAs from the TZD-treated adipocytes were subtracted cDNAs from those treated in the standard manner, *i.e*., without TZD. The resultant pool of genes enriched for genes down-regulated in adipocytes was further screened against cDNAs from 3 T3-L1 preadipocytes.

cDNAs corresponding to two separate gene products were isolated multiple times upon screening the bacterial library comprising inserts of cDNAs expressed in untreated adipocytes but not expressed in adipocytes treated with rosiglitazone. One sequence was identical to steroyl CoA dehydrogenase, which has been previously demonstrated to be induced during adipogenesis yet down-regulated by TZDs. Leptin, another gene with a similar expression profile, was not identified in this screen. Without wishing to be bound by any particular theory, it may be that leptin is expressed at very low levels in untreated 3T3-L 1 adipocytes.

The second cDNA isolated using the differential screening method, *i.e*., a nucleic acid having the sequence of SEQ ID NO:1, was unique except for a mouse EST identified upon conducting a BLAST search of the GenBank library. This gene has been named *resistin* because of its biological properties. A TBLASTN search of the human EST database disclosed the existence of an apparent human homolog of mouse *resistin.* The cDNA sequence of this human gene, termed either human *resistin* or human RELM-A (hRELM-A), is depicted in Figure 15 (SEQ ID NO:3).

Further TBLASTN search of the pertinent databases demonstrated that human *resistin* is localized to a contig (GenBank Accession No. AC008763) localized to human Chromosome 19. The sequence of the human *resistin* gene (SEQ ID NO:5) is depicted in Figures 22A-E, and comprises from about nucleotide 159120 to about 154701 of the sequence of GenBank Acc. No. AC008763. The sequence depicted in Figures 22A-E commences with nucleotide 159120 of GenBank Acc. No. AC008763 along the bottom strand and ends with nucleotide 154701 of GenBank Acc. No. AC008763. Figures 22A-E comprises the complete sequence of human resistin.

In addition, the organization of the mouse *resistin* gene is illustrated diagrammatically in Figure 23.

Further, BLASTP search of the databases the closest homologous protein, *i.e*., sharing the highest percent sequence identity with the sequence of SEQ ID NO:4, is ultra high-sulfur keratin 1 (GenBank Acc. No. S 18946). This protein shares very little sequence identity with human resistin, sharing only 30% identity over 13 of 43 amino acids while the full length human resistin protein comprises 114 amino acids. Thus, the searches of the relevant databases demonstrated that mammalian resistin of the present invention is a novel protein.

Northern blot analysis demonstrated that *resistin* expression was induced during adipocyte differentiation, with a time course similar to that of a known adipocyte-induced gene, PPARγ (Figure 4A). In contrast, *resistin* gene expression in adipocytes was markedly down-regulated by rosiglitazone treatment as demonstrated by the level of RNA encoding *resistin* present during adipocyte differentiation and exposure to rosiglitazone (Figure 4B). This is dramatically different than what is typically found for adipocyte inducible genes. The example of the fatty acid binding protein aP2 is depicted in Figure 4B. Like *resistin,* aP2 gene expression is increased during adipogenesis. However, in stark contrast to *resistin,* aP2 expression is increased by rosiglitazone treatment.

Down-regulation of *resistin* gene expression was also observed with other antidiabetic TZDs including pioglitazone and troglitazone (Figure 5). It should be noted that rosiglitazone is currently FDA-approved for type 2 diabetes treatment (under the tradename "Avandia") as is pioglitazone (tradename "Actos"). The effect of TZDs was not maximal until 48 hours after exposure to rosiglitazone (Figure 6), consistent with the long half-life of the *resistin* mRNA although, without wishing to be bound by theory, it is also possible that the *resistin* gene may not be a direct target of TZD action.

### Resistin is a secreted molecule

The deduced amino acid sequence of the resistin protein is disclosed in Figure 15. The sequence is not similar to any known proteins. The sequence is notable for a cysteine-rich carboxyl terminus that is reminiscent of EGF-repeat containing proteins. An exciting feature of the resistin amino acid sequence is a hydrophobic 20 amino acid stretch at the N-terminus that is predicted by the Prosort algorithm to be a functional export signal. The prosort algorithm analysis was performed using the program freely available at http://psort.nibb.ac.jp/. The PSORT II algorithm is based upon the collaboration of Paul Horton, Real World Computing Partnership (horton@rwcp.or.jp. The PSORT II algorithm is based upon the YPDϑ and SWISS-PROT data and is freely available upon request to knakai@ims.u-tokyo.ac.jp.

Fusion of a green fluorescent protein (GFP) tag epitope to the C-terminus of resistin (resistin-GFP) altered the subcellular distribution of transfected GFP to a pattern consistent with localization to Golgi apparatus (Figure 7). Unlike GFP, resistin-GFP was secreted from 293T cells transfected by resistin-GFP (Figure 8). Moreover, wild type GFP was detected in the medium of transfected 293T cells using immunoblot analysis with rabbit anti-*resistin* polyclonal antibody (Figure 9).

Anti-resistin antiserum was used to assess endogenous resistin expression during adipogenesis of 3T3-L 1 cells. Endogenous resistin protein expression was detected on day 4, and was maximally induced by day 8-10 (Figure 10A). This was very similar to the time course of resistin gene expression. Remarkably, resistin was also abundant in the media of 3T3-L1 adipocytes (Figure 2D), indicating that the endogenous protein was secreted by the fat cells. Moreover, 3T3-L1 secretion was markedly reduced by exposure of the adipocytes to TZDs (Figure 11). These data are consistent with the down-regulation of *resistin* gene expression by this treatment.

### Resistin is a fat-specific protein that circulates in the blood

Resistin gene expression in multiple mouse tissues was assessed. In a survey of 13 different tissues, *resistin* RNA was only detected in white adipose tissue (Figure 12A). Resistin was very highly expressed in white fat, and expression was somewhat higher in perirenal than in epididymal fat (Figure 12B). Further, the data disclosed herein demonstrate that there appears to be a sexual dimorphism with 2-3 fold greater resistin expression in females than in males (Figure 12B). Low levels of resistin gene expression were detected in brown adipose tissue, and expression was barely detectable, if at all, in tissue obtained from muscle, liver , kidney, and intestine (Figure 12B).

Resistin protein was detectable in white adipose tissue, and was markedly reduced during fasting as was *resistin* in RNA (Figure 13). Resistin was also detectable in serum from AKR and SWR mice using immunoblot analysis using anti-resistin antibody (Figure 14). Remarkably, the level of resistin in mouse serum was greatly increased when these mice were fed a high fat diet leading to obesity and hyperinsulinemia (Figure 14).

### Comparison of mouse and human resistin

The novel sequence of mouse resistin (SEQ ID NO:1) raised the question of whether related molecules exist in humans. In all mouse tissues examined, only a single band was observed using a mouse probe at high stringency as in the experiments shown elsewhere herein. Nonetheless, a TBLASTN search of the human EST database demonstrated the existence of a highly related species, whose cDNA sequence (SEQ ID NO:3) is depicted in Figure 3 and whose putative protein sequence (SEQ ID NO:4) is depicted in Figure 16.

Like mouse resistin, the human homolog, which has been termed either human *resistin* or human Resistin-like molecule A (hRELM-A), contains a predicted signal sequence at its N-terminus consistent with it being a secreted protein like mouse resistin. The overall amino acid identity between mouse and human *resistin* is 55.6%, with even greater identity in the C-terminus region where the identity is about 72%. This sequence structure is depicted schematically in Figure 17.

A direct comparison of human (hRELM-A) and mouse resistin amino acid sequences is depicted in Figure 18. The data disclosed herein demonstrate that in addition to the high degree of sequence identity between the proteins, there is also additional conservation of amino acid type (indicated by the "+" in the figure) throughout the molecules. Furthermore, 11 cysteine residues are completely conserved.

These data suggest that the mouse and human resistin proteins define a new family of cytokine-like, secreted, circulating signaling molecules. An immunoblot analysis of human serum using antiserum raised against mouse resistin revealed a band migrating identically with mouse resistin (Figure 19). This result suggests that human resistin, like mouse resistin, circulates in blood.

### Neutralization of resistin enhances basal and insulin-stimulated glucose uptake

Resistin was identified in a search for an adipocyte signal leading to target tissue insulin resistance. The tissues that are most insulin responsive with respect to glucose uptake are adipose and skeletal muscle. Insulin responsiveness of 3T3-L1 adipocytes has been well documented. TZDs have been shown to increase both basal and insulin-stimulated glucose uptake into 3T3-L1 cells. The data disclosed herein demonstrating, for the first time, that 3T3-L1 cells express the resistin gene at high levels and secrete abundant resistin into their medium suggests that resistin can modulate the basal as well as insulin-stimulated glucose uptake in an autocrine or paracrine manner.

Since antibodies to other hormones and signaling molecules can neutralize their function, the effect of anti-resistin antibodies on glucose uptake was examined. More specifically, anti-resistin antiserum, or preimmune control serum, was added to 3T3-L1 adipocytes and the basal and insulin-stimulated uptake of radiolabeled 2-deoxyglucose was assessed for each cell population.

Addition of IgG purified from preimmune control serum had little effect on either basal or insulin-stimulated glucose uptake. Anti-resistin IgG increased insulin-stimulated glucose uptake approximately 250-300% (Figure 20). These data strongly suggest that resistin functions as a signal to decrease glucose uptake. Further, these data demonstrate the usefulness of methods of inhibiting resistin expression and/or translation in a cell to treat diseases, disorders or conditions associated with decreased glucose uptake, insulin resistance, or both, such as, but not limited to, type 2 diabetes, syndrome X, and polycystic ovarian disease.

### Administration of resistin reduces glucose tolerance

The dramatic effect of anti-resistin antibodies in increasing glucose uptake into cells is mirrored by the reduction in glucose tolerance to diabetic levels in animals mediated by administration of purified resistin as depicted in Figure 21.

The data disclosed herein demonstrate that blood glucose levels of mice dosed with resistin increased to diabetic levels. The blood glucose levels of mice dosed with resistin are depicted using dotted lines in Figure 21, and control glucose values are indicated using solid lines (Figure 21). More specifically, eight female mice (4 per group) were treated with either vehicle (solid lines) or resistin preparation (dashed lines) prepared using conditioned medium obtained from recombinant 293T cells as described elsewhere herein. Baseline glucose in the control mice was about 86 ± 7.4 mg/dl, while that in the resistin-treated mice was significantly higher (p<0.5) at about 99 ± 6.6 mg/dl. These data are consistent with a diabetogenic effect of resistin.

At 30 minutes following administration of glucose, blood glucose in the control injected mice was 232 ± 39 mg/dl. This increase over baseline was similar to that expected historically in this glucose tolerance test protocol. By contrast, the blood glucose 30 minutes after glucose administration to the resistin-treated mice was 395 ± 67 mg/dl. This increase in blood sugar was highly significantly different from that of controls (p < 0.01) and well above diabetic levels. Therefore, these data demonstrate that administration of resistin mediates a diabetogenic response reducing glucose tolerance to diabetic levels in mammals.

The data disclosed herein identify a novel gene, *resistin,* as a potential link between obesity, diabetes, and the mechanism of action of antidiabetic drugs (*e.g.,* TZDs). The data disclosed herein demonstrate that resistin is a new signaling molecule that is induced during adipogenesis and secreted by 3T3-L1 cells. Resistin gene expression and protein secretion are markedly reduced by antidiabetic drugs, TZDs. Moreover, resistin expression *in vivo* is specific to white adipose tissue, where protein levels are regulated by fasting and dietary fat. The protein is also found in the serum of normal mice. These data indicate that resistin is a candidate adipocyte-derived factor that contributes to insulin resistance *in vivo*. Thus, resistin is a target for the antidiabetic actions of TZDs. The ability of resistin antibodies to enhance basal and insulin-stimulated glucose uptake further supports that resistin is an important therapeutic target for diabetes treatments.

Resistin is the prototype of a novel family of potential signaling molecules, the RELMs. However, although human resistin (alternatively referred to herein as " hRELM-A") is highly homologous to mouse resistin, the divergence between the mouse and human sequences suggest, without wishing to be bound by any particular theory, that human resistin disclosed herein it may not be a true human homolog of mouse resistin. Similarly, without wishing to be bound by any particular theory, additional members of the resistin-like family of molecules can be discovered as additional EST libraries are sequenced using the sequences disclosed herein as probes. Further, the data disclosed herein regarding the conservation of cysteine residues in the amino acid sequence of mammalian resistin and other members of the RELM molecule family, will also allow the identification and characterization of additional resistin-like molecules.

Without wishing to be bound by any particular theory, the presence of invariable cysteine residues suggests that resistin belongs to a class of signaling molecules related to cytokines. Leptin is another important adipocyte-derived signaling molecule that is clearly cytokine-related. However, the data disclosed herein demonstrate that there is no similarity between the sequences of resistin and leptin.

The data disclosed herein suggest that resistin signaling is receptor-mediated, although the molecular nature of the receptors for resistin and other RELMs cannot be predicted from the structure of resistin and the receptor(s) remains to be elucidated.

Without wishing to be bound by any particular theory, down-regulation of resistin is likely to explain, at least part, the antidiabetic action of TZDs. As stated previously elsewhere herein, despite the fact that TZDs represent an exciting breakthrough in the therapy of type 2 diabetes, the mechanism(s) by which these compounds mediate there antidiabetic effect is unknown.

Troglitazone, the first TZD approved for clinical use, rapidly became the drug of choice for type 2 diabetes in the United States. However, use of this drug has been disapproved because of its association with life-threatening hepatotoxicity. It is not yet clear whether this is an idiosyncratic effect of troglitazone only, a class effect of TZDs generally, or a consequence of PPARγ activation that would also be observed with non-TZD PPARγ ligands. In any case, PPARγ is expressed in a variety of cell types, and PPARγ ligands would be expected to regulate many genes that may not be related to their antidiabetic effects. This includes the adipogenic gene program, and indeed TZD therapy is associated with weight gain. The potential exacerbation of colonic neoplasia or atherosclerosis by PPARγ ligands has also been debated.

The complications associated with treatments that mediate systemic activation of PPARγ can be avoided by making resistin the target of antidiabetic therapy. That is, by affecting resistin-mediated effects which are downstream from PPARγ, further fine-tunes the cell processes which are impacted by the therapy. Such resistin-based therapies can include reduction of: *resistin* nucleic acid expression, serum resistin level, and/or resistin biological activity, as well as antagonism of resistin action at the level of its cellular receptor(s). These therapies are encompassed in the present invention.

The disclosures of each and every patent, patent application, and publication cited herein are hereby incorporated herein by reference in their entirety.

While the invention has been disclosed with reference to specific embodiments, it is apparent that other embodiments and variations of this invention may be devised by others skilled in the art .

**The embodiments of the invention are set forth in the claims.**

## Claims

1. Use of an antibody or an antibody fragment that specifically binds to a mammalian resistin polypeptide for the preparation of a medicament for the treatment of a diabetic or prediabetic condition wherein said mammalian resistin polypeptide shares at least about 95% sequence identity with SEQ ID NO: 2 or SEQ ID NO: 4.

2. Use according to claim 1, wherein said condition is Syndrome X.

3. Use according to claim 1 or 2, wherein said condition is type 2 diabetes.

4. Use according to claim 1, wherein the composition is for administration in a resistin-inhibiting amount and has the ability to antagonize insulin action on glucose uptake.

5. Use according to any of claims 1 to 3, wherein said antibody is selected from the group consisting of a polyclonal antibody, a monoclonal antibody, a humanized antibody, and a synthetic antibody.

6. Antibody or an antibody fragment that specifically binds to a mammalian resistin polypeptide for use in the treatment of a diabetic or prediabetic condition wherein said mammalian resistin polypeptide shares at least about 95% sequence identity with SEQ ID NO: 2 or SEQ ID NO: 4.

7. Use of a composition comprising an antibody or an antibody fragment that specifically binds to a mammalian resistin polypeptide for the preparation of a medicament for the treatment of a diabetic or prediabetic condition wherein said mammalian resistin polypeptide shares at least about 95% sequence identity with SEQ ID NO: 2 or SEQ ID NO: 4.

8. Composition comprising an antibody or an antibody fragment that specifically binds to a mammalian resistin polypeptide for use in he treatment of a diabetic or prediabetic condition wherein said mammalian resistin polypeptide shares at least about 95% sequence identity with SEQ ID NO: 2 or SEQ ID NO: 4.

## Patentansprüche

1. Verwendung eines Antikörpers oder eines Antikörperfragmentes, welche spezifisch an ein Säugetier-Resistin-Polypeptid binden, für die Herstellung eines Medikaments für die Behandlung eines diabetischen oder prädiabetischen Zustandes, wobei besagtes Säugetier-Resistin-Polypeptid zumindest 95% Sequenzidentität mit SEQ ID NO: 2 oder SEQ ID NO: 4 zeigt.

2. Verwendung gemäß Anspruch 1, wobei besagter Zustand Syndrom X ist.

3. Verwendung gemäß Anspruch 1 oder 2, wobei besagter Zustand Diabetes Typ 2 ist.

4. Verwendung gemäß Anspruch 1, wobei die Zusammensetzung für die Verabreichung in einer Resistin-inhibierenden Menge ist, und die Fähigkeit hat, die Insulin-Wirkung auf die Glukose-Aufnahme zu antagonisieren.

5. Verwendung gemäß irgendeinem der Ansprüche 1 bis 3, wobei besagter Antikörper ausgewählt ist aus der Gruppe bestehend aus einem polyclonalen Antikörper, einem monoclonalen Antikörper, einem humanisierten Antikörper und einem synthetischen Antikörper.

6. Antikörper oder Antikörperfragment, welche spezifisch an ein Säugetier-Resistin-Polypeptid binden zur Verwendung in der Behandlung eines diabetischen oder prädiabetischen Zustandes, wobei besagtes Säugetier-Resistin-Polypeptid zumindest ungefähr 95% Sequenz-Identität mit SEQ ID NO: 2 oder SEQ ID NO: 4 zeigt.

7. Verwendung einer Zusammensetzung umfassend einen Antikörper oder ein Antikörperfragment, welche spezifisch an ein Säugetier-Resistin-Polypeptid binden, für die Herstellung eines Medikamentes für die Behandlung eines diabetischen oder prädiabetischen Zustandes, wobei besagtes Säugetier-Resistin-Polypeptid zumindest ungefähr 95% Sequenz-Identität mit SEQ ID NO: 2 oder SEQ ID NO: 4 zeigt.

8. Zusammensetzung umfassend einen Antikörper oder ein Antikörperfragment, welche spezifisch ein Säugetier-Reisistin-Polypeptid binden, zur Verwendung in der Behandlung eines diabetischen oder prädiabetischen Zustandes, wobei besagtes Säugetier-Resistin-Polypeptid zumindest ungefähr 95% Sequenz-Identität mit SEQ ID NO:2 oder SEQ ID NO: 4 zeigt.

## Revendications

1. Utilisation d'un anticorps ou d'un fragment d'anticorps qui se lie spécifiquement à un polypeptide de résistine de mammifère pour la préparation d'un médicament destiné au traitement d'un état diabétique ou prédiabétique, ledit polypeptide de résistine de mammifère partageant au moins 95 % environ d'identité de séquence avec la SEQ ID N° : 2 ou la SEQ ID N° : 4.

2. Utilisation selon la revendication 1, dans laquelle ledit état est le syndrome X.

3. Utilisation selon la revendication 1 ou 2, dans laquelle ledit état est un diabète de type 2.

4. Utilisation selon la revendication 1, dans laquelle la composition est destinée à une administration dans une quantité inhibant la résistine et est apte à antagoniser l'action de l'insuline sur la capture du glucose.

5. Utilisation selon l'une quelconque des revendications 1 à 3, dans laquelle ledit anticorps est choisi parmi le groupe constitué d'un anticorps polyclonal, d'un anticorps monoclonal, d'un anticorps humanisé et d'un anticorps synthétique.

6. Anticorps ou fragment d'anticorps qui se lie spécifiquement à un polypeptide de résistine de mammifère pour une utilisation dans le traitement d'un état diabétique ou prédiabétique, dans lequel ledit polypeptide de résistine de mammifère partage au moins 95 % environ d'identité de séquence avec la SEQ ID N° : 2 ou la SEQ ID N° : 4.

7. Utilisation d'une composition comprenant un anticorps ou un fragment d'anticorps qui se lie spécifiquement à un polypeptide de résistine de mammifère pour la préparation d'un médicament destiné au traitement d'un état diabétique ou prédiabétique, dans laquelle ledit polypeptide de résistine de mammifère partage au moins 95 % environ d'identité de séquence avec la SEQ ID N° : 2 ou la SEQ ID N° : 4.

8. Composition comprenant un anticorps ou un fragment d'anticorps qui se lie spécifiquement à un polypeptide de résistine de mammifère pour une utilisation dans le traitement d'un état diabétique ou prédiabétique, dans laquelle ledit polypeptide de résistine de mammifère partage au moins 95 % environ d'identité de séquence avec la SEQ ID N° : 2 ou la SEQ ID N° : 4.


### SEQUENCE LISTING

<110> TRUSTEES OF THE UNIVERSITY OF PENNSYLVANIA
   LAZAR, Mitchell A.
<120> COMPOSITIONS, METHODS AND KITS RELATING TO RESISTIN
<130> 053893-5032EP (9596-113EP)
<150> US 60/131,263 <151> 1999-04-27
<150> PCT/US00/11272 <151> 2000-04-27
<160> 5
<170> PatentIn version 3.1
<210> 1
   <211> 576
   <212> DNA
   <213> Mus musculus
<400> 1
   gtgggacagc gagctaatac ccagaactga gttgtgtcct gctaagtcct ctgccacgta   60
   cccacgggat gaagaacctt tcatttcccc tccttttcct tttcttcctt gtccctgaac  120
   tgctgggctc cagcatgcca ctgtgtccca tcgatgaagc catcgacaag aagatcaaac  180
   aagacttcaa ctccctgttt ccaaatgcaa taaagaacat tggcttaaat tgctggacag  240
   tctcctccag agggaagttg gcctcctgcc cagaaggcac agcagtcttg agctgctcct  300
   gtggctctgc ctgtggctcg tgggacattc gtgaagaaaa agtgtgtcac tgccagtgtg  360
   caaggataga ctggacagca gcccgctgct gtaagctgca ggtcgcttcc tgatgtcggg  420
   gaagtgagcg tggtttccag cacagccacc cgttcctgta gctccagaga tgtctgatgt  480
   cctccggtct ctacaggcac ctgcactcac gtgcgcgaat ccacacacaa gcacacatac  540
   ttaaaaataa aacaaaacag gctggaaaaa aaaaaa                            576
<210> 2
   <211> 114
   <212> PRT
   <213> Mus musculus <400> 2
<210> 3
   <211> 479
   <212> DNA
   <213> Homo sapiens
<220>
   <221> misc_feature
   <222> (140)..(140)
   <223> n=a, t, c, g
<400> 3
   gtgtgccgga tttggttagc tgagcccacc gagaggcgcc tgcaggatga aagctctctg   60
   tctcctcctc ctccctgtcc tggggctgtt ggtgtctagc aagaccctgt gctccatgga  120
   agaagccatc aatgagaggn tccaggaggt cgccggctcc ctaatattta gggcaataag  180
   cagcattggc ctggagtgcc agagcgtcac ctccaggggg gacctggcta cttgcccccg  240
   aggcttcgcc gtcaccggct gcacttgtgg ctccgcctgt ggctcgtggg atgtgcgcgc  300
   cgagaccaca tgtcactgcc agtgcgcggg catggactgg accggagcgc gctgctgtcg  360
   tgtgcagccc tgaggtcgcg cgcagcgcgt gcacagcgcg ggcggaggcg gctccaggtc  420
   cggaggggtt gcgggggagc tggaaataaa cctggagatg atgatgatga tgatgatgg   479
<210> 4
   <211> 108
   <212> PRT
   <213> Homo sapiens
<400> 4
   Met Lys Ala Leu Cys Leu Leu Leu Leu Pro Val Leu Gly Leu Leu Val
   1               5                   10                  15
   Ser Ser Lys Thr Leu Cys Ser Met Glu Glu Ala Ile Asn Glu Arg Ile
               20                  25                  30
   Gln Glu Val Ala Gly Ser Leu Ile Phe Arg Ala Ile Ser Ser Ile Gly
           35                  40                  45
   Leu Glu Cys Gln Ser Val Thr Ser Arg Gly Asp Leu Ala Thr Cys Pro
       50                  55                  60
   Arg Gly Phe Ala Val Thr Gly Cys Thr Cys Gly Ser Ala Cys Gly Ser
   65                  70                  75                  80
   Trp Asp Val Arg Ala Glu Thr Thr Cys His Cys Gln Cys Ala Gly Met
                   85                  90                  95
   Asp Trp Thr Gly Ala Arg Cys Cys Arg Val Gln Pro
               100                 105
<210> 5
   <211> 4420
   <212> DNA
   <213> Homo sapiens
<400> 5
   aacctagcca acatggtgaa actccttctc tactaaaaat acaaaaatta gccaggtatg 60
   gtggcgagcg cctgtagtcc cagctacgtg ggaggctgag gcaggagaat cgcttgaacc 120
   caggaggcag aggcttgcgg tgagccgaga ttgcaccact gcactccagc ctgggcaaca 180
   gagcgagacc ctgtctcaaa aaaaaaaaac ttggtttcgt gtggtgtatc ttcgcttgtt 240
   tctgtgtgat ctgtgattgt ccttctgtcg tttcttggtt ttctcttatt ctcggcgtgt 300
   tatgttgcgc tgtgcttcgt ttggttctac tgttttctgt ttccttcttt ctcgtttttg 360
   tcagtcgtct tgtctgtctc cgcagcgcgc ttgtcactct ggtcgcgttg cctgtacgtc 420
   attcgtcgtt ctgcctgctc gttatcgttc tcgcatgatt gttttcctcc gggatcgcat 480
   ggctgctccc cttcttgtat gtcttcttgt ctcctgggct cgtcttctcc cgcttcttcg 540
   tttgtctttc attctctctt ttcattcctc tttctttcac aattacattt cctctccgac 600
   agtgagtcga ttgtctagtg tcaggggaag ggaagggaag aaacgaaacc ctggggggga 660
   tctaggagca gacaagtccc ctgctctgtg ttttcataat ctagtatcca ggaaggggta 720
   agcaccctgc gtgtatctgg ttgtaactaa ctactcacaa ctgcacttgc ctgtgtgaaa 780
   acgtgagctt gtgatgatgc gtgacgtcag gtaggcgtcc ctgactctcc gtaacccaac 840
   tttgcctgtg ccttggggat tcctccttgc aggtaggaag tgaggggtac aggttccagc 900
   tctgggctga gacatgattc agggttccac cctgacctgg ggctcctgga gtcttggggc 960
   cctggagggt cccgtccact gcccagactg acccaggtcc tcgatgaagc ctcattatga 1020
   ggactggggg aaaaggaccc agccacttcc tggggaggtc ggagacccca gggtgagcgt 1080
   caaggtagcc tcaaagatga gacgtcacct cttgaaggca gccatgagcc ttgggtgggg 1140
   acgtcactag aggaagttca ggccctattt tcggaggaag cagttggaga ccccatagga 1200
   ggaagggcga tggggcagta gaaagtcgcg gtgtccccgc cccctccagc agctacgcgc 1260
   cccactctct tggagacgct agatcagtcc ctccgggcct actaaagaaa ccacgcaggg 1320
   ctcagatccg ctccatcatc atcatcatca tcatcatcat catctccagg tttatttcca 1380
   gctcccccgc aacccctccg gacctggagc cgcctccgcc cgcgctgtgc acgcgctgcg 1440
   cgcgacctca gggctgcaca cgacagcagc gcgctccggt ccagtccatg cccgcgcact 1500
   ggcagtgaca tgtggtctcg gcgcgcacat cccacgagcc acaggcggag ccacaagtgc 1560
   agccggtgac ggcgaagcct gcagcccgga acacaggagc gtggactctg agctgggagg 1620
   ctgagggtgg gagcgggagg ggggtgggga gcgcggaggg gggttggggg ggcgggggtg 1680
   gggacgggga cggctggagg ctccaaccac tgaatgggca ctggaggcag ggagtgaggg 1740
   tggacaccag tgtccagatg gtgggcggag aaggctggga gtcaggacca agatcctagg 1800
   ggagtagagg ctggacacgg ggaacgtggc ggggaggggg cattcccagg ggacttggaa 1860
   cagaaatggg cgcctggaca acagtctcct gcactcacct cgggggcaag tagccaggtc 1920
   ccccctggag gtgacgctct ggcactccag gccaatgctg cttattgccc taaatactgg 1980
   ggggcaggag gaaaggagac agggggagct gtgagaccaa acggtccctc ccccatcctc 2040
   ccctagccct gttggtttgg agctaggtcc ctgtgggcat aggagctcac tggcctccag 2100
   gaccctgtct tgagttgggt gttttggagt aagggaaggt ttggagtgag agcggggatt 2160
   gggtttggag ccgtggataa ggtggggaca gtcggagggg ttgggagtgg agttggggtt 2220
   gaatttatga tctggttgga tttgaggatg agatttggtg agcgctgggg ctgggttgga 2280
   gtcaggtctg tgccagggat cagtgaggtc tctgagaccc ttggggagct tgcccaagtg 2340
   gggggtcctc acttagggag ccggcgacct cctggatcct ctcattgatg gcttcttcca 2400
   tggagcacag ggtcttgcta gacaccaaca gccccaggac agggaggagg aggagacaga 2460
   gagctttcat cctgcaggcg ctgaaagagg gaaccaagag acccacagct ggatcagccc 2520
   tgccctgtgg ggaagatccg gcccatggag ggagtaggat ctgcccctgg acctggaccc 2580
   ctgtcccccc atgtggggga cagggatgga ggctcagcct tgaccccagc ctccccgctg 2640
   gtgccatggc aagcgcagga gcagctgtca cttaccctct cggtgggctc agctaaccaa 2700
   atccggcaca cgaattcctg caccgcagct ctttctttga ggcctcttgg ggtggggctt 2760
   cctggcttgg ctaataagtc cctgggcccc caaccctccg gtcccacatc cggggccaag 2820
   aggaagcccc tgagcagaca gtaagggctg gaggaggaag ggagccttcc cacttccaac 2880
   agggcctccg tcttcatgtc cagagactgg tcaggaggtg gtgccccagg gataatgcca 2940
   ggggctgtgg tctgaggaac aggtagacaa gcagagtttt gcatgcaagg gtggctgatg 3000
   caaacatgac aaaattaatg cctcttgcta ggcatggtgc ggacaagcac ttgtagtccc 3060
   agctactaag gaggctgacg tgagagaatt gcttgagccc gggagttcga agctacagtg 3120
   acttatgatc acagcactgc actccagtct gggcaacaga gcaagaccac ttctctaaaa 3180
   tagtaataat aattatgtct ctgggtgaga atgacatacc acattcatac ccaaatgccc 3240
   atgagcaata gaactggtaa ataaaatcat ggtttatggc cggtggctca cgcctgtaat 3300
   cccagcactt tgggaggcca aggcgggcgg atcacttgag gtcaggagct tgagaccaac 3360
   ctggccaaca tgatgaaacc ctgtctccat tagacataca aaaattaact gggcgtggtg 3420
   gcgtgtgcct gtaatcccag ctacttggga ggctgaggtg ggagaatcac ttgaacccgg 3480
   gatgtggagg ttgcagtgca ctgagatcgt gcccctgcac tccatcctgg atgactagct 3540
   tgggcaccat agcaagactc catctcaaaa agaagaaaga aaaatcatgg tttattccat 3600
   caatggcatc acctgcaaca gaagttggaa agccattgct catgggccaa ggtccagctc 3660
   atgtttcttc ttggaccacc catgagcttg gaatggttat acatttttat ttgttctttg 3720
   tttccagtac aacgggcctt tttgtggtaa aatacatata acatacaact taccattata 3780
   acttactttt ttctgttttt gagacggaat cttgctctgt cgcccaggct ggagtgcagt 3840
   ggcgcgatct cggctcacta caagctccgc ctcctgggtt cacgccattc tcctgcttca 3900
   gcctcccaag tagctgggac tacaggcgcc tgccaccacg cccagctaat tttttgtatt 3960
   tttttttttt tagtagagat ggagtttcac cgtgttagcc aggatggtct cgatcccctg 4020
   accttgtgat ctgcccgcct tggcctccca aagtgctggg attacaggcg tgaaccaccg 4080
   tgcccggcct tttttttttt ttttttgaga cggggtcttg ctatgttgcc caagctagtg 4140
   tcagactcct ggcttcaagt aatcctccca ccttggactc cccagtagct gaagctacag 4200
   gtatgcacca tcttgttcca ttttaaccat tgcttttgtt tgtttctttg tttcagagtc 4260
   tcactcagtt gctcaggctg gagtacagtg gctcaatctt ggctcactgc aacctccacc 4320
   tcctgggttc aagcaattct cctgcctcag cctcccgagt agctgggatt acaggcgtgc 4380
   accaccatgc ccggctaatt ttttgtattt ttagtagaga                       4420